# EUROPEAN PATENT APPLICATION

(11) **EP 4 446 326 A1**
(43) Date of publication of application: **16.10.2024**
(21) Application number: 22903630.6
(22) Date of filing: 12.12.2022
(51) Int. Cl.: C07D 491/18, A61K 31/4748, A61P 31/12, A61P 31/14, A61P 19/10, A61P 9/00, A61P 11/00, A61P 17/00, A61P 25/00, A61P 27/00, A61P 43/00

(54) **CYCLIC BISBENZYL TETRAHYDROISOQUINOLINE COMPOUND, AND PREPARATION METHOD THEREFOR AND USE THEREOF**

(30) Priority: 10.12.2021 CN 202111514962
(71) Applicant: Shanghai Institute of Materia Medica, Chinese Academy of Sciences, Pudong, Shanghai 201203 (CN); Wuhan Institute Of Virology, Chinese Academy of Sciences, Wuhan, Hubei 430071 (CN)
(72) Inventor: SHEN, Jingshan, Shanghai 201203 (CN); GAO, Zhaobing, Shanghai 201203 (CN); XIAO, Gengfu, Wuhan, Hubei 430071 (CN); LI, Jia, Shanghai 201203 (CN); YANG, Feipu, Shanghai 201203 (CN); ZHANG, Leike, Wuhan, Hubei 430071 (CN); XIA, Bingqing, Shanghai 201203 (CN); JIANG, Xiangrui, Shanghai 201203 (CN); HE, Yang, Shanghai 201203 (CN); JIANG, Hualiang, Shanghai 201203 (CN)
(74) Representative: dompatent von Kreisler Selting Werner - Partnerschaft von Patent- und Rechtsanwälten mbB
(86) International application number: PCT/CN2022/138457
(87) International publication number: WO 2023/104213

(57) **Abstract**

The present invention provides a cyclic bisbenzyl tetrahydroisoquinoline compound as represented by formula (I), and a pharmaceutically acceptable salt, an enantiomer, a diastereoisomer, a racemate, a crystalline hydrate, and a solvate thereof, wherein R₁ and R₂ are as defined in the description. The present invention further provides a method for preparing the compound and the use of the compound for the preparation of an inhibitor for inhibiting viruses, inflammation, fibrosis and abnormal differentiation of T cells and/or for the preparation of a drug for preventing and/or treating related diseases caused by viruses, such as respiratory tract infections and pneumonia, inflanmmation-related diseases, fibrosis-related diseases and autoimmune diseases.

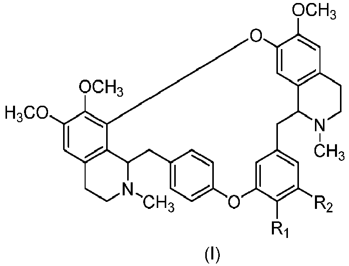

## Description

### Technical field

The present invention relates to the field of pharmaceutical chemistry and chemical synthesis. Specifically, the present invention relates to cyclic bisbenzyl tetrahydroisoquinoline compound and preparation method therefor and use thereof.

### Background

The vast majority of acute infectious diseases are viral infectious diseases, which have a high morbidity and mortality rate. Because of limited means of detection and diagnosis, new epidemic outbreaks caused by new viruses are often characterized by suddenness, randomness and unpredictability, and once an outbreak occurs, without effective means of prevention and treatment, it is very prone to develop large-scale epidemic, seriously threatening people's health and life safety.

The novel coronavirus (SARS-CoV-2) is the seventh coronavirus discovered by human beings, and the novel coronavirus pneumonia (Corona Virus Disease 2019, COVID-19) is an acute infectious disease caused by this virus, which is now sweeping across the world. The number of infections and deaths caused by this virus has far exceeded that of SARS in 2003, seriously threatening human health and social and economic development.

Although vaccines and antiviral drugs have been approved and marketed for severe pneumonia disease caused by SARS-CoV-2 coronavirus, their safety or efficacy remains unsatisfactory. As of November 2021, several mutant strains of the novel coronavirus have emerged successively, such as the South African strain, the Delta strain, the Omicron strain, etc., which have become the major epidemic mutant viruses around the world, characterized by high loads, strong infectiousness, latency, and immune escape, and there is an urgent need for effective preventive and therapeutic measures and drugs in the clinic. Therefore, it is of great social significance to develop low-toxic and high-efficient antiviral drugs against SARS-CoV-2 coronaviruses to meet the clinical needs of SARS-CoV-2 infected patients at home and abroad.

Cyclic bisbenzyl tetrahydroisoquinolines with good antipulmonary injury, anti-inflammatory, antifibrotic, and immunomodulatory effects, represented by oxyacanthine, berbamine, and tetrandrine, have attracted the attention of the technicians in this field. Since the outbreak of the novel coronavirus epidemic, the inventors have screened a large number of natural products for antiviral activity and found that cyclic bisbenzyl tetrahydroisoquinoline alkaloids, such as berbamine, oxyacanthine and tetrandrine, have antiviral effects against novel coronaviruses; however, they have weak antiviral activity and high cytotoxicity, which results in a very low selectivity index/therapeutic index (SI) and poses a potential safety hazard.

In summary, there is an urgent need in this field for the development of novel cyclic bisbenzyl tetrahydroisoquinolines with improved safety to inhibit SARS-CoV-2 coronavirus and for the treatment of pneumonia caused by novel coronavirus infection.

In addition to this, the inventors have explored other potential uses of cyclic bisbenzyl tetrahydroisoquinolines and found that they have the ability to inhibit the in vitro differentiation of CD4⁺T cells. CD4⁺T cell (Th1 and Th17 cells)-mediated autoimmunity has been implicated as an important cause of the triggering of multiple sclerosis. Interferon-gamma (IFN-γ)-producing Th1 CD4⁺T cells and interleukin-17 (IL-17A)-secreting Th17 CD4⁺T cells play key roles in mouse model of experimental allergic encephalomyelitis (EAE), and T-cell polarization, leukocyte migration, and infiltration into the central nervous system are very important steps in the pathogenesis of EAE. There is growing evidence that Th17 (characterized by IL-17 production) is no less important than Th1 in the pathogenesis of multiple sclerosis (MS), e.g., mice with low numbers of Th17 cells are less susceptible to EAE, and Th17 cells have been identified in brain tissue lesions of MS patients. However, there is still no specific drug in the treatment of MS, and most of the revealed regulatory factors are transcription factors or cytokines. Small molecules that can regulate the differentiation of Th1 and Th17 cells, especially those that can inhibit the differentiation of both Th1 and Th17 cells, have not been reported, and these molecules have potential anti-MS applications.

Th17 cells are involved in the maintenance of immune homeostasis, especially on the surface of intestinal mucosa, and contribute to chronic inflammation associated with autoimmune diseases such as rheumatoid arthritis.Th17 cells play an important role in the pathogenesis of rheumatoid arthritis by mediating immune dysfunction, especially in the early stages of disease progression. Inhibiting the over-differentiation of Th17 cells can slow down or prevent the onset and progression of rheumatoid arthritis.

Th17 cells play an important role in host defense against foreign pathogens, and in addition to their role in infectious diseases, Th17 cells have been implicated in the pathogenesis of liver diseases such as viral hepatitis, autoimmune liver disease, and metabolic liver disease. Pathogenic Th17 cells also play a key role in liver diseases developed in response to toxicity or metabolic injury, such as hepatitis B virus (HBV)/hepatitis C virus (HCV) infections, non-alcoholic fatty liver disease (NAFLD), alcoholic liver disease (ALD), hepatocellular carcinoma (HCC), and cholestatic liver disease. The number of Th17 cells increases in the liver and serum of patients with various forms of acute and chronic liver injury.Th17 cells secrete two cytokines important for the development of the inflammatory process, IL-17 and IL-22. The IL-17 receptor is expressed on a number of different cells including monocytes, cholangiocytes, and hepatic stellate cells, and the receptor activation induces secretion of pro-inflammatory cytokines such as IL-1β, IL-6, TNF, and TGFβ. IL-17 also directly induces the production of type I fibrillar collagen by hepatic stellate cells through activation of the signal transducer and activator of transcription 3 (STAT3) signaling pathway. In addition, IL-22 has pro-inflammatory effects in hepatitis B virus infection. Therefore, compounds with inhibitory effects on Th17 over differentiation may play a role in alleviating liver fibrosis, hepatitis B virus (HBV)/hepatitis C virus (HCV)-infected liver disease, non-alcoholic fatty liver disease (NAFLD), alcoholic liver disease (ALD), hepatocellular carcinoma (HCC), and cholestatic liver disease.

Th17 cells secrete glycoprotein IL-17A, which is also a pro-inflammatory cytokine involved in chronic inflammation and autoimmune diseases. IL-17A is implicated in the onset and progression of pulmonary fibrosis in both TGF-β1-dependent and independent ways, and the IL-17A signaling pathway is a potential therapeutic target for the treatment of fibroproliferative lung disease. Inhibition of IL-17A secretion also ameliorated silica-induced lung dysfunction, lung inflammation and fibrosis in mice. Thus, it is evident that inhibition of over differentiation of IL17A-secreting Th17 cells reduces IL-17A levels, which in turn alleviates the progression of many pulmonary fibrosis diseases, including silicosis.

Few small molecules have been reported in the literature with good drugability that can inhibit the differentiation of both Th1 and Th17 cells, and such molecules have potential applications in the treatment of autoimmune diseases such as rheumatoid arthritis, psoriasis, multiple sclerosis, systemic lupus erythematosus, systemic sclerosis, neuromyelitisoptica, autoimmune enterocolitis, autoimmune hepatitis, and hepatic fibrosis, lung fibrosis, renal fibrosis, scleroderma and silicosis, and is also expected to alleviate liver diseases, including liver fibrosis, hepatitis B virus (HBV)/hepatitis C virus (HCV) infectious liver disease, non-alcoholic fatty liver disease (NAFLD), alcoholic liver disease (ALD), hepatocellular carcinoma (HCC), and cholestatic liver disease, etc.

### Summary

An object of the present invention is to provide an inhibitor for inhibiting the activity of virus, particularly SARS-CoV-2 coronavirus with higher safety, a preparation method therefor, and a use thereof in the treatment, prevention, and alleviation of diseases caused by viral infections, particularly diseases such as pneumonia caused by novel coronavirus infection.

Another object of the present invention is to provide a compound with anti-inflammatory effect, a preparation method therefor, and a use thereof in the treatment, prevention, and alleviation of inflammation-related diseases such as pneumonia, non-alcoholic steatohepatitis, colitis, nephritis, pancreatitis, myocarditis, arthritis, inflammatory pain and the like.

Another object of the present invention is to provide a compound with anti-fibrosis effect, a preparation method therefor, and a use thereof in the treatment, prevention, and alleviation of fibrosis-related diseases such as pulmonary fibrosis, silicosis, hepatic fibrosis, renal fibrosis, myocardial fibrosis, dermatological fibrosis, retinal fibrosis, myelofibrosis, inflammatory pain and the like.

Another object of the present invention is to provide an inhibitor for inhibiting T-cell differentiation, in particular an inhibitor for inhibitingTh1 and Th17 cell differentiation, a preparation method therefor and a use thereof in the treatment, prevention, and alleviation of autoimmune diseases associated with abnormal T-cell differentiation such as psoriasis, rheumatoid arthritis, systemic lupus erythematosus, multiple sclerosis, neuromyelitisoptica, myasthenia gravis, ankylosing spondylitis, ulcerative colitis, Crohn's disease, delayed type hypersensitivity, graft-versus-host disease and the like.

In the first aspect of the present invention, provided is a cyclic bisbenzyl isoquinoline compound of Formula I, or a pharmaceutically acceptable salt, an enantiomer, a diastereoisomer, a racemate, or a crystalline hydrate, a solvate thereof, or a mixture thereof, 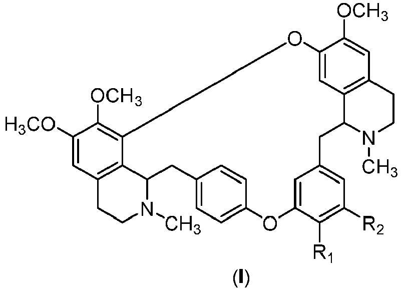 wherein,
R₁ and R₂ are each independently selected from: hydrogen, halogen, nitro, hydroxyl, thiol, C₁-C₆alkoxy, C₁-C₆haloalkoxy, hydroxyl C₁-C₆alkoxy, C₁-C₆alkylthiol, C₁-C₆ alkyl, C₁-C₆haloalkyl, amino C₁-C₆ alkyl, hydroxyl C₁-C₆ alkyl, cyanoC₁-C₆ alkyl, C₃-C₈cycloalkyl, C₃-C₈cycloalkoxy, C₂-C₆ alkenyl, C₂-C₆ alkenyl carbonyl, C₂-C₆alkenoxy, C₂-C₆alkynyl, C₂-C₆alkynoxy, amino, C₁-C₆ alkyl-substituted amino, benzyl-substituted amino, C₁-C₆alkanoyl-substituted amino, C₂-C₆alkenoyl-substituted amino, cyano, C₁ -C₆ carboxyl, C₁ -C₆ aldehyde, C₁-C₆alkanoyl, C₃-C₆cycloalkyl acyl, C₁-C₆haloalkanoyl, sulfonamido, C₁-C₆ alkyl-substituted sulfonamido, carbamoyl, phenylcarbamoyl, N-methyl-N-methoxyamino, C₁-C₆ alkyl-substituted carbamoyl, C₃-C₆cycloalkyl-substituted carbamoyl, adamantylcarbamoyl, pyridinyl- or pyrimidinyl-substituted carbamoyl, hydroxyC₁-C₆alkoxyC₁-C₆ alkyl-substituted carbamoyl, C₁-C₆alkoxyC₁-C₆ alkyl-substituted carbamoyl, hydroxyC₁-C₆alkoxy-substituted C₁-C₆alkoxycarbonyl, phenoxycarbonyl, C₃-C₆cycloalkyl-substitutedhydroxymethyl, carboxyl C₁-C₆ alkyl, C₁-C₆alkylsulfonyl, C₁-C₆haloalkylsulfonyl, C₁-C₆ alkyl-substituted amino C₁-C₆ alkyl, C₁-C₂₀ alkyl-substituted carbamoyloxy (or C₁-C₆ alkyl-substituted carbamoyloxy), C₃-C₁₀ocycloalkyl-substituted carbamoyloxy, C₁-C₆alkanoyl-substituted amino C₁-C₆ alkyl, C₁-C₆alkoxycarbonyl, carbamoyl C₁-C₆ alkyl, C₁-C₆ alkyl-substituted carbamoyl C₁-C₆ alkyl, C₂-C₆ alkenyl acyloxy (C₂-C₆ alkenyl-CO-O-), C₂-C₁₀ ester group, 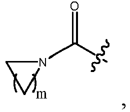 and -O-Z, wherein Z is 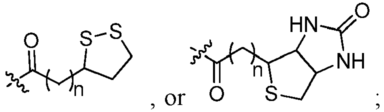
wherein n is 0, 1, 2, 3, or 4; and m is 1, 2, 3, or 4;
wherein the phenylcarbamoyl, and phenoxycarbonyl are optionally substituted by one or more substituents selected from F, Cl, Br, I, C₁-C₆ alkyl, C₁-C₆haloalkyl, C₁-C₆alkoxyandC₁-C₆haloalkoxy;
or R₁ and R₂ together with the adjacent benzene ring form a benzo[5 to 6-membered monocyclic heterocycle]which is unsubstituted or substitutedby1 to 4 substituents selected from halogen, hydroxyl, thiol, oxo, thio, C₁-C₆ alkyl, C₁-C₆alkoxy and cyano; and the heterocycle contains from 1 to 3 heteroatoms selected from N, O, and S;
with the proviso that the compound does not include the following compound:

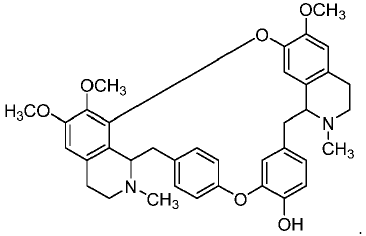

In another preferred embodiment, the compound does not include the following compound:

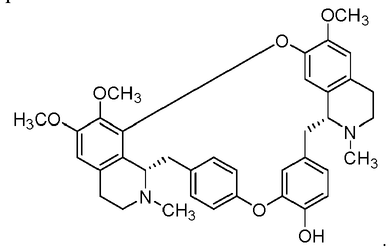

In another preferred embodiment, the compound of formula I is a non-natural product.

In another preferred embodiment, the benzo[5 to 6-membered monocyclic heterocycle] is selected from:

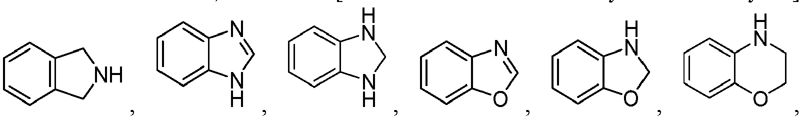

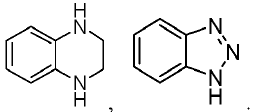

In another preferred embodiment, in the compound, R₁ and R₂ are each independently selected from: hydrogen, halogen, nitro, hydroxyl, thiol, C₁-C₄alkoxy, C₁-C₄haloalkoxy, hydroxyl C₁-C₄alkoxy, C₁-C₄alkylthiol, C₁-C₄ alkyl, C₁-C₄haloalkyl, amino C₁-C₄ alkyl, hydroxyl C₁-C₄ alkyl, cyanoC₁-C₄ alkyl, C₃-C₆cycloalkyl, C₃-C₆cycloalkoxy, C₂-C₅ alkenyl, C₂-C₅ alkenyl carbonyl, C₂-C₅alkenoxy, C₂-C₅alkynyl, C₂-C₅alkynoxy, amino, C₁-C₄ alkyl-substituted amino, benzyl-substituted amino, C₁-C₄alkanoyl-substituted amino, C₂-C₅alkenoyl-substituted amino, cyano, C₁-C₄ carboxyl, C₁-C₄ aldehyde, C₁-C₄alkanoyl, C₃-C₅cycloalkyl acyl, C₁-C₄haloalkanoyl, sulfonamido (-SO₂NH₂), C₁-C₄ alkyl-substituted sulfonamido (-SO₂NH₂), carbamoyl(-CONH₂), phenylcarbamoyl, N-methyl-N-methoxyamino, C₁-C₄ alkyl-substituted carbamoyl, C₃-C₅cycloalkyl-substituted carbamoyl, adamantylcarbamoyl, pyridinyl- or pyrimidinyl-substituted carbamoyl, hydroxyC₁-C₄alkoxyC₁-C₄ alkyl-substituted carbamoyl, C₁-C₄alkoxyC₁-C₄ alkyl-substituted carbamoyl, hydroxyC₁-C₄alkoxy-substituted C₁-C₄alkoxycarbonyl, phenoxycarbonyl, Cs-Cscycloalkyl-substituted hydroxymethyl, carboxyl C₁-C₄ alkyl, C₁-C₄alkylsulfonyl, C₁-C₄haloalkylsulfonyl, C₁-C₄ alkyl-substituted amino C₁-C₄ alkyl, C₁-C₁₀ alkyl-substituted carbamoyloxy (or C₁-C₄ alkyl-substituted carbamoyloxy), C₃-C₈cycloalkyl-substituted carbamoyloxy, C₁-C₄alkanoyl-substituted amino C₁-C₄ alkyl, C₁-C₄alkoxycarbonyl, carbamoyl C₁-C₄ alkyl, C₁-C₄ alkyl-substituted carbamoyl C₁-C₄ alkyl, C₂-C₅ alkenyl acyloxy (C₂-C₅ alkenyl-CO-O-), C₂-C₆ ester group, 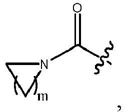 and -O-Z, wherein Z is 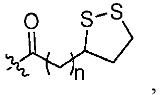 or 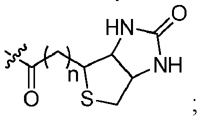 n is 0, 1, 2, 3, or 4; and m is 1, 2, 3, or 4;
or R₁ and R₂ together with the adjacent benzene ring form a benzo[5 to 6-membered monocyclic heterocycle] which is unsubstituted or substituted by 1 to 2 substituents selected from halogen, hydroxyl, thiol, oxo (=O), thio (=S), C₁-C₆ alkyl, C₁-C₆alkoxy and cyano; and the heterocycle contains from 1 to 3 heteroatoms selected from N, O, and S.

In another preferred embodiment, in the compound, R₁ and R₂ are each independently selected from: hydrogen, fluorine, chlorine, bromine, nitro, hydroxyl, thiol, methoxy, ethoxy, trifluoromethoxy, -SCH₃, -SCH₂CH₃, propyl, cyclopropyl, isopropyl, tert-butyl, trifluoromethyl, difluoromethoxy, bromomethyl, chloromethyl, vinyl, vinylmethyl, amino, N-methylamino, N-ethyl amino, N,N-dimethylamino, N,N-diethylamino, cyano, carboxyl, aldehyde, -CH₂NH₂, -CH₂CH₂NH₂, -CH₂OH, -CH₂CH₂OH, -CH₂CN, -CH₂CH₂CN, formyl, acetyl, propionyl, trifluoroacetyl, sulfonamido, carbamoyl, N-methylcarbamoyl, N,N-dimethylcarbamoyl, N-ethylcarbamoyl, N,N-diethylcarbamoyl, -O(C=O)NH(C4 alkyl), cyclopentylcarbamoyloxy, cyclohexylcarbamoyloxy, -CH₂CO₂H, -CH₂CH₂CO₂H, -SO₂CH₃, -SO₂CF₃, -CH₂NHMe, -CH₂NMe₂, -CH₂CONH₂, -CONH₂, -NHCOCH₃, -CH₂ NHCOCH₃, -(C=O)OCH₂CH₂OCH₂CH₂OH, -(C=O)OCH₃, -CH₂CONHMe, -CONHMe, -CONH(cyclopropyl), -CH₂CONMe₂,and -O-Z, wherein Z is 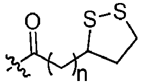 or 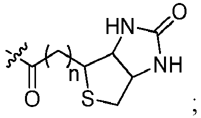 n is an integer from 0 to 4;
orR₁ and R₂ together with the adjacent benzene ring form a benzo[5 to 6-membered monocyclic heterocycle] which is unsubstituted or substituted by 1 to 2 substituents selected from halogen, hydroxyl, thiol, oxo-, thio-, and C₁-C₆ alkyl; and the heterocycle contains from 1 to 3 heteroatoms selected from N, O, and S.

In another preferred embodiment, R₁ is hydrogen, fluorine, chlorine, bromine, thiol, methoxy, ethoxy, trifluoromethoxy, -SCH₃, -SCH₂CH₃, propyl, cyclopropyl, isopropyl, tert-butyl, trifluoromethyl, difluoromethoxy, vinyl, vinylmethyl, amino, N-methylamino, N-ethyl amino, N,N-dimethylamino, N,N-diethylamino, cyano, carboxyl, aldehyde, -CH₂NH₂, -CH₂CH₂NH₂, -CH₂OH, -CH₂CH₂OH, -CH₂CN, -CH₂CH₂CN, formyl, acetyl, propionyl, trifluoroacetyl, sulfonamido, carbamoyl, N-methylcarbamoyl, N,N-dimethylcarbamoyl, N-ethylcarbamoyl, N,N-diethylcarbamoyl, -CH₂CO₂H, -CH₂CH₂CO₂H, -CH₂NHMe, -CH₂NMe₂, -CH₂CONH₂, -NHCOCH₃, -CH₂NHCOCH₃, -CH₂CONHMe or -CH₂CONMe₂.

In another preferred embodiment, R₁ is carbamoyl, N-methylcarbamoyl, N,N-dimethylcarbamoyl, N-ethylcarbamoyl, N-n-propylcarbamoyl, N-isopropylcarbamoyl, N-cyclopropylcarbamoyl, N,N-diethylcarbamoyl, or N-cyclopropylcarbamoyl.

In another preferred embodiment, R₂ is hydrogen, fluorine, chlorine, bromine, nitro, amino, N-methylamino, N-ethylamino, N,N-dimethylamino, N,N-diethylamino, sulfonamidoor -NHCOCH₃.

In another preferred embodiment, the compound of formula I is chiral or achiral.

In another preferred embodiment the compound has a structure of formula I-a:

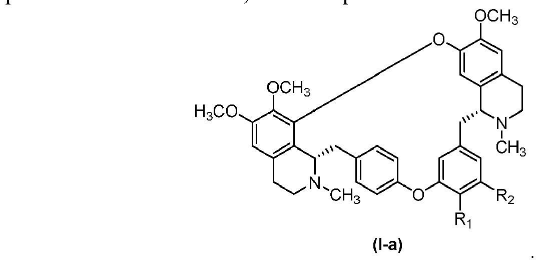

In another preferred embodiment, the compound has a structure of formula I-b:

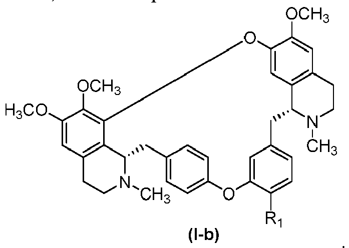

In another preferred embodiment, the compound of formula I is selected from the following compound:

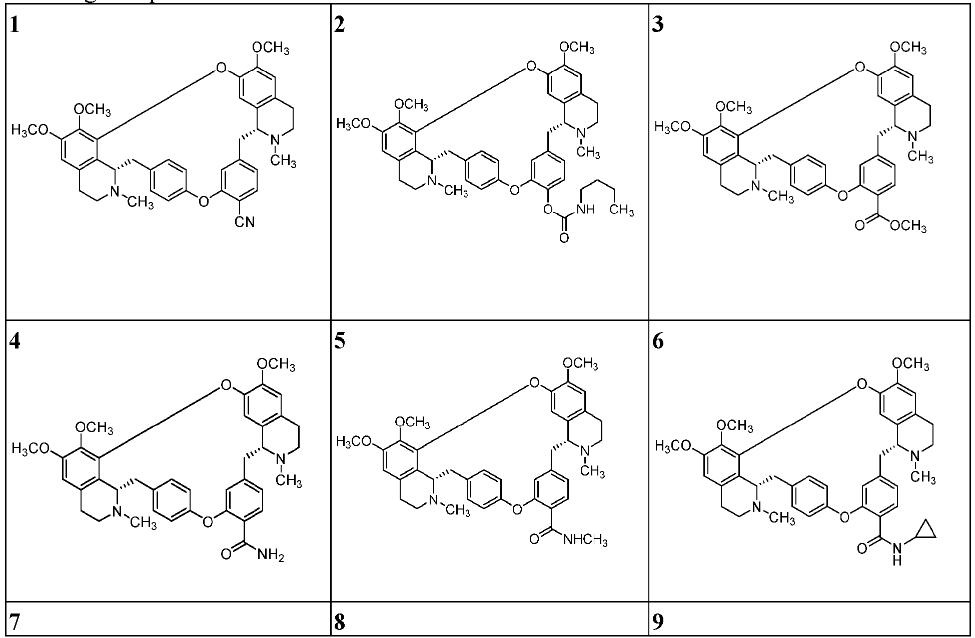

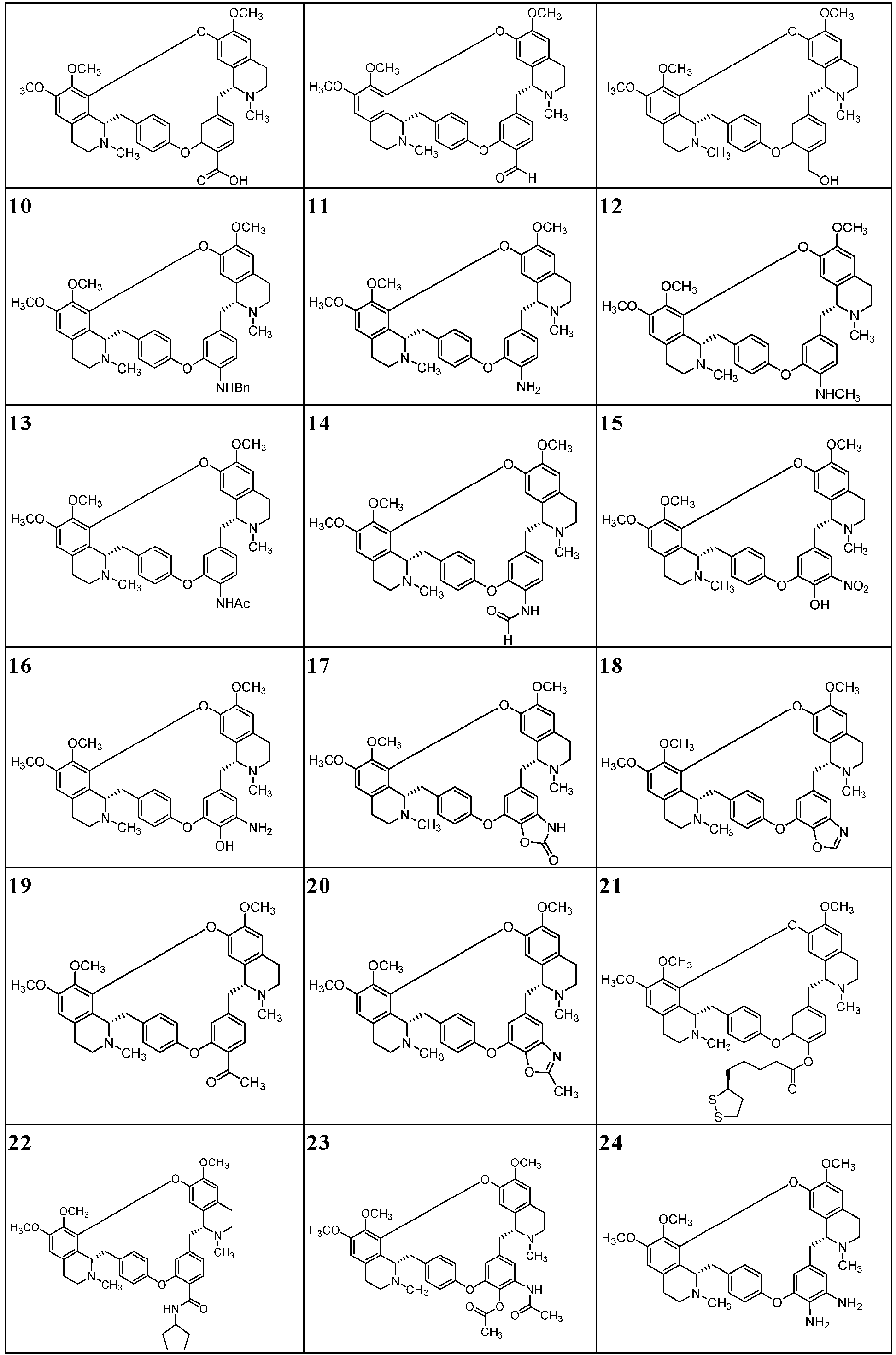

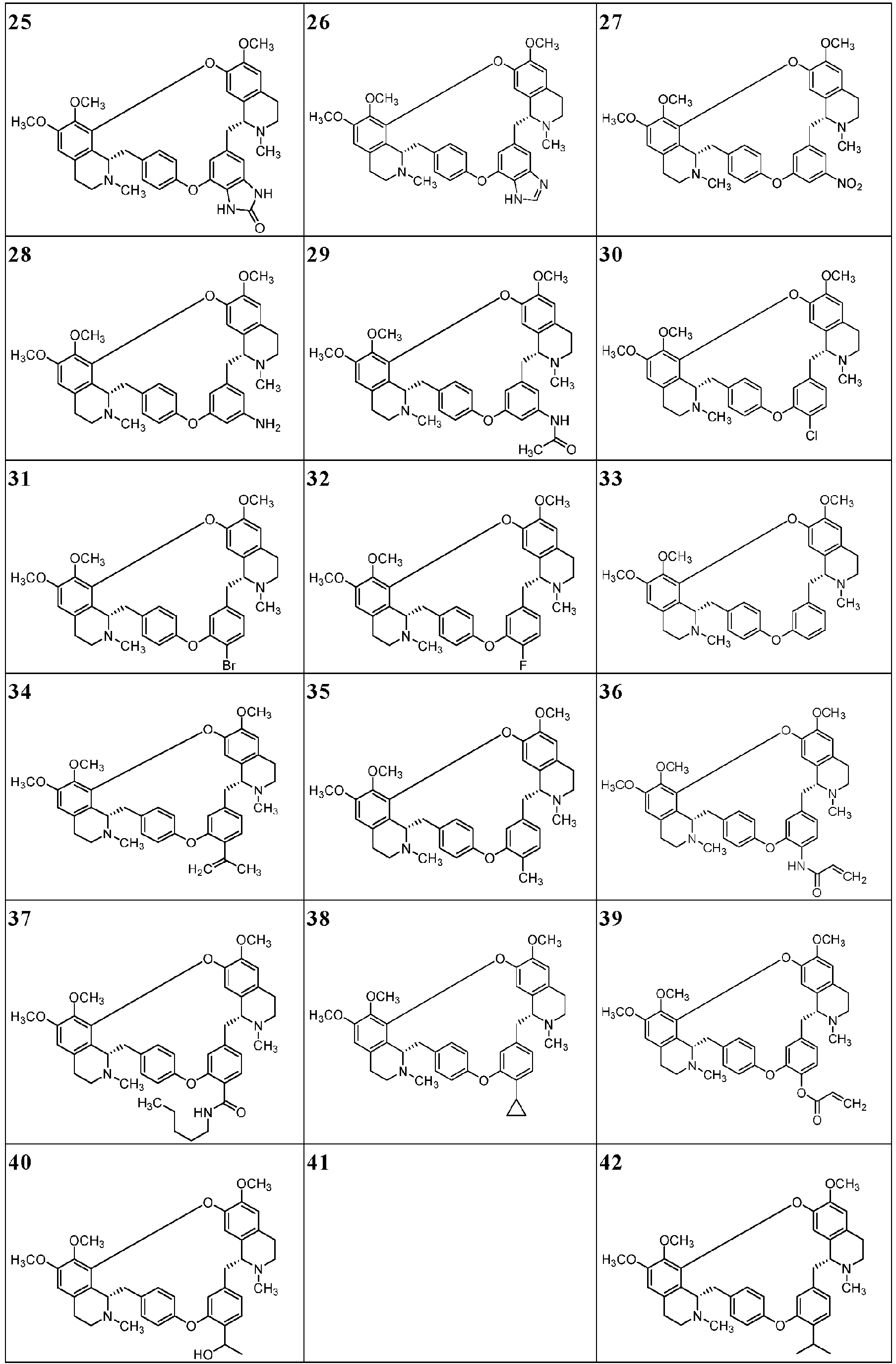

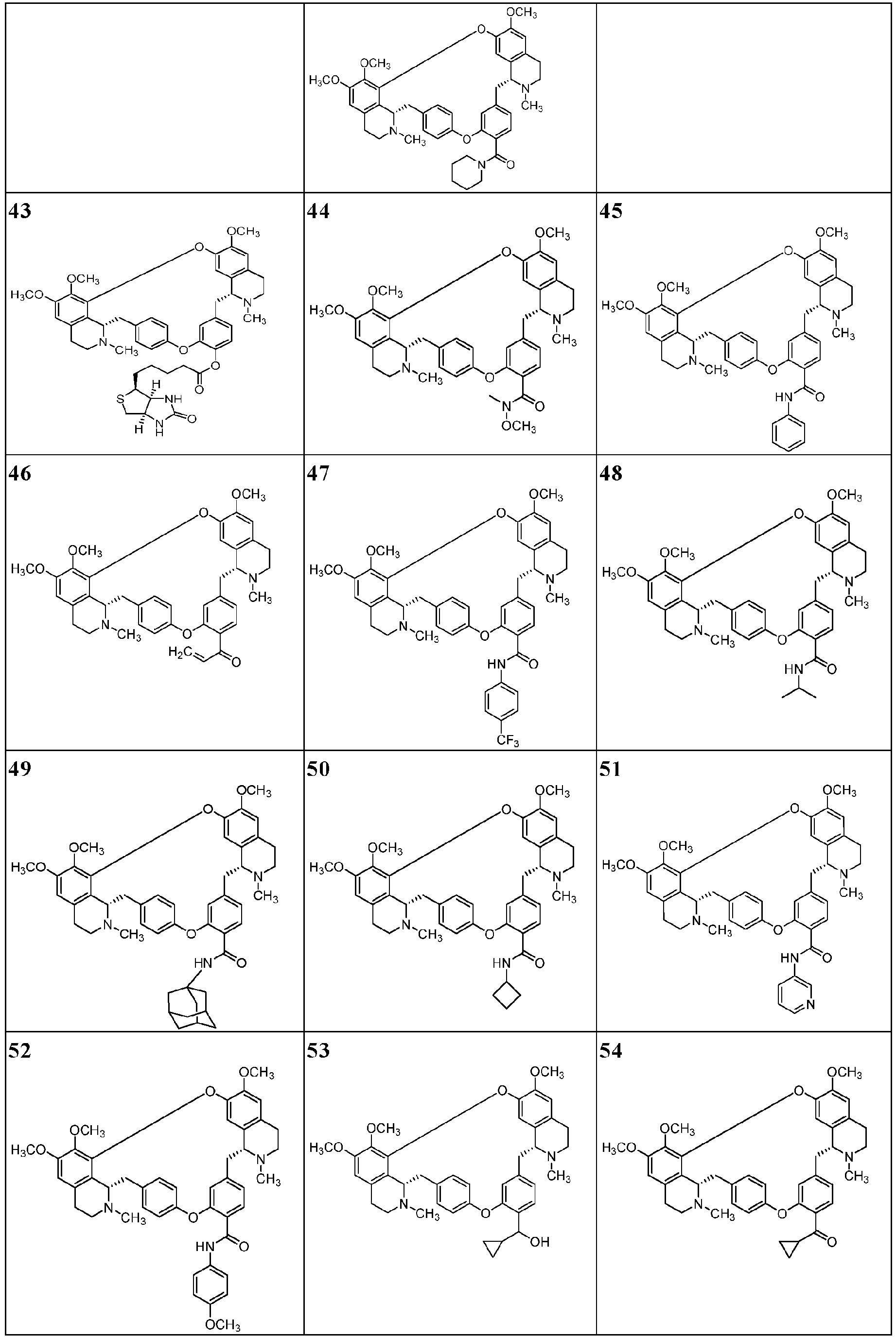

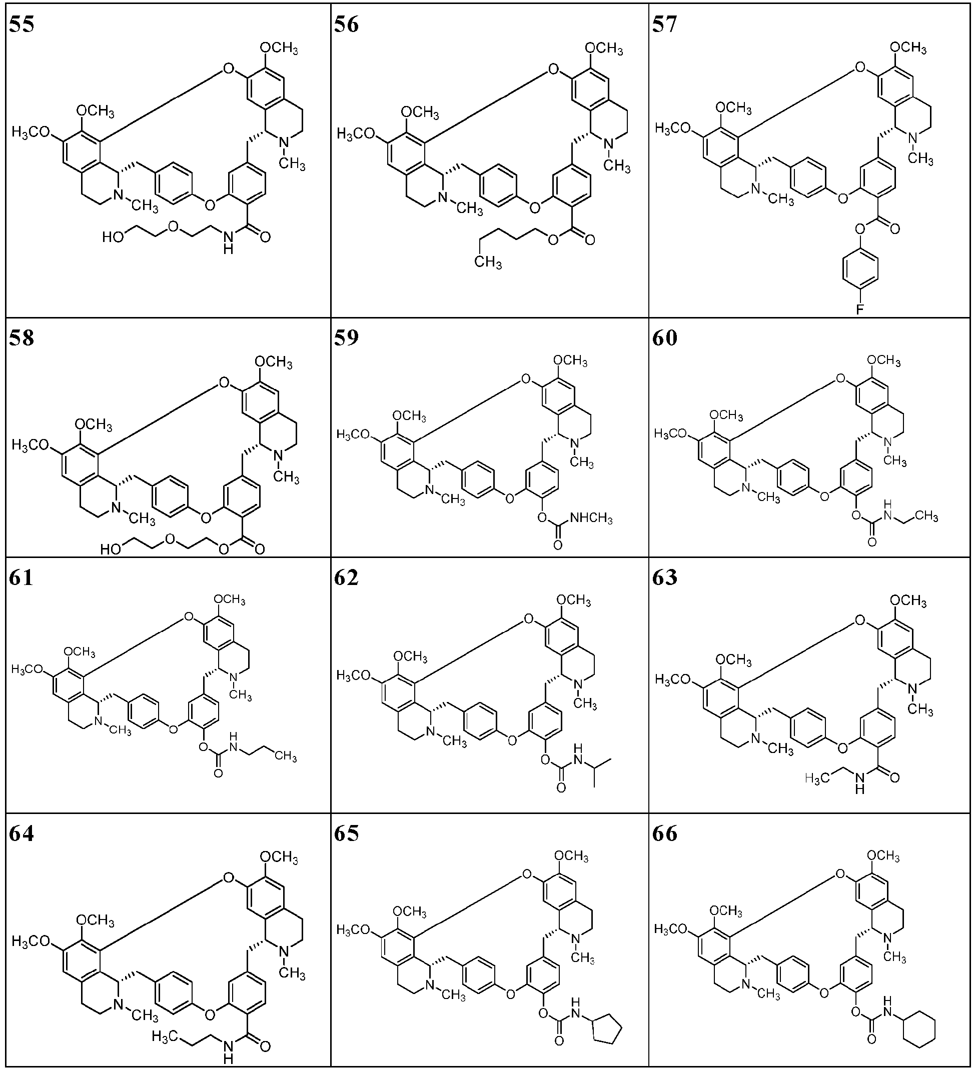

In another preferred embodiment, the compound of formula I is selected from the following compound:

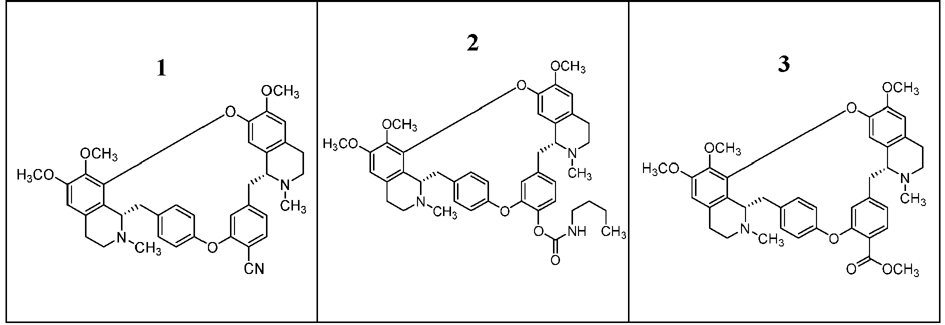

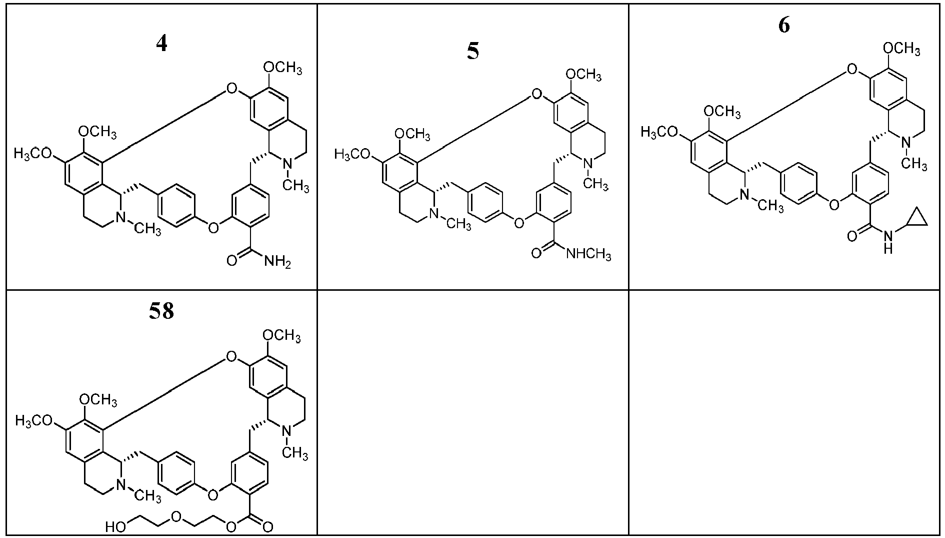

In the second aspect of the present invention, provided is a method for preparing cyclic bisbenzyl isoquinoline compound described in the first aspect, the method is selected from the group consisting of:
a) Cyclic bisbenzyl isoquinoline containing phenolic hydroxyl group is taken as a raw material, and subjected to an alkylation reaction with alkylation reagent to obtain the cyclic bisbenzyl isoquinoline compound;
b) Cyclic bisbenzyl isoquinoline containing phenolic hydroxyl group is taken as a raw material, and subjected to an acylation reaction with acylation reagent to obtain the cyclic bisbenzyl isoquinoline compound;
c) Oxyacanthine is taken as a raw material, and reacts with sulfonylation reagent in the presence of base to obtain compound (I-1b);

Compound(I-1b) and a coupling reagent undergo a coupling reaction to obtain the cyclic bisbenzyl isoquinoline compound 1-1 of formula 1-1, and the reaction is as shown in reaction formula 1: 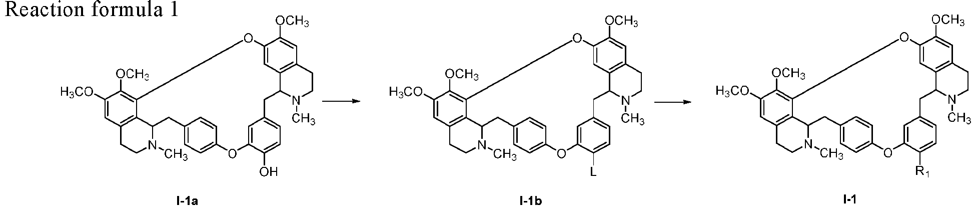
in Formula I-1, R₁ is selected from hydrogen, halogen, C₁-C₆alkylthiol, C₁-C₆ alkyl, C₃-C₆cycloalkyl, C₂-C₆ alkenyl, C₂-C₆alkynyl, C₁-C₆ alkyl-substituted amino, benzyl-substituted amino, cyano, carboxyl, aldehyde and C₁-C₆alkanoyl;
L in formula 1-1b is selected from the leaving group;
d) Oxyacanthine is taken as a raw material, and subjected to nitration, reduction, and ring-closing reaction to obtain the compound of formula (I-2), as shown in the following reaction formula 2: 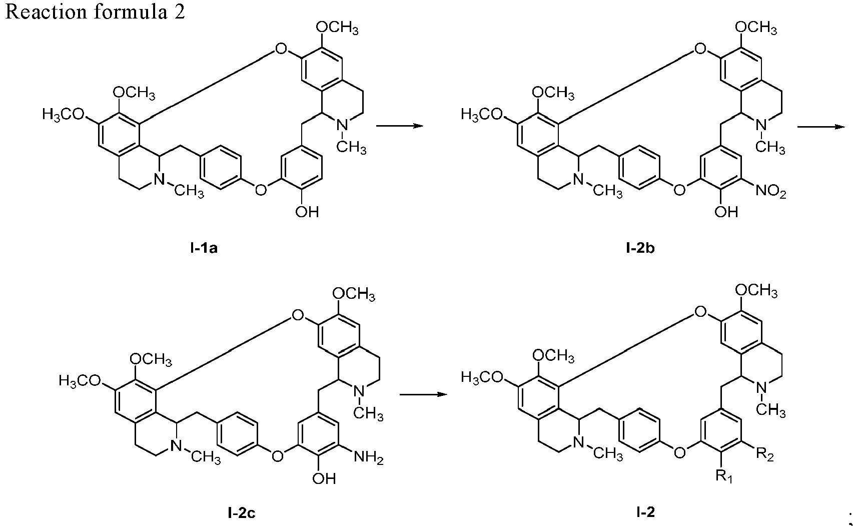
in Formula I-2, R₁ and R₂ together with the adjacent benzene ring form a benzo[5- to 6-membered monocyclic heterocycle]which is unsubstituted or substituted by 1 to 4 substituents;
e) Oxyacanthine is taken as raw material, and undergoes multi-steps of reaction to obtain the compound of formula (I-3), as shown in reaction formula 3: 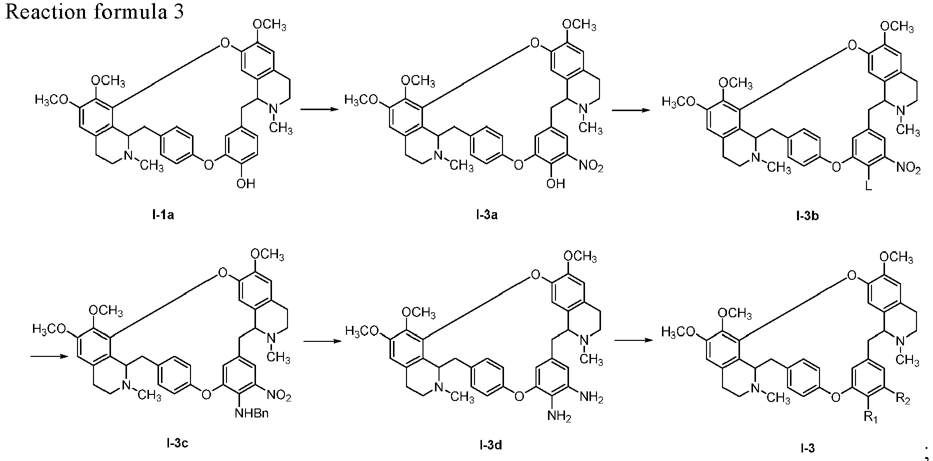
in Formula I-3, R₁ and R₂ together with the adjacent benzene ring form a benzo[5- to 6-membered monocyclic heterocycle] which is unsubstituted or substituted by 1 to 4 substituents;
f) The compounds obtained from methods a) to e) are subjected to a functional group conversion to obtain the cyclic bisbenzyl isoquinoline compounds.

In the above method, the raw material "oxyacanthine" is present in the form of oxyacanthine, or a pharmaceutically acceptable salt, an enantiomer, a diastereoisomer, a racemate, or a crystalline hydrate, a solvate thereof, or a mixture thereof.

In another preferred embodiment, the method is as follows: oxyacanthine is taken as raw material, and subjected to condensation acylation reaction with carboxylic acid to obtain the cyclic bisbenzyl isoquinoline compounds.

In another preferred embodiment, the condensation acylation reaction is carried out in the presence of a condensation agent, and the condensation agent is selected from the group consisting of N,N'-dicyclohexylcarbodiimide (DCC), 1 -ethyl-3-(3-dimethylaminopropyl)carbodiimide (EDCI), O-benzotriazole-N,N,N',N'-tetramethyluroniumtetrafluoroborate (TBTU), and combinations thereof.

In another preferred embodiment, the leaving group L is selected from the group consisting of C₁-C₆alkylsulfonyloxy, C₁-C₆haloalkylsulfonyloxy, phenylsulfonyloxy, naphthylsulfonyloxy, and combinations thereof, more preferably, L is methylsulfonyloxy or trifluoromethanesulfonyloxy.

In another preferred embodiment, the sulfonylation reagent is selected from: C₁-C₆alkylsulfonyl chloride, C₁-C₆alkylsulfonic anhydride, benzenesulfonyl chloride, benzenesulfonic anhydride, naphthylsulfonyl chloride, naphthylsulfonic anhydride, preferably methanesulfonyl chloride, methanesulfonic anhydride, trifluoromethanesulfonyl chloride and trifluoromethanesulfonic anhydride.

In another preferred embodiment, the solvent is selected from dichloromethane, tetrahydrofuran, N,N-dimethylformamide, methanol, ethanol, acetonitrile, toluene, acetone, dioxane and chloroform.

In another preferred embodiment, the base is selected from an inorganic base or an organic base.

In another preferred embodiment, the inorganic base is selected from: sodium hydroxide, potassium hydroxide, cesium hydroxide, barium hydroxide, potassium hydride, sodium hydride, sodium tert-butoxide, potassium tert-butoxide, potassium carbonate, sodium carbonate, or calcium carbonate; and the organic base is selected from: pyridine, triethylamine, diisopropylethylamine, N,N-dimethylaniline, and N,N-dimethylpyridine.

In another preferred embodiment, the coupling reaction is carried out in the presence of a palladium catalyst and a base.

In another preferred embodiment, the palladium catalyst is selected from: palladium acetate (Pd(OAc)₂), bis(triphenylphosphine)dichloropalladium ((Ph₃P)₂ PdCl₂), bis(benzonitrile)palladium chloride ((PhCN)₂PdCl₂), tetrakis(triphenylphosphine)palladium (Pd(PPh₃)₄), bis(triphenylphosphine)palladium acetate ((Ph₃P)₂Pd(Oac)₂), [1,2-Bis(diphenylphosphino)ethane]dichloropalladium((PdCl₂(dppe)₂)), Bis[1,2-bis(diphenylphosphino)ethane]palladium(Pd(dppe)₂), Bis(dibenzylideneacetone)palladium(Pd(dba)₂), Tris(dibenzylideneacetone)dipalladium(Pd₂(dba)₃), dichloro[1,3-bis(diphenylphosphino)propane]palladium(PdCb(dippp)), [1,1'-Bis(diphenylphosphino)ferrocene]dichloropalladium(II)(Pd(dppfCl₂), and combinations thereof.

In another preferred embodiment, other reaction solvents that do not interfere with the reaction may also be included in reaction formula 1.

In another preferred embodiment, a suitable ligand may also be added to the reaction formula 1 as a reaction promoter.

In another preferred embodiment, the suitable ligand is selected from: 2,2'-diphenylphosphino-1,1'-binaphthyl (BINAP), tri-tert-butyl (P(t-Bu)₃), 1,1-bis-(diphenylphosphino)ferrocene (dppf), 2-dicyclohexylphosphino-2,4,6-triisopropylbiphenyl (x-phos), 4,5-bis(diphenylphosphino)-9,9-dimethylxanthene (Xantphos), tri-tert-butylphosphinetetrafluoroborate and tris(2-methylphenyl)phosphine (P(o-tolyl)₃), and combinations thereof.

In another preferred embodiment, the coupling reagent is selected from the group consisting of C₁-C₆alkylboronic acid, cyclopropylboronic acid, benzylamine, potassium cyanide, zinc cyanide, tributylvinyltin, CO, CO₂, formic acid, sodium formate, lithium formate, sodiumC₁-C₆ alkylthiolate, sodium methanesulfonate, and combinations thereof.

In another preferred embodiment, the benzo[5 to 6-membered monocyclic heterocycle] is selected from:

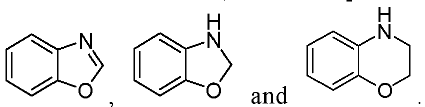

In another preferred embodiment, for the benzo[5 to 6-membered monocyclic heterocycle], the substituent is selected from halogen, hydroxyl, thiol, amino, oxo-(=O), thio-(=S), andC₁-C₆ alkyl; and the heterocycle contains from 1 to 3 heteroatoms selected from N, O and S.

In another preferred embodiment, the nitration reagent in the nitration reaction is selected from: nitric acid, a mixture of concentrated sulfuric acid and nitric acid, a mixture of nitric acid, sodium nitrate and concentrated sulfuric acid, a mixture of potassium nitrate and concentrated sulfuric acid, a mixture of sodium nitrite and concentrated sulfuric acid, a mixture of acetic acid and nitric acid.

In another preferred embodiment, the nitration reagent in the nitration reaction is a mixture of acetic acid and nitric acid.

In another preferred embodiment, the mixing ratio of the mixture is not limited.

In another preferred embodiment, the temperature of the nitration reaction is -20°C to room temperature.

In another preferred embodiment, the reduction reaction is catalyzed by Pd/C,

In another preferred embodiment, the reduction reaction uses hydrogen, ammonium formate or formic acid as the reducing agent,

In another preferred embodiment, the reduction reaction is carried out in lower alcohol or mixed solvent of lower alcohol-water.

In another preferred embodiment, the lower alcohol is selected from: methanol, ethanol, isopropanol and combinations thereof.

In another preferred embodiment, the reduction reaction is carried out at 0 to 100 °C, preferably 0 to 40 °C.

In another preferred embodiment, the reduction reaction is carried out at atmospheric pressure.

In another preferred embodiment, the reduction reaction is hydrogenated for 1 to 10h.

In another preferred embodiment, the ring-closing reaction is carried out in the presence of a ring-closing reagent.

In another preferred embodiment, the ring-closing reagent comprises: phosgene, triphosgene, 1,1'-carbonyldiimidazole (CDI), urea, carbon tetrabromide, formic acid, trimethylorthoformate, trimethyl orthoacetate, triethylorthoacetate, acetochlorine, chloroacetochlorine, bromoacetyl bromide, bromoacetyl chloride, and combinations thereof.

In another preferred embodiment, the ring-closing reaction is carried out in the presence or absence of base.

In another preferred embodiment, the base is selected from: an inorganic base or an organic base; wherein the inorganic base is selected from sodium hydroxide, potassium hydroxide, potassium hydride, sodium hydride, sodium tert-butoxide, potassium tert-butoxide, potassium carbonate, sodium carbonate, cesium carbonate, and sodium bicarbonate; wherein the organic base is selected from pyridine, triethylamine, diisopropylethylamine, N,N-dimethylaniline and N,N-dimethylpyridine;

In another preferred embodiment, other reaction solvents that do not interfere with the reaction may also be included in reaction formula 2.

In another preferred embodiment, for the benzo[5 to 6-membered monocyclic heterocycle], the substituent is selected from halogen, hydroxyl, thiol, amino, oxo(=O), thio(=S), and C₁-C₆ alkyl; and the heterocycle contains from 1 to 3 heteroatoms selected from N, O and S.

In another preferred embodiment, the benzo[5 to 6-membered monocyclic heterocycle] is selected from:

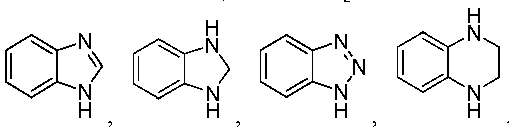

In the formula I-3b, L is a leaving group.

In another preferred embodiment, the leaving group L is selected from the group consisting of:C₁-C₆alkylsulfonyloxy, C₁-C₆haloalkylsulfonyloxy, phenylsulfonyloxy, and naphthylsulfonyloxy, preferably, methylsulfonyloxy and trifluoromethanesulfonyloxy.

In another preferred embodiment, the method e) includes the following steps:
e1) Compound of formula (I-1a) and a nitration reagent undergo an ortho-nitration reaction to obtain a compound of formula (I-3a);
e2) Compound of formula (I-3a) reacts with sulfonylation reagent in the presence of a base in a suitable solvent to obtain a compound of formula (I-3b);
e3) Compound of formula (I-3b) and benzylamine undergo a Buchwald-Hartig reaction to obtain a compound of formula (I-3c);
e4) Compound of formula (I-3c) undergoes a reduction reaction to obtain a compound of formula (I-3d);
e5) Compound of formula (I-3d) undergoes a ring-closing reaction to obtain a compound of formula (I-3).

In another preferred embodiment, the nitration reagent in step e1) is a mixture of concentrated sulfuric acid and nitric acid, a mixture of nitric acid, sodium nitrate and concentrated sulfuric acid, a mixture of potassium nitrate and concentrated sulfuric acid, a mixture of sodium nitrite and concentrated sulfuric acid, a mixture of acetic acid and nitric acid, preferably a mixture of acetic acid and nitric acid.

In another preferred embodiment, the mixing ratio of the mixture is not limited.

In another preferred embodiment, the temperature of the nitration reaction is -20°C to room temperature.

In another preferred embodiment, the time of the nitration reaction is from 10 minutes to 12 hours.

In another preferred embodiment, the sulfonation reagent in step e2) is selected from: C₁-C₆alkylsulfonyl chloride, C₁-C₆alkylsulfonic anhydride, benzenesulfonyl chloride, benzenesulfonic anhydride, naphthylsulfonylchloride, naphthylsulfonicanhydride, preferably methanesulfonyl chloride, methanesulfonic anhydride, trifluoromethanesulfonyl chloride and trifluoromethanesulfonic anhydride.

In another preferred embodiment, the solvent in step e2) is selected from: dichloromethane, tetrahydrofuran, N,N-dimethylformamide, methanol, ethanol, acetonitrile, toluene, acetone, dioxane and chloroform.

In another preferred embodiment, the base in step e2) is selected from an inorganic base and an organic base; wherein the inorganic base is selected from sodium hydroxide, potassium hydroxide, cesium hydroxide, barium hydroxide, potassium hydride, sodium hydride, sodium tert-butoxide, potassium tert-butoxide, potassium carbonate, sodium carbonate and calcium carbonate; wherein the organic base is selected from pyridine, triethylamine, diisopropylethylamine, N,N-dimethylaniline and N,N-dimethylpyridine.

In another preferred embodiment, the coupling reaction in step e3) is carried out in the presence of a palladium catalyst and a base.

In another preferred embodiment, the palladium catalyst is selected from: palladium acetate (Pd(OAc)2), bis(triphenylphosphine)dichloropalladium ((Ph₃P)₂ PdCl₂), bis(benzonitrile)palladium chloride ((PhCN)₂PdCl₂), tetrakis(triphenylphosphine)palladium (Pd(PPh₃)₄), bis(triphenylphosphine)palladium acetate ((Ph₃P)₂Pd(oAc)₂), [1,2-Bis(diphenylphosphino)ethane]dichloropalladium((PdCl₂(dppe)₂)), Bis[1,2-bis(diphenylphosphino)ethane]palladium(Pd(dppe)₂), Bis(dibenzylideneacetone)palladium(Pd(dba)₂), Tris(dibenzylideneacetone)dipalladium(Pd₂(dba)₃), dichloro[1,3-bis(diphenylphosphino)propane]palladium(PdCl₂(dippp)), [1,1'-Bis(diphenylphosphino)ferrocene]dichloropalladium(II)(Pd(dppfCl₂), and combinations thereof.

In another preferred embodiment, the base is selected from: sodium bis(trimethylsilyl)amide, potassium tert-butoxide, sodium tert-butoxide, cesium carbonate, potassium phosphate, sodium phosphate, sodium methanol, sodium ethanol, potassium hydroxide, sodium hydroxide, potassium fluoride, sodium fluoride, tetrabutylammonium fluoride (TBAF) , sodium acetate, potassium acetate, cesium carbonate, potassium carbonate, sodium carbonate, and combinations thereof.

In another preferred embodiment, the reaction may also comprise other reaction solvents that do not interfere with the reaction.

In another preferred embodiment, in step e3),the reaction can also be carried out by adding a suitable ligand as a reaction promoter.

In another preferred embodiment, the suitable ligand is selected from the group consisting of: 2,2'-diphenylphosphino-1,1'-binaphthyl (BINAP), tri-tert-butyl (P(t-Bu)₃), 1,1-bis-(diphenylphosphino)ferrocene (dppf), 2-dicyclohexylphosphino-2,4,6-triisopropylbiphenyl (x-phos), 4,5-bis(diphenylphosphino)-9,9-dimethylxanthene (Xantphos), tri-tert-butylphosphinetetrafluoroborate and tris(2-methylphenyl)phosphine (P(o-tolyl)₃).

In another preferred embodiment, the reduction reaction in step e4) is catalyzed by Pd/C, palladium hydroxide or palladium hydroxide charcoal.

In another preferred embodiment, the reduction reaction in step e4) uses ammonium formate or hydrogen as reducing agent.

In another preferred embodiment, the reduction reaction is carried out in lower alcohol or mixed solvent of lower alcohol-water.

In another preferred embodiment, the lower alcohol is selected from: methanol, ethanol, isopropanol and combinations thereof.

In another preferred embodiment, the reduction reaction is carried out at 20 to 100°C.

In another preferred embodiment, the reduction reaction is carried out at atmospheric pressure.

In another preferred embodiment, the ring-closing reaction in step e5) is carried out in the presence of a ring-closing reagent.

In another preferred embodiment, the ring-closing reagent is selected from the group consisting of: phosgene, triphosgene, 1,1'-carbonyldiimidazole (CDI), urea, formic acid, sodium nitrite, carbon disulfide, thiophosgene, triethylorthoacetate, chloroacetic acid, bromoacetic acid, ethyl bromoacetate, methyl bromoacetate, chloroacetamide, and combinations thereof.

In another preferred embodiment, the ring-closing reaction is carried out in the presence or absence of a base.

In another preferred embodiment, the base is selected from an inorganic base and an organic base.

In another preferred embodiment, the inorganic base is selected from sodium hydroxide, potassium hydroxide, potassium hydride, sodium hydride, sodium tert-butoxide, potassium tert-butoxide, potassium carbonate, sodium carbonate, cesium carbonate, and sodium bicarbonate; wherein the organic base is selected from pyridine, triethylamine, diisopropylethylamine, N,N-dimethylaniline and N,N-dimethylpyridine;

In another preferred embodiment, other reaction solvents that do not interfere with the reaction may also be included in step e5).

In another preferred embodiment, the leaving group L is selected from the group consisting of: C₁-C₆alkylsulfonyloxy, C₁-C₆haloalkylsulfonyloxy, phenylsulfonyloxy, naphthylsulfonyloxy; more preferably methylsulfonyloxy and trifluoromethanesulfonyloxy.

In another preferred embodiment, the sulfonylating reagent is selected from: C₁-C₆alkylsulfonyl chloride, C₁-C₆alkylsulfonic anhydride, benzenesulfonyl chloride, benzenesulfonic anhydride, naphthylsulfonylchloride, naphthylsulfonicanhydride, preferably methanesulfonyl chloride, methanesulfonic anhydride, trifluoromethanesulfonyl chloride and trifluoromethanesulfonic anhydride. The solvent is selected from dichloromethane, tetrahydrofuran, N,N-dimethylformamide, methanol, ethanol, acetonitrile, toluene, acetone, dioxane and chloroform.

In another preferred embodiment, the base is selected from an inorganic base and an organic base.

In another preferred embodiment, the inorganic base is selected from: sodium hydroxide, potassium hydroxide, cesium hydroxide, barium hydroxide, potassium hydride, sodium hydride, sodium tert-butoxide, potassium tert-butoxide, potassium carbonate, sodium carbonate, and calcium carbonate; and the organic base is selected from: pyridine, triethylamine, diisopropylethylamine, N,N-dimethylaniline, and N,N-dimethylpyridine.

In another preferred embodiment, the coupling reaction is carried out in the presence of a palladium catalyst and a base.

In another preferred embodiment, the palladium catalyst is selected from the group consisting of: palladium acetate (Pd(OAc)₂), bis(triphenylphosphine)dichloropalladium ((Ph₃P)₂ PdCl₂), bis(benzonitrile)palladium chloride ((PhCN)₂PdCl₂), tetrakis(triphenylphosphine)palladium (Pd(PPh₃)₄), bis(triphenylphosphine)palladium acetate ((Ph₃P)₂Pd(OAc)₂), [1,2-Bis(diphenylphosphino)ethane]dichloropalladium((PdCl₂(dppe)₂)), Bis[1,2-bis(diphenylphosphino)ethane]palladium(Pd(dppe)₂), Bis(dibenzylideneacetone)palladium(Pd(dba)₂), Tris(dibenzylideneacetone)dipalladium(Pd₂(dba)₃), dichloro[1,3-bis(diphenylphosphino)propane]palladium(PdCl₂(dippp)), [1,1'-Bis(diphenylphosphino)ferrocene]dichloropalladium(II)(Pd(dppfCl₂), and combinations thereof.

In another preferred embodiment, the base is selected from: sodium bis(trimethylsilyl)amide, potassium tert-butoxide, sodium tert-butoxide, cesium carbonate, potassium phosphate, sodium phosphate, sodium methanol, sodium ethanol, potassium hydroxide, sodium hydroxide, potassium fluoride, sodium fluoride, tetrabutylammonium fluoride (TBAF), sodium acetate, potassium acetate, cesium carbonate, potassium carbonate, sodium carbonate, and combinations thereof.

In another preferred embodiment, other reaction solvents that do not interfere with the reaction may also be included in the reaction formula 4.

In another preferred embodiment, a suitable ligand may also be added to the reaction formula 4 as a reaction promoter for the reaction.

In another preferred embodiment, the suitable ligand is selected from: 2,2'-diphenylphosphino-1,1'-binaphthyl (BINAP), tri-tert-butyl (P(t-Bu)₃), 1,1-bis-(diphenylphosphino)ferrocene (dppf), 2-dicyclohexylphosphino-2,4,6-triisopropylbiphenyl (x-phos), 4,5-bis(diphenylphosphino)-9,9-dimethylxanthene (Xantphos), tri-tert-butylphosphinetetrafluoroborate and tris(2-methylphenyl)phosphine (P(o-tolyl)₃). The coupling reagents include, but are not limited to, C₁-C₆alkylboronic acid, cyclopropylboronic acid, benzylamine, potassium cyanide, zinc cyanide, tributylvinyltin, CO, CO₂, formic acid, sodium formate, lithium formate, sodium C₁-C₆alkylthiolate, and sodium methanesulfonate.

In another preferred embodiment, the functional group conversion reaction comprises: an oxidative hydrolysis reaction, a borohydride-oxidation reaction, a condensation acylation reaction, a reduction reaction, an acylation reaction, an esterification reaction, a Grignard reaction, a chlorination reaction and a bromination reaction.

In another preferred embodiment, the oxidative hydrolysis reaction is carried out in the presence of an oxidizing agent and a base.

In another preferred embodiment, the oxidative hydrolysis system comprises: hydrogen peroxide/sodium hydroxide, hydrogen peroxide/potassium hydroxide, hydrogen peroxide/potassium carbonate, hydrogen peroxide/sodium carbonate.

In another preferred embodiment, the borohydride-oxidation reaction is the addition of an olefinic group with a boron reagent followed by oxidative hydrolysis to afford an alcohol.

In another preferred embodiment, the boron reagent comprises: borane, 9-BBN;

In another preferred embodiment, the condensation acylation reaction is carried out in the presence of a condensing agent.

In another preferred embodiment, the condensing agent comprises: N,N'-dicyclohexylcarbodiimide (DCC), 1-ethyl-3-(3-dimethylaminopropyl)carbodiimide (EDCI), O-benzotriazole-N,N,N',N'-tetramethylureatetrafluoroborate (TBTU) .

In another preferred embodiment, the reduction reaction is carried out in the presence of a reducing agent.

In another preferred embodiment, the reducing agent comprises: hydrogen, ammonium formate, sodium borohydride, potassium borohydride, diisobutylhydroaluminum (DIBAL), borane.

In another preferred embodiment, the acylation reaction is carried out in the presence of an acylation reagent.

In another preferred embodiment, the acylation reagent comprises: acetyl chloride, acetic anhydride, propionyl chloride, propionic anhydride, methanesulfonyl chloride and the like.

In another preferred embodiment, the esterification reaction system includes, but is not limited to: sulfoxide chloride/methanol, sulfoxide chloride/ethanol, and the like.

In another preferred embodiment, the chlorination reaction is carried out in the presence of a chlorination reagent.

In another preferred embodiment, the chlorination reagent includes, but is not limited to: sulfoxide chloride, phosphorus pentachloride, and N-chlorosuccinimide (NCS), and the like.

In another preferred embodiment, the Grignard reaction is carried out in the presence of a Grignard reagent.

In another preferred embodiment, the Grignard reagent includes, but is not limited to: methyl magnesium bromide, methyl magnesium chloride, methyl magnesium iodide, and the like.

In another preferred embodiment, the bromination reaction is carried out in the presence of a bromination reagent.

In another preferred embodiment, the bromination reagent includes, but is not limited to: monomeric bromine, N-bromosuccinimide (NBS), and the like.

In the third aspect of the present invention, provided is a pharmaceutical composition, comprising:
(A1) A first active ingredient, the first active ingredient comprising a therapeutically effective amount of one or a mixture of more of the cyclic bisbenzyl isoquinoline compound shown in Formula I as described in any one of the first aspect, a enantiomer, a diastereoisomer, a racemate, a pharmaceutically acceptable salt, a crystalline hydrate, and a solvate thereof, and
(B) pharmaceutically acceptable carriers.

In another preferred embodiment, the pharmaceutical composition further comprises (A2) a second active ingredient.

In another preferred embodiment, the second active ingredient is selected from: (Y1) an RNA replicase inhibitor (e.g., Remdesivir (Remdesivir or GS-5734), favipiravir, Molnupiravir); (Y2) a 3CL enzyme inhibitor (e.g., nirmatrelvir); (Y3) an antibody; and (Y4) any combination of Y1 to Y3.

In another preferred embodiment, the antibody comprises an antibody against coronavirus.

In the fourth aspect of the present invention, provided is a use of the cyclic bisbenzyl isoquinoline compound described in the first aspect or the pharmaceutical composition described in the third aspect, i.e., in the preparation of an inhibitor against viral replication, inflammation, fibrosis, and abnormal differentiation of T-cells; and/or in the preparation of a medication for preventing and/or treating associated diseases caused by viral infection, inflammation-related diseases(pneumonia, nonalcoholic steatohepatitis, colitis, nephritis, pancreatitis, myocarditis, arthritis, inflammatory pain), fibrosis-related diseases (pulmonary fibrosis, silicosis, hepatic fibrosis, renal fibrosis, myocardial fibrosis, dermatofibrosis, retinal fibrosis, myelofibrosis)), autoimmune diseases associated with abnormal differentiation of Th1 and/or Th17 (psoriasis, rheumatoid arthritis, systemic lupus erythematosus, multiple sclerosis, neuromyelitisoptica, myasthenia gravis, ankylosing spondylitis, ulcerative colitis, Crohn's disease, delayed type hypersensitivity, graft-versus-host disease, etc.), osteoporosis, and neurodegenerative disease.

In another preferred embodiment, the virus is selected from: Filoviridae, Flaviviridae, Paramyxoviridae, Arenaviridae, Coronaviridae, and combinations thereof.

In another preferred embodiment, the Filoviridae is selected from: Marburg virus and Ebola virus.

In another preferred embodiment, the Coronaviridae is selected from the group consisting of: coronaviruses infecting humans, severe acute respiratory syndrome coronavirus SARS-CoV, the 2019 novel coronavirus and its variants (SARS-CoV-2) , Middle East respiratory syndrome coronavirus MERS-CoV, coronaviruses causing common cold, and combinations thereof.

In another preferred embodiment, the coronaviruse causing common cold is selected from: Human coronavirus OC₄₃, Human coronavirus 229E, Human coronavirus NL63, and Human coronavirus HKUl.

In another preferred embodiment, the associated disease caused by coronavirus infection is selected from the group consisting of: human coronavirus-induced common cold, high-risk symptomatic infections, respiratory infections, pneumonia and complications thereof, SARS-CoV-2-induced novel coronavirus pneumonia (Corona Virus Disease 2019, COVID-19), SARS-CoV-2-induced novel coronavirus infectious disease, and combinations thereof.

In another preferred embodiment, the associated disease caused by 2019 novel coronavirus infection is selected from the group consisting of: respiratory tract infection, pneumonia and complications thereof, and combinations thereof.

In another preferred embodiment, the use is for the preparation of (a) an inhibitor for inhibiting the replication of the 2019 novel coronavirus and its variants (SARS-CoV-2); and/or (b) a medicament for treating and/or preventing, or alleviating a disease associated with the infection caused by the 2019 novel coronavirus (SARS-CoV-2).

In another preferred embodiment, the disease is pneumonia, non-alcoholic steatohepatitis, colitis, nephritis, pancreatitis, myocarditis, arthritis, inflammatory pain, pulmonary fibrosis, silicosis, hepatic fibrosis, renal fibrosis, myocardial fibrosis, dermal fibrosis, retinal fibrosis, myelofibrosis, psoriasis, rheumatoid arthritis, systemic lupus erythematosus, multiple sclerosis, neuromyelitisoptica, severe myasthenia gravis, ankylosing spondylitis, ulcerative colitis, Crohn's disease, delayed type hypersensitivity, graft-versus-host disease, osteoporosis, neurodegenerative disease, or a combination thereof.

In another preferred embodiment, the use is for the preparation of a medicament for treating and/or preventing, alleviating a disease associated with inflammation, fibrosis, or abnormal differentiation of T-cells.

It should be understood that within the scope of the present invention, the above-mentioned technical features of the present invention and the technical features specifically described in the following (such as embodiments) can be combined with each other to form a new or preferred technical solution. Limited to space, it will not be repeated here.

### Description of the drawings

Figure 1 shows the inhibitory effect of compounds 1, 2, 3, 4, 6, and 58 against Th1/Th17 differentiation.
Figure 2 shows that compounds 1, 2, 3, 4, 6, and 58 did not affect cell viability in the LPS-induced inflammation model.
Figure 3 shows that compounds 1, 2, 4, and 58 significantly inhibited inflammatory factors in the LPS-induced inflammation model.
Figure 4 shows that compounds 1, 2, 3, 4, 6, and 58 did not affect cell viability in the TNF-α-induced inflammation model.
Figure 5 shows that compounds 1, 2, 3, 4, 6, and 58 significantly inhibited inflammatory factors in the TNF-α-induced inflammation model.
Figure 6 shows that compound 4 significantly inhibited LPS-induced acute inflammation in mice.
Figure 7 shows that compound 1 significantly increased cell survival in the TNF-α-induced A549 lung cell injury model.
Figure 8 shows that compounds 3, and 58 significantly inhibited the damage factors in the TNF-α-induced A549 lung cell injury model.
Figure 9 shows that compounds 1, 2, 3, 4, 6, and 58 significantly alleviated H2O2-induced ALM12 hepatocyte injury.
Figure 10 and 11 show that compounds 1, 2, and 4 alleviated TGF-β1-induced fibrosis in A549 lung epithelial cells.
Figure 12 shows that compounds 2, and 4 alleviated CC14-induced acute liver fibrosis in mice.

### Detailed Description of the Invention

After extensive and in-depth research and through extensive screening, the present inventors have for the first time unexpectedly developed a class of active compositions that can effectively inhibit the replication of coronaviruses such as the 2019 novel coronavirus (SARS-CoV-2). Experiments showed that the active ingredients of the present invention (cyclic bisbenzyl isoquinoline compounds of Formula I, or a pharmaceutically acceptable salt, an enantiomer, a diastereoisomer, a racemate, or a crystalline hydrate, a solvate thereof, or mixtures thereof) can efficiently inhibit the replication and viability of coronaviruses such as the 2019 novel coronavirus (SARS-CoV-2). The present invention has been accomplished on this basis.

Unexpectedly, compared with natural compounds such as oxyacanthine, berbamine, and tetrandrine, the cyclic bisbenzyl isoquinoline compound described in Formula I have significantly improved anti-coronaviral activity (up to about 10-fold or more increase in antiviral activity) and/or lower cytotoxicity, and thus, have a better therapeutic index SI for the prevention and treatment of coronavirus, such as SARS-CoV-2 virus, and have good prospects for clinical application.

In addition to this, the inventors have explored other potential effects of the cyclic bisbenzyl isoquinoline compounds, which have been found have inhibitory activity against CD4⁺ T cell differentiation. Experiments showed that the active ingredients of the present invention (cyclic bisbenzyl isoquinoline compound of Formula I, or a pharmaceutically acceptable salt, an enantiomer, a diastereoisomer, a racemate, or a crystalline hydrate, a solvate thereof, or mixtures thereof) can effectively inhibit diseases associated with viral infection, inflammation-related diseases(pneumonia, nonalcoholic steatohepatitis, colitis, nephritis, pancreatitis, myocarditis, arthritis, inflammatory pain), fibrosis-related diseases (pulmonary fibrosis, silicosis, hepatic fibrosis, renal fibrosis, myocardial fibrosis, dermatofibrosis, retinal fibrosis, myelofibrosis)), autoimmune diseases associated with abnormal differentiation of Th1 and/or Th17 (psoriasis, rheumatoid arthritis, systemic lupus erythematosus, multiple sclerosis, neuromyelitisoptica, myasthenia gravis, ankylosing spondylitis, ulcerative colitis, Crohn's disease, delayed type hypersensitivity, graft-versus-host disease, etc.), osteoporosis, and neurodegenerative disease.

### Terms

As used herein, "compounds of the present invention", "cyclic bisbenzyl isoquinoline compounds", "compounds of the present invention with inhibitory activity against coronavirus", "compounds of the present invention for inhibiting coronavirus replication" are used interchangeably to refer to cyclic bisbenzyl isoquinoline compounds having excellent inhibition against coronavirus replication, including the compound of Formula I, or a pharmaceutically acceptable salt, an enantiomer, a diastereoisomer, a racemate, or a crystalline hydrate, a solvate thereof, or mixtures thereof. The compounds of the present invention do not include natural compounds (e.g., tetrandrine, fangchinoline, and oxyacanthine).

As used herein, "formulation of the invention" means a formulation containing the compound of the invention.

As used herein, the term "including" or its variant forms such as "comprising" or "containing", etc., is understood to include the elements or components described and does not exclude other elements or other components.

As used herein, the terms "novel coronavirus," "2019-nCov," or "SARS-CoV-2" are used interchangeably, and the 2019 novel coronavirus is the seventh coronavirus known to infect human and causes COVID-19, and is one of the most serious infectious diseases threatening human health worldwide.

As used herein, halogen generally refers to fluorine, chlorine, bromine and iodine; preferably fluorine, chlorine or bromine; more preferably fluorine or chlorine.

C₁-C₆ alkyl refers to a straight or branched saturated hydrocarbonyl containing 1-6 carbon atoms, e.g., methyl, ethyl, n-propyl, isopropyl, n-butyl, isobutyl, tert-butyl, sec-butyl, n-pentyl, 1-ethylpropyl, isopentyl, neopentyl, isohexyl, 3-methylpentyl or n-hexyl, etc., preferably methyl, ethyl, n-propyl, isopropyl, butyl, isobutyl, or tert-butyl.

C₁-C₆haloalkyl means that the hydrogen atoms of a straight or branched saturated hydrocarbonyl containing 1-6 carbon atoms are substituted by one or more identical or different halogen atoms, such as trifluoromethyl, fluoromethyl, difluoromethyl, chloromethyl, bromomethyl, dichlorofluoromethyl, chloroethyl, bromopropyl, 2-chlorobutyl, or pentafluoroethyl.

C₁-C₆Alkoxy refers to a straight or branched alkoxy containing 1-6 carbon atoms, such as, for example, methoxy, ethoxy, n-propoxy, isopropoxy, n-butoxy, isobutoxy, tert-butoxy, sec-butoxy, n-pentyloxy, isopentyloxy, neopentyloxy, isohexyloxy, 3-methylpentyloxy or n-hexyloxy, etc., preferably methoxy, ethoxy, propoxy, isopropoxy, n-butoxy, isobutoxy or tert-butoxy.

C₁-C₆haloalkoxy means that the hydrogen atom of a straight or branched alkoxy containing 1-6 carbon atoms is substituted by one or more identical or different halogen atoms, e.g., -OCF₃, -OCH₂CH₂Cl, -OCHBrCH₂Cl, or -OCF₂CF₃, etc.

C1-C6 alkylthiol refers to a straight or branched alkylthiol containing 1-6 carbon atoms, such as, methylthio, ethylthio, n-propylthio, isopropylthio, n-butylthio, isobutylthio, tert-butylthio, sec-butylthio, n-pentylthio, isopentylthio, neopentylthio, or n-hexylthio and the like, preferably methylthio, ethylthio, n-propylthio, isopropylthio, n-butylthio, isobutylthio, or tert-butylthio.

C₂-C₆ alkenyl refers to a straight or branched unsaturated hydrocarbonyl containing 1 to 3 double bonds and 2 to 6 carbon atoms in both cis and trans configurations, e.g., vinyl, 1-propenyl, 2-propenyl, 1-methyl-1-propenyl, 2-methyl-1-propenyl, 2-methyl-2-propenyl, 1-butenyl, 2-butenyl, 3-butenyl, 1-pentenyl, 2-pentenyl, 3-pentenyl, 4-pentenyl, 1,3-butadienyl, 1,3-pentadienyl, 1-hexenyl, 2-hexenyl, 3-hexenyl, 4-hexenyl, 5-hexenyl, 3,3-dimethyl-1-propenyl, or 2-ethyl-1-propenyl and the like.

C₂ -C₆alkynyl refers to a straight or branched alkynyl containing 2-6 carbon atoms, e.g., ethynyl, 2-propynyl, 2-butynyl, 3-butynyl, 1-methyl-2-propynyl, 2-pentynyl, 2-pentynyl or 2-hexynyl, and the like.

C₂ -C₆alkenoxy refers to a straight or branched alkenoxy containing 1 to 3 double bonds and 2-6 carbon atoms, such as vinyloxy, 1-propenyloxy, 1-methyl-1-propenyloxy, 2-methyl-1-propenyloxy, 1-pentenyloxy, 1,3-pentadienyloxy or 2-pentenyloxy.

C₂ -C₆alkynoxy refer to a straight or branched alkynoxy containing 2-6 carbon atoms, e.g., ethynyloxy, 2-propynyloxy, 2-butynyloxy, 3-butynyloxy, 1-methyl-2-propynyloxy, 2-pentynyloxy, or 2-hexynyloxy, and the like.

C₁ -C₆alkanoyl refers to a straight or branched alkanoyl groups containing 1-6 carbon atoms, such as formyl, acetyl, propionyl, butyryl, isobutyryl, pentanoyl, tert-butyryl or hexanoyl.

C₁ -C₆haloalkanoyl refers to a straight or branched alkanoyl containing 1-6 carbon atoms in which the hydrogen atom was substituted by one or more identical or different halogen atoms, e.g. trifluoroacetyl, etc.

C₁-C₆ alkyl-substituted carbamoyl means that the hydrogen atom on the carbamoyl group is substituted by 1 or 2 identical or different C₁-C₆ alkyl, such as -CONHMe, -CONHEt, -CON(Me)Et, -CONEt₂, or -CONMe₂ and the like.

HydroxyC₁-C₆alkoxyC₁-C₆ alkyl substituted carbamoyl means that the hydrogen atom on the carbamoyl is substituted with 1 or 2 identical or different hydroxyC₁-C₆alkoxyC₁-C₆ alkyl, e.g., -CONHCH₂OCH₂OH, -CONHCH₂CH₂ OCH₂CH₂OH, and the like.

HydroxyC₁-C₆alkoxy substituted C₁-C₆alkoxycarbonyl means that an alkyl carbon atom of the C₁-C₆alkoxycarbonyl is attached to the oxygen atom of a hydroxyC₁-C₆alkoxy, such as -COOCH₂OCH₂OH, -COOCH₂CH₂OCH₂CH₂OH, and the like.

Hydroxyl C₁-C₆ alkyl refers to a straight or branched alkyl containing 1-6 carbon atoms with one carbon atom attached to a hydroxyl, such as -CH₂OH, -CH₂CH₂OH, -CH(OH)CH₃, -CH₂CH₂CH₂OH, -CH₂CH₂CH₂CH₂OH, or -CH₂CH(CH₃)CH₂OH.

Amino C₁-C₆ alkyl refers to a straight or branched alkyl containing 1-6 carbon atoms with one carbon atom attached to an amino group, e.g. -CH₂NH₂, -CH₂CH₂NH₂, -CH(NH₂)CH₃, -CH₂CH₂CH₂NH₂ or -CH₂CH₂CH₂CH₂NH₂, etc.

C₁-C₆ alkyl-substituted amino C₁-C₆ alkyl means that the hydrogen atom on the amino group is substituted by 1 or 2 identical or different C₁-C₆ alkyl group, e.g., -CH₂NHMe or -CH₂CH₂NEt₂, etc.

Carbamoyl C₁-C₆ alkyl refers to a straight or branched alkyl containing 1-6 carbon atoms with one carbon atom attached to the carbonyl carbon of the carbamoyl group, e.g., -CH₂CONH₂, -CH₂CH₂CONH₂, -CH(CONH₂)CH₃, or -CH₂CH₂CH₂CONH₂ and the like.

C₁-C₆ alkyl-substituted carbamoyl C₁-C₆ alkyl means that the amino hydrogen atom on the carbamoyl C₁-C₆ alkyl is substituted with 1 or 2 identical or different C₁-C₆ alkyl, such as -CH₂CONHMe, -CH₂CH₂CONHEt, -CH₂CH₂CONMe₂ or -CH₂CONEt₂ and the like.

Cyano C₁-C₆ alkyl refers to a straight or branched alkyl containing 1-6 carbon atoms with one carbon atom attached to a cyano, such as cyanomethyl, 2-cyanoethyl, 1-cyanoethyl, 3-cyanopropyl, 4-cyano-butyl, or 5-cyanopentyl.

Carboxyl C₁-C₆ alkyl refers to a straight or branched alkyl containing 1-6 carbon atoms with one carbon atom attached to a carboxyl, such as carboxymethyl, 2-carboxyethyl, 1-carboxethl 3-carboxypropyl, 4-carboxybutyl, or 5-carboxypentyl, etc., e.g.

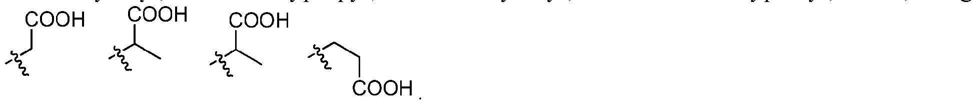

C₁-C₆alkylsulfonyl refers to a straight or branched alkylsulfonyl containing 1-6 carbon atoms, such as methylsulfonyl, ethylsulfonyl or propylsulfonyl.

C₁-C₆haloalkylsulfonyl refers to straight or branched chain alkylsulfonyl containing 1-6 carbon atoms on which the hydrogen atom was substituted by one or more identical or different halogen atoms, e.g., trifluoromethanesulfonyls.

C₁-C₆ alkyl-substituted amino means that the hydrogen atom on the amino is substituted by 1 or 2 identical or different C₁-C₆ alkyl or C₁-C₆alkanoyl, such as -NHMe or -NEt₂, etc.

C₃-C₆Cycloalkyl refers to saturated cyclic hydrocarbyl containing 3-6 carbon atoms, such as cyclopropyl, cyclobutyl, cyclopentyl, and cyclohexyl.

C₃-C₆Cycloalkoxy refers to saturated cyclic hydrocarbyloxy containing 3-6 carbon atoms, such as cyclopropoxy, cyclobutoxy, cyclopentyloxy, cyclohexyloxy, and the like.

C₂-C₁₀ ester group refers to a saturated or unsaturated straight or branched chain ester group containing 2-10 carbon atoms, such as -COO-(C₁-C₉ alkyl), -COO-(C₂-C₈ alkenyl), or -COO-(C₃-C₉cycloalkyl), or -O-CO-(C₁-C₉ alkyl), -O-CO-(C₂-C₈ alkenyl), or -O-CO-(C₃-C₉cycloalkyl), and the like, wherein the alkyl, alkenyl or cycloalkyl may be substituted or unsubstituted (e.g., halogenated).

### Cyclic bisbenzyl isoquinoline compound

Cyclic bisbenzyl isoquinoline alkaloid is a very important class of natural product with a wide range of biological activities, and representative substances include oxyacanthine, tetrandrine, cepharanthine and fangchinoline, etc..

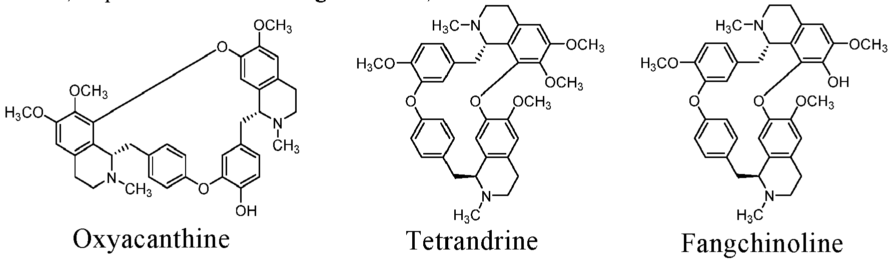

Oxyacanthine is a white crystal, bitter powder with a melting point of 216-217 °C, and optical rotation of +131.5° (chloroform). Oxyacanthine is almost insoluble in water, soluble in ethanol, chloroform, ether, dilute acid. It is found in the roots of *Berberis vulgaris* of the Berberidaceae family, and the roots of *Thalictrumlucidum L.* of the Ranunculaceae family. Oxyacanthine has antibacterial, antihypertensive, choleretic and antileukemic effects.

### The compound of the present invention:

The compound of the present invention is the cyclic bisbenzyl isoquinoline compound of Formula I, or a pharmaceutically acceptable salt, an enantiomer, a diastereoisomer, a racemate, or a crystalline hydrate, a solvate thereof, or mixtures thereof, wherein the cyclic bisbenzyl isoquinoline compound is the compound shown in Formula I: 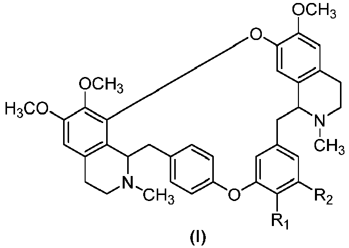 whereinR₁ and R₂ are as defined above.

Preferably, the compound of formula I of the present invention is non-natural compound.

In the present invention, the inventors unexpectedly found that when the compound did not contain phenolic hydroxyl, their inhibitory activity against the coronavirus SARS-CoV-2 was significantly increased, with an EC₅₀ value about 10-timeslower than that of oxyacanthine hydrochloride.

Preferably, the compound of the present invention is selected from Table A:

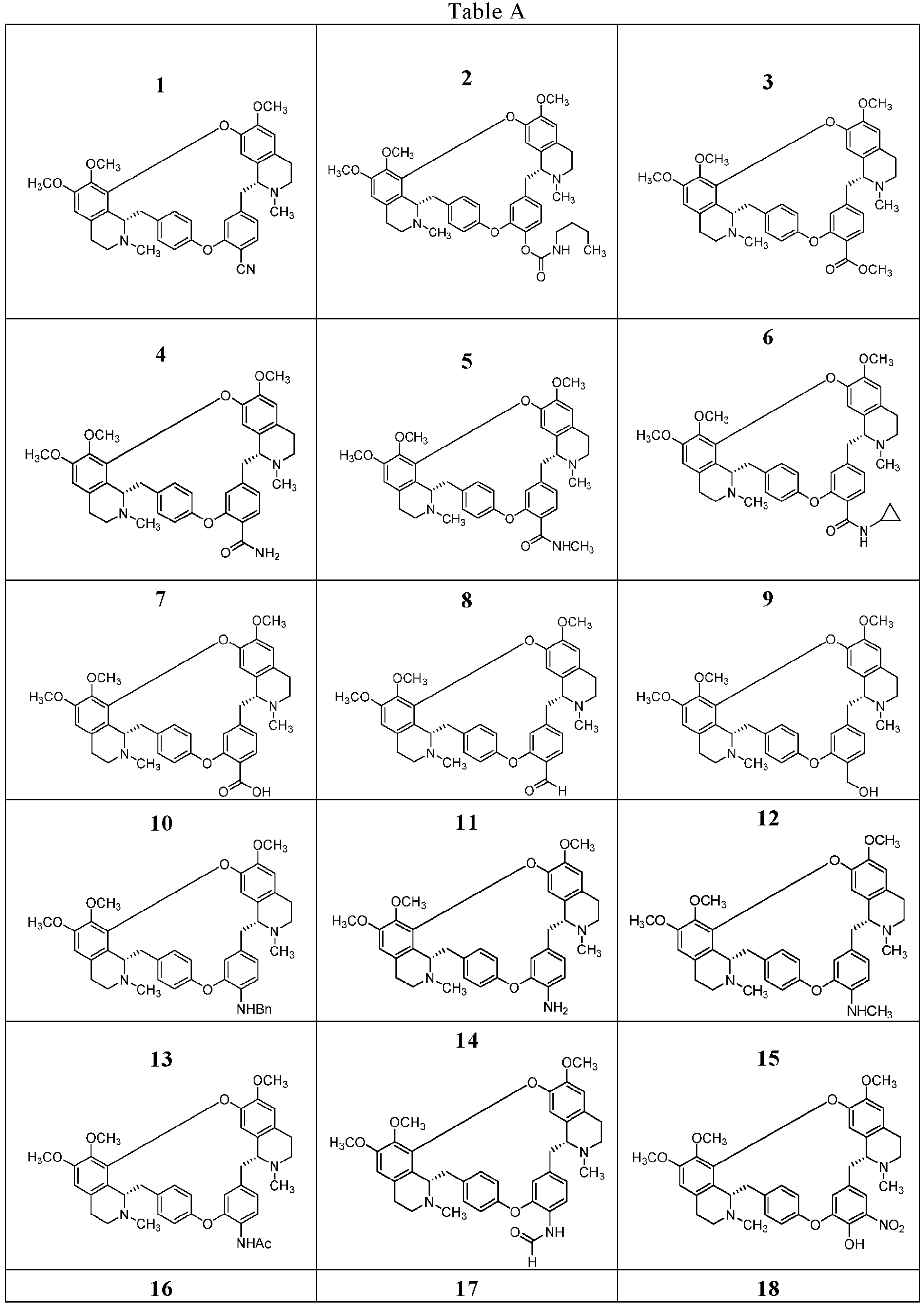

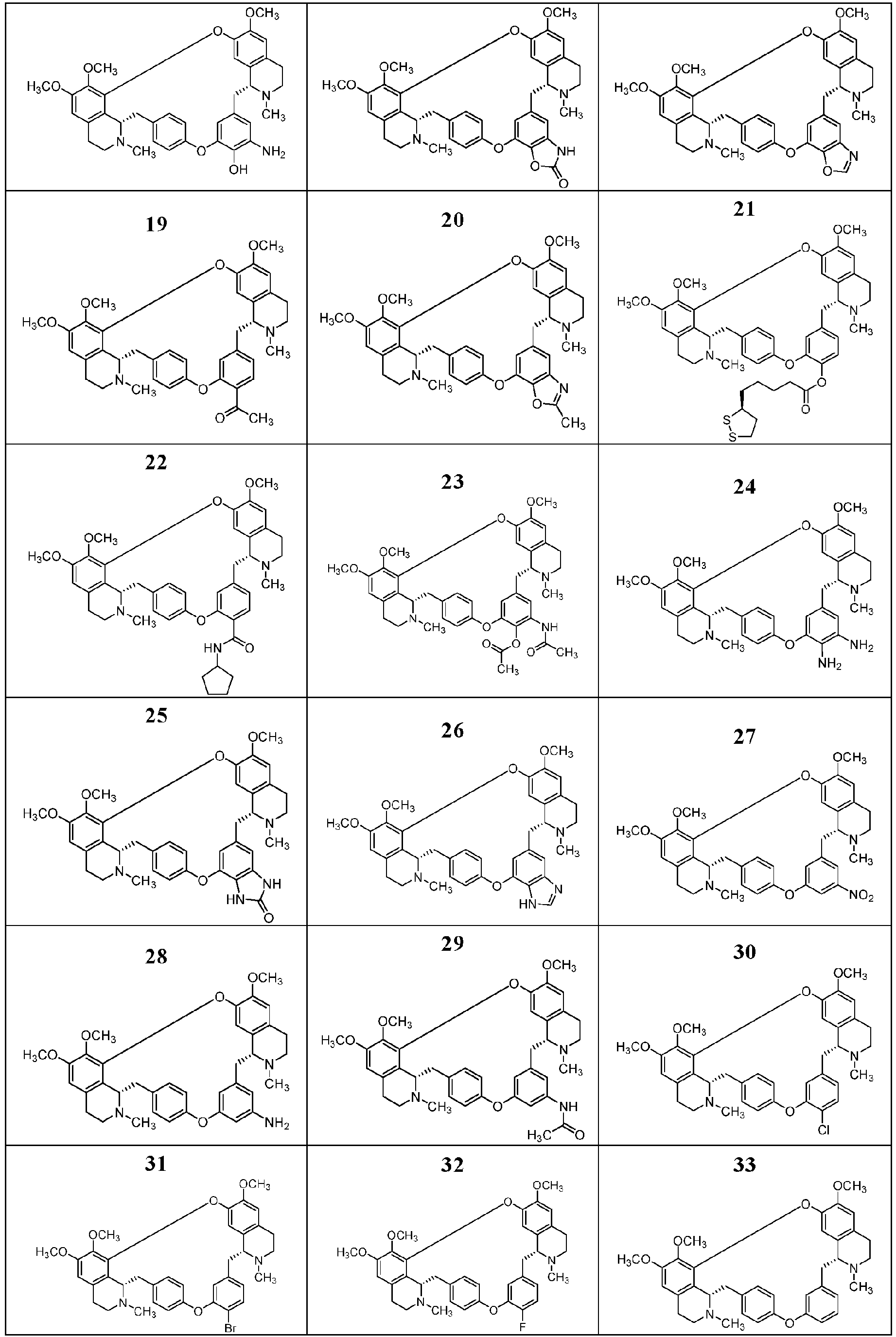

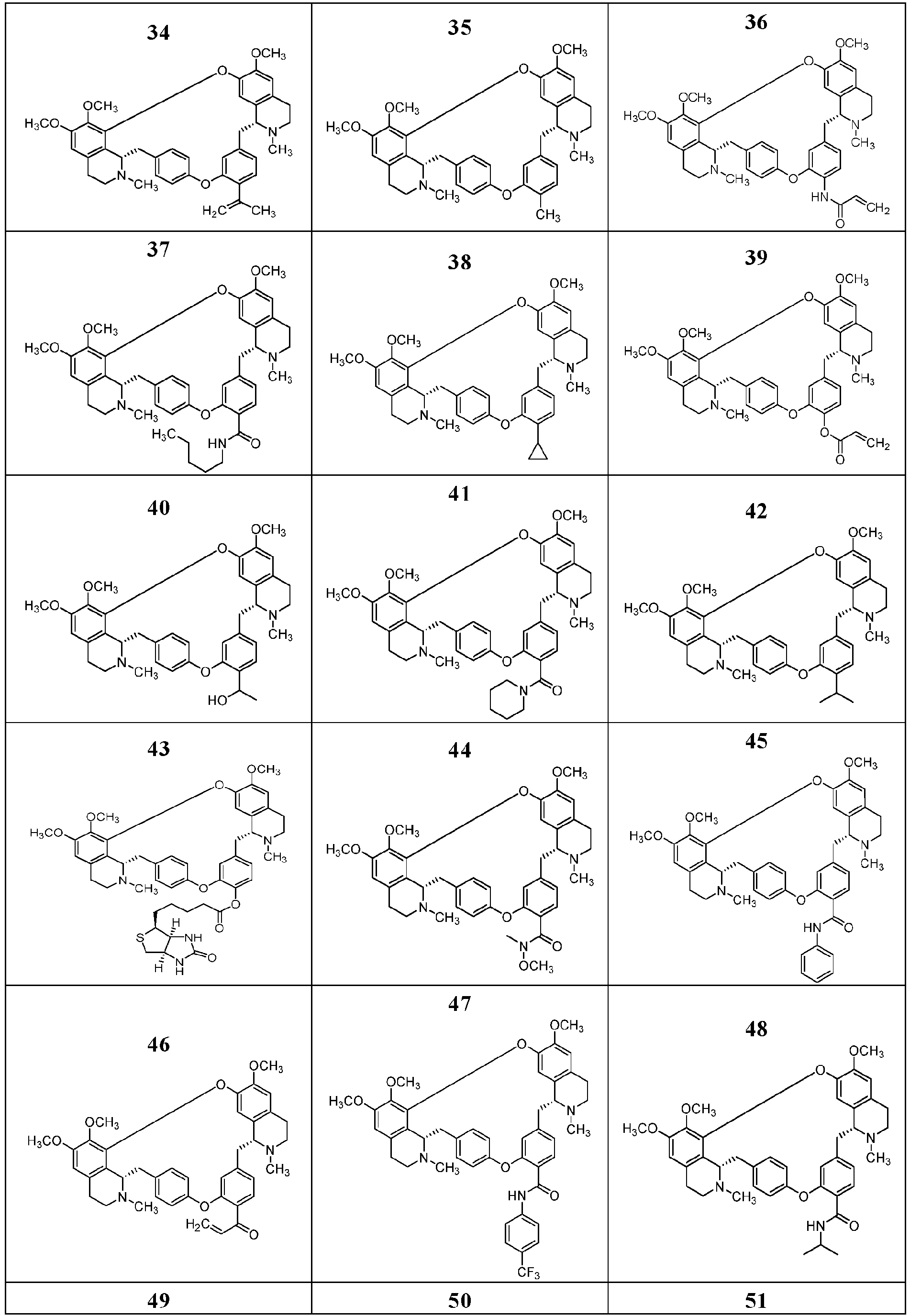

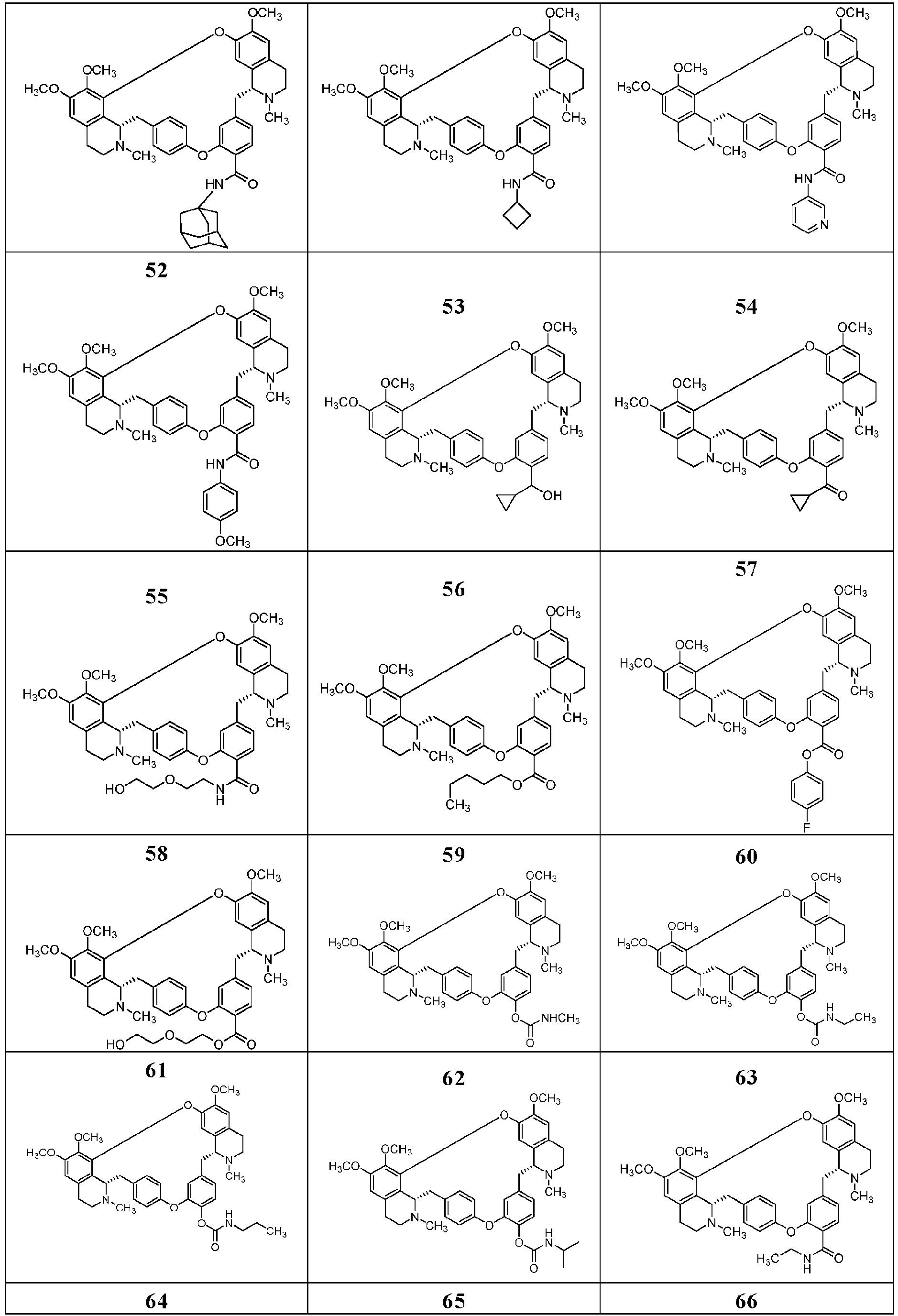

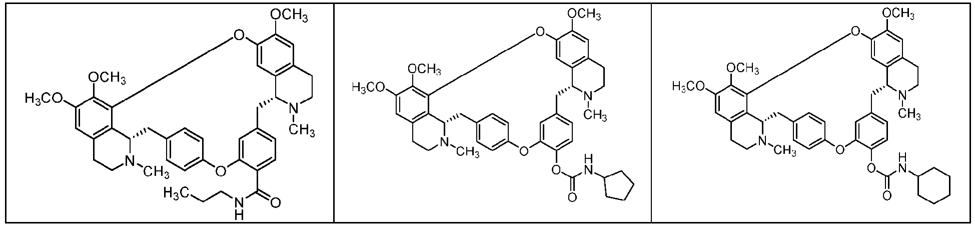

Preferably, the compound of the present invention is selected from Table A-I:

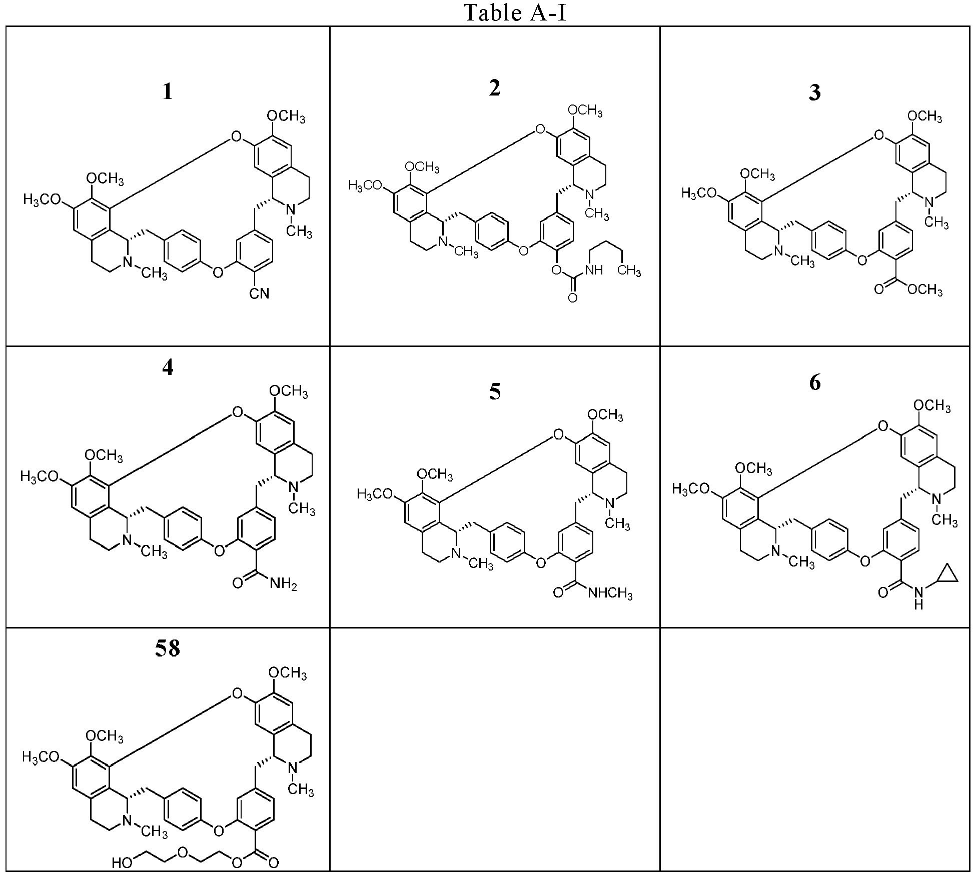

The "pharmaceutically acceptable salt" is a conventional non-toxic salt formed by reacting the active compound of the invention with an inorganic or organic acid. For example, conventional non-toxic salts can be produced by reacting the active compounds of the present invention with inorganic or organic acids, the inorganic acid includes hydrochloric acid, hydrobromic acid, sulfuric acid, nitric acid, sulfamicacid and phosphoric acid, etc., and the organic acid includes citric acid, tartaric acid, lactic acid, pyruvic acid, acetic acid, benzenesulfonic acid, p-toluenesulfonic acid, methanesulfonic acid, naphthalene sulfonate, ethanesulfonic acid, naphthalene disulfonate, maleic acid, malic acid, propanedioic acid, fumaric acid, succinic acid, propionic acid, oxalic acid, trifluoroacetic acid, stearic acid, pamoic acid, hydroxymaleic acid, phenylacetic acid, benzoic acid, salicylic acid, glutamic acid, ascorbic acid, p-aminobenzenesulfonic acid, 2-acetoxybenzoic acid, and hydroxyethanesulfonic acid, etc.; or the sodium, zinc, potassium, calcium, aluminum or ammonium salts formed by an inorganic base with an ester formed by the active compound of the present invention and propionic acid, oxalic acid, propanedioic acid, succinic acid, fumaric acid, maleic acid, lactic acid, malic acid, tartaric acid, citric acid, aspartic acid or glutamic acid; or the corresponding inorganic acid salts formed byhydrochloric, hydrobromic acid, hydrofluoric acid, sulfuric acid, nitric acid or phosphoric acid with the esters formed by the active compound of the present invention with lysine, arginine, or ornithine, or the corresponding organic acid salts formed by formic, acetic, picric, methanesulfonic, or ethanesulfonic acid with the esters formed by the active compound of the present invention with lysine, arginine, or ornithine; or the sodium salt, zinc salt, potassium salt, calcium salt, aluminum salt or ammonium salt formed by the carboxyl/phenolic hydroxyl group in the molecule of the active compound with an inorganic base.

In addition, the active ingredients of the present invention are particularly suitable for use in combination with other anti-coronaviral agents. Representative other anti-coronaviral agents include (but are not limited to): interferons, RNA-dependent RNA polymerase inhibitors (e.g., Remdesivir (Remdesivir or GS-5734), favipiravir, Galidesivir, GS-441524, Molnupiavir (Monupiravir or EIDD-2801), NHC (EIDD-1931); 3CL protease inhibitors (nirmatrelvir, ensitrelvir, GC-376), Lopinavir, Ritonavir, Nelfinavir; Chloroquine, Hydroxychloroquine, Cyclosporine, Carrimycin, Baicalin, Baicalein, Naphthoquine, Ciclesonide, Ribavirin, Penciclovir, Leflunomide, Teriflunomide, nafamostat, nitazoxanide, Darunavir, Arbidol, Camostat, Niclosamide, Ivermectin, Baricitinib, Ruxolitinib, Dasatinib, Saquinavir, Beclabuvir, Simeprevir, or a pharmaceutically acceptable salt thereof, or a combination thereof. The interferon comprises one or more of interferon α-2a, interferon α-2b, interferon α-n1, interferon α-n3, interferon β-1a, interferon β-1b.

In addition, since SARS-CoV-2 infection can cause acute lung injury, inflammatory responses and even cytokine storms, the active ingredients of the present invention are also particularly suitable for use in combination with drugs having an effect of improving acute lung injury, anti-inflammation, or immunomodulation. Representative drugs include, but are not limited to, Zinc, Fingolimod, Vitamin C, OlmesartanMedoxomil, valsartan, Losartan, Thalidomide, glycyrrhizic acid, Artemisinin, dihydroartemisinin, Artesunate, Artemisone, Azithromycin, Escin, Naproxen.

Preferably, the active ingredient of the present invention is used in combination with an artemisinin-based drug (one or more of artemisinin, dihydroartemisinin, artesunate, artemisinone). Numerous studies have shown that artemisinin-based drugs have multiple anti-inflammatory and immunomodulatory mechanisms, achieving anti-inflammatory and immunomodulatory functions by inhibiting T-cell proliferation and activation, inhibiting B-cell activation and antibody production, increasing regulatory T-cells as well as decreasing the release of inflammatory cytokines, and are expected to alleviate the symptoms of immune injury caused by novel coronavirus (SARS-CoV-2) infection.

Preferably, the active ingredient of the present invention is used in combination with an artemisinin-based drug (one or more of artemisinin, dihydroartemisinin, artesunate, artemisinone),and azithromycin.

The active ingredient of the present invention inhibits the infection activity of novel coronaviruses such as SARS-CoV-2. Thus, when the active ingredient of the present invention is therapeutically administered or given, the infection of 2019 novel coronavirus (SARS-CoV-2) can be inhibited, thereby achieving an antiviral effect.

### Preparation method

Method for preparing the cyclic bisbenzyl isoquinoline compound of the present invention is selected from one or more of the following methods:
a) Cyclic bisbenzyl isoquinoline containing phenolic hydroxyl group is taken as raw material, and subjected to alkylation reaction with alkylation reagent to obtain the cyclic bisbenzyl isoquinoline compound;
b) Cyclic bisbenzyl isoquinoline containing phenolic hydroxyl group is taken as raw material, and subjected to acylation reaction with acylation reagent to obtain the cyclic bisbenzyl isoquinoline compound;

In another preferred embodiment, the method is as follows: oxyacanthine is taken as raw material, and undergoes condensation acylation reaction with carboxylic acid to obtain the cyclic bisbenzyl isoquinoline compound.

In another preferred embodiment, the condensation acylation reaction is carried out in the presence of a condensation agent, and the condensation agent includes (but is not limited to) N,N'-dicyclohexylcarbodiimide (DCC), 1 -ethyl-3-(3-dimethylaminopropyl)carbodiimide (EDCI), O-benzotriazole- N,N,N',N'-tetramethyluroniumtetrafluoroborate (TBTU), and the like.
c) Oxyacanthine is taken as raw material, and reacts with sulfonylation reagent in the presence of a base to obtain compound (I-1b);
Compound (I-1b) and a coupling reagent undergo coupling reaction to obtain the cyclic bisbenzyl isoquinoline compounds of formula I-1, the reaction is as shown in reaction formula 1:

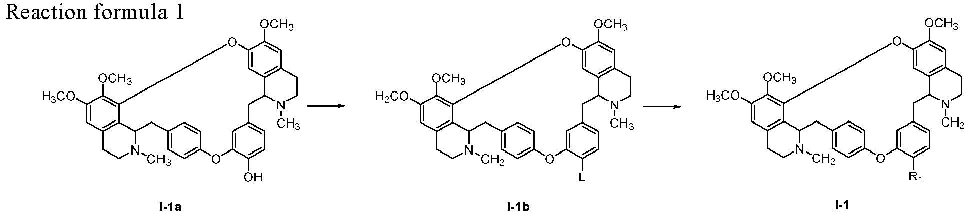

In Formula I-1, R₁ is selected from hydrogen, halogen, C₁-C₆alkylthiol, C₁-C₆ alkyl, C₃-C₆cycloalkyl, C₂-C₆ alkenyl, C₂-C₆alkynyl, C₁-C₆ alkyl-substituted amino, cyano, carboxyl, aldehyde and C₁-C₆alkanoyl;
L in formula 1-1b is selected from the leaving group such as C₁-C₆alkylsulfonyloxy, C₁-C₆haloalkylsulfonyloxy, phenylsulfonyloxy, naphthylsulfonyloxy, preferably, methylsulfonyloxy, trifluoromethanesulfonyloxy.

d) Oxyacanthine is taken as raw material, and subjected to nitration, reduction, and ring-closing reaction to obtain a compound of formula (I-2), as shown in the following reaction formula 2:

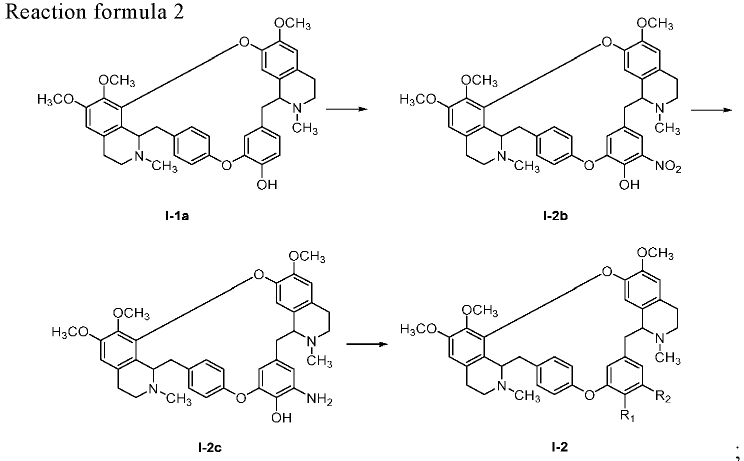

In Formula 1-2, R₁, and R₂ together with the adjacent benzene ring form a benzo[5- to 6-membered monocyclic heterocycle] which is unsubstituted or substituted with 1 to 4 substituents;

In another preferred embodiment, the benzo5 to 6-membered monocyclic heterocycle] is selected from:

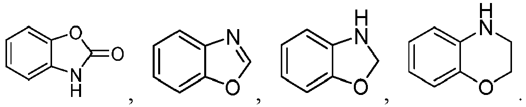

In another preferred embodiment, the substituent for substitution is selected from halogen, hydroxyl, thiol, amino, oxo (=O), thio (=S), and C₁-C₆ alkyl; the heterocycle contains from 1 to 3 heteroatoms selected from N, O and S;
e) Oxyacanthine is taken as raw material, and undergoes multi-steps of reaction to obtain the compound of formula (I-3), as shown in reaction formula 3:

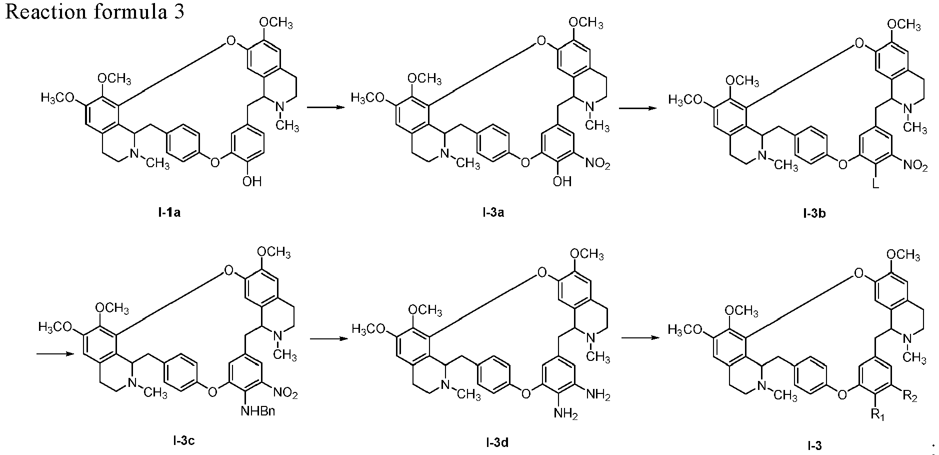

In Formula I-3, R₁ and R₂ together with the adjacent benzene ring form a benzo[5- to 6-membered monocyclic heterocycle] which is unsubstituted or substituted with 1 to 4 substituents;
the substituent for substitution is selected from halogen, hydroxyl, thiol, amino, oxo (=O), thio (=S), and C₁-C₆ alkyl;
the heterocycle contains from 1 to 3 heteroatoms selected from N, O and S.

In another preferred embodiment, the benzo5 to 6-membered monocyclic heterocycle] is selected from:

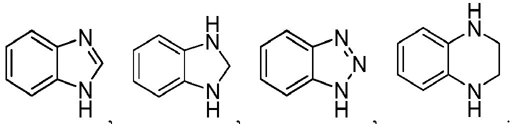

In formula I-3b, L is a leaving group, such as C₁-C₆alkylsulfonyloxy, C₁-C₆haloalkylsulfonyloxy, phenylsulfonyloxy, naphthylsulfonyloxy, preferably, methylsulfonyloxy, and trifluoromethanesulfonyloxy.

In another preferred embodiment, the method e) includes the following steps:
e1) Compound of formula (I-1a) and a nitration reagent undergo an ortho-nitration reaction to obtain a compound of formula (I-3a);
e2) Compound of formula (I-3a) reacts with sulfonylation reagent in the presence of a base in a suitable solvent to obtain a compound of formula (I-3b);
e3) Compound of formula (I-3b) and benzylamine undergo a Buchwald-Hartig reaction to obtain a compound of formula (I-3c);
e4) Compound of formula (I-3c) undergoes a reduction reaction to obtain a compound of formula (I-3d);
e5) compound of formula (I-3d) undergoes a ring-closing reaction to obtain a compound of formula (I-3).
f) The compounds obtained from methods a) to e) are subjected to a functional group conversion to obtain the cyclic bisbenzyl isoquinoline compound.

### Pharmaceutical compositions and applications

The present invention also provides a use of the active compound of Formula I of the present invention for inhibiting replication of coronaviruses, or a pharmaceutically acceptable salt thereof, or a prodrugs thereof, or a mixture of one or more thereof, as an active ingredient, in the preparation of a medicament for treating and/or preventing, or alleviating respiratory infections, pneumonia, and other related diseases caused by coronavirus infections such as the novel coronavirus of 2019.

The pharmaceutical compositions provided by the present invention preferably contain from 0.001 to 99 wt% by weight of the active ingredient, preferably in a ratio of 0.1 wt% to 90 wt%, or 1 wt% to 50 wt% by weight of the active compound of the invention as the active ingredient, with the rest being a pharmaceutically acceptable carrier, dilution, or solution, or salt solution.

When desired, one or more pharmaceutically acceptable carriers may also be added to the drug of the invention. The carriers include pharmaceutically conventional diluents, excipients, fillers, binders, wetting agents, disintegrants, absorption enhancers, surfactants, adsorbent carriers, lubricants and the like.

The compounds and pharmaceutical compositions provided herein may be in a variety of forms, such as tablets, capsules, powders, syrups, solution forms, suspensions, and aerosols, and may be present in suitable solid or liquid carriers or dilutions or in suitable sterilized instruments for injection or drip.

Various dosage forms of the pharmaceutical compositions of the present invention may be prepared according to conventional methods of preparation in the field of pharmacy. The dosage formulations thereof typically contain 0.05-400 mg of the active compound of the present invention in a unit measurement, preferably, the formulations contain 1 mg-500 mg of the active compound of the present invention in a unit measurement.

The compounds and pharmaceutical compositions of the present invention can be used clinically in mammals, including human and animal, and can be administered by routes of administration such as the mouth, nose, skin, lung or gastrointestinal tract, etc.; most preferably, by mouth. The most preferred daily dosage is 0.01-400 mg/kg body weight in a single dose, or 0.01-200 mg/kg body weight in divided doses. Regardless of the method of administration, the optimal dosage for an individual should be based on the specific treatment. It is common to start with a small dose and gradually increase until the most appropriate dose is found.

The drugs or inhibitors of the present invention may be administered in a variety of different ways, for example, they may be introduced into the body such as intramuscularly, intradermally, subcutaneously, intravenously, through mucosal tissues by injection, jetting, nasal, ophthalmic, osmotic, absorptive, physically or chemically mediated methods; or they may be introduced into the body by mixing with or encapsulated by other substances.

Typically, the active ingredient of the present invention or pharmaceutical compositions comprising the same may be administered in unit dose form by intestinal or non-intestinal routes such as oral, intravenous, intramuscular, subcutaneous, nasal, oral mucosa, ocular, pulmonary and respiratory, dermal, vaginal, rectal, and the like.

The dosage form may be liquid dosage form, solid dosage form or semi-solid dosage form. Liquid dosage forms can be solutions (including true solutions and colloidal solutions), emulsions (including o/w-type, w/o-type and multiple emulsions), suspensions, injections (including injection workshop, powder injections and infusions), eye drops, nose drops, lotions and liniments, etc.; solid dosage forms can be tablets (including ordinary tablets, enteric-coated tablets, lozenges, dispersible tablets, chewing tablets, effervescent tablets and oral disintegration tablets), capsules (including hard capsules, soft capsules, enteric-coated capsules), granules, powders, micro-pills, dropping pills, suppositories, films, patches, aerosol, spray, etc.; and semi-solid dosage forms can be ointments, gels, pastes, etc..

The active ingredients of the present invention can be made into generic formulations, as well as into sustained-release formulations, controlled-release formulations, targeted formulations, and various particulate delivery systems.

In order to make the active ingredient of the present invention into tablets, a wide range of various excipients known in the art can be used, including diluents, binders, wetting agents, disintegrants, lubricants, and glidants. The diluent may be starch, dextrin, sucrose, glucose, lactose, mannitol, sorbitol, xylitol, microcrystalline cellulose, calcium sulfate, calcium hydrogen phosphate, calcium carbonate, etc.; the wetting agent may be water, ethanol, isopropanol, etc.; the binder may be starch paste, dextrin, molasses, honey, dextrose solution, microcrystalline cellulose, gum Arabic slurry, gelatin slurry, sodium carboxymethylcellulose, methyl cellulose, hydroxypropyl methyl cellulose, ethyl cellulose, acrylic resin, carbomer, polyvinylpyrrolidone, polyethylene glycol, etc.; the disintegrant can be dry starch, microcrystalline cellulose, low-substituted hydroxypropyl cellulose, crosslinked polyvinylpyrrolidone, crosslinked carboxymethylcellulose sodium, carboxymethyl starch sodium, sodium bicarbonate and citric acid, polyoxyethylene sorbitol fatty acid ester, sodium dodecylsulphonate, etc.; lubricantsand glidants can be Talc, silicon dioxide, stearate, tartaric acid, liquid paraffin, polyethylene glycol, etc..

The tablets may further be made into coated tablets, such as sugar-coated tablets, film-coated tablets, enteric-coated tablets, or bilayer and multilayer tablets.

In order to make the delivery unit into a capsule, the active ingredient of the present invention can be mixed with diluent, and glidant, and the mixture can be placed directly into a hard or soft capsule. The active ingredient may also be made into granules or micropills with diluent, binder and disintegrant before being placed into a hard or soft capsule. Each of the diluent, binder, wetting agent, disintegrant, and glidant used in the preparation of tablets of the present invention may also be used in the preparation of capsules of the present invention.

In order to make the active ingredient of the present invention into an injection, water, ethanol, isopropanol, propylene glycol or mixtures thereof can be used as a solvent and appropriate amounts of solubilizers, co-solvents, pH modifiers and osmolality regulators commonly used in the field can be added. Solubilizers or co-solvents can be poloxamer, lecithin, hydroxypropyl-β-cyclodextrin, etc.; pH regulators can be phosphate, acetate, hydrochloric acid, sodium hydroxide, etc.; osmolality regulators can be sodium chloride, mannitol, glucose, phosphate, acetate, etc.. To prepare lyophilized powder injections, mannitolglucose, etc. can also be added as a proppant.

In addition, colorants, preservatives, spices, flavoring agents, or other additives may be added to the pharmaceutical formulation, if desired.

The active ingredients or compositions of the present invention may be taken alone or in combination with other therapeutic or symptomatic drugs.

When the active ingredient of the present invention has a synergistic effect with other therapeutic agents, its dosage should be adjusted accordingly.

### The main advantages of the present invention include:

1) The compounds of the present invention have good inhibitory activity against coronavirus replication; EC₅₀ of the inhibitory effect of some of the compounds on novel coronavirus RNA replication even reaches <1 µM.
2) The compounds of the present invention do not contain phenolic hydroxyl group, and have a significantly improved anti-coronavirus SARS-CoV-2 activity, as well as good physicochemical property, good metabolic property, and high oral bioavailability.
3) The compounds of the present invention have good selectivity for hERG ion channels and Nav1.5 ion channels and are expected to have low toxicity to the heart.
4) The compounds of the present invention are characterized by high activity, a large therapeutic index SI, oral effectiveness, low potency dose and low toxic side effects, and can be used for the prevention and/or treatment of diseases related to coronavirus infections, and have a good prospect for clinical application.
5) The compounds of the present invention simultaneously have therapeutic effects against diseases associated with viral infection, inflammation-related diseases (pneumonia, non-alcoholic steatohepatitis, colitis, nephritis, pancreatitis, myocarditis, arthritis, inflammatory pain), fibrosis-related diseases (pulmonary fibrosis, silicosis, hepatic fibrosis, renal fibrosis, myocardial fibrosis, dermatofibrosis, retinal fibrosis, myelofibrosis)), Th1 and/or Th17 abnormal differentiation-related autoimmune diseases (psoriasis, rheumatoid arthritis, systemic lupus erythematosus, multiple sclerosis, neuromyelitisoptica, myasthenia gravis, ankylosing spondylitis, ulcerative colitis, Crohn's disease, delayed-type hypersensitivity, graft-versus-host disease, etc.), osteoporosis, and neurodegenerative diseases.

The present invention is further described below in conjunction with specific embodiments. It is to be understood that these examples are intended to illustrate the invention only and not to limit the scope of the invention. The experimental methods that do not indicate specific conditions in the following examples usually follow the conventional conditions, such as those described in Sambrook et al., Molecular Cloning: A Laboratory Manual (New York: Cold Spring Harbor Laboratory Press, 1989), or the conditions suggested by the manufacturer. Unless otherwise specified, percentages and parts are calculated by weight.

### Example 1 Preparation of compound 1

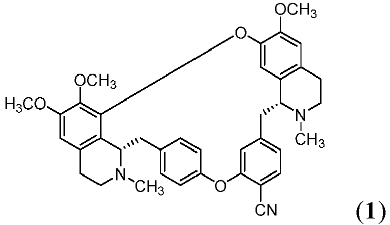

The steps are shown below:

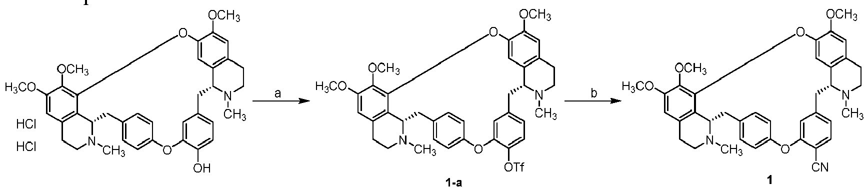

### Step 1:

To a 50 mL round-bottomed single-necked flask was added **oxyacanthine hydrochloride** (1.2 g, 1.76 mmol), dichloromethane (12 mL) and triethylamine (0.89 g, 8.80 mmol, 5 eq), and stirred for 10 min under an ice bath. Trifluoromethanesulfonicanhydride (0.58 mL, 3.45 mmol, 1.96 eq) was added dropwise slowly, after addition, the mixture was stirred for 2 h at room temperature. TLC showed complete conversion of raw material. The resulting solution was then added with 1 N hydrochloric acid (5.3 mL), and stirred; the organic layer was separated and washed once with saturated aqueous sodium bicarbonate, dried over anhydrous sodium sulfate, concentrated, and subjected to silica gel column chromatography to obtain a pale yellow solid **1-a** (1.08 g, 1.46 mmol) with a yield of83%. ¹H NMR (600 MHz, Acetone-*d₆*) δ 7.50 (dd, *J* = 8.5, 2.3 Hz, 1H), 7.22 (d, *J* = 8.4 Hz, 1H), 7.06 (dd, *J* = 8.2, 2.3 Hz, 1H), 7.00 (dd, *J* = 8.4, 2.6 Hz, 1H), 6.94 (dd, *J* = 8.3, 2.0 Hz, 1H), 6.71 (s, 1H), 6.49 (s, 1H), 6.46 (s, 1H), 6.33 (dd, *J* = 8.2, 2.7 Hz, 1H), 5.66 (d, *J* = 2.0 Hz, 1H), 4.09 (d, *J* = 5.4 Hz, 1H), 3.76 (s, 3H), 3.68 (s, 3H), 3.62 (d, *J* = 3.5 Hz, 1H), 3.31 (d, *J* = 14.5 Hz, 1H), 3.24 (dd, *J* = 14.0, 4.1 Hz, 1H), 3.16 (s, 3H), 3.15 - 3.10 (m, 1H), 3.01 - 2.88 (m, 2H), 2.86 - 2.79 (m, 2H), 2.77 - 2.71 (m, 1H), 2.63 (dd, *J* = 5.2, 1.8 Hz, 1H), 2.60 (s, 3H), 2.56 (s, 3H), 2.37 - 2.20 (m, 3H).ESI-MS (*m*/*z*): 741.38 [M + H]⁺.

### Step 2:

To a mixture of **1-a** (850 mg, 1.15 mmol) and N, N-dimethylacetamide (10 mL) was added zinc cyanide (200 mg, 1.70 mmol, 1.48 eq), tris(dibenzylideneacetone)dipalladium (55 mg, 0.06 mmol, 0.05 eq), 1,1'-bis(diphenylphosphino)ferrocene (65 mg, 0.12 mmol, 0.1 eq), and zinc powder (12 mg, 0.18 mmol, 0.16 eq) in turn, and replaced four times with nitrogen, then heated to 140-150 °C and reacted for 2 h. TLC showed complete conversion of raw material. After cooling to 30-40 °C, the reaction solution was diluted with ethyl acetate, and the insoluble material was filtered off, the filtrate was washed three times with 5% aqueous sodium bicarbonate. The ethyl acetate layer was separated, dried over anhydrous sodium sulfate, filtered, concentrated, and subjected to silica gel column chromatography to obtain the title Compound **1** (583 mg, 0.94 mmol), as a pale yellow solid with a yield of 82%. ¹H NMR (600 MHz, Acetone-*d₆*) δ 7.51 - 7.45 (m, 2H), 7.07 (dd, J = 8.2, 2.3 Hz, 1H), 7.01 (dd, J = 8.4, 2.6 Hz, 1H), 6.98 (dd, J = 8.0, 1.4 Hz, 1H), 6.70 (s, 1H), 6.48 (s, 1H), 6.45 (s, 1H), 6.34 (dd, J = 8.2, 2.7 Hz, 1H), 5.60 (d, J = 1.4 Hz, 1H), 4.09 (d, J = 5.4 Hz, 1H), 3.76 (s, 3H), 3.69 (s, 3H), 3.67 (m, 1H), 3.35 - 3.25 (m, 2H), 3.15 (s, 3H), 3.13 - 3.07 (m, 1H), 3.02 - 2.88 (m, 2H), 2.89 - 2.78 (m, 2H), 2.73 (m, 1H), 2.63 (m, 1H), 2.60 (s, 3H), 2.56 (s, 3H), 2.38 - 2.19 (m, 3H). ESI-MS (*m*/*z*): 618.37 [M + H]⁺.

### Example 2 Preparation of compound 2

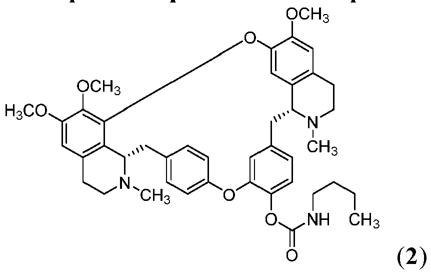

To a suspension of 500 mg of **oxyacanthine hydrochloride** in acetonitrile (12 mL) was added triethylamine (260 mg) at room temperature, and then the reaction solution was added slowly dropwise with 114 mg of n-butyl isocyanate, and stirred for 2-3h at 25-28°C. The resulting solution was then diluted with ethyl acetate, and washed three times with water, the organic layer was separated, dried over anhydrous sodium sulfate, filtered, concentrated, and subjected to silica gel column chromatography to obtain 385 mg of the title compound 2**,** as a pale yellow solid with a yield of 74%. ¹H NMR (600 MHz, Acetone-*d₆*) δ 7.43 (dd, J = 8.4, 2.4 Hz, 1H), 6.96 (dd, J = 8.2, 2.3 Hz, 1H), 6.92 (dd, J = 8.3, 2.6 Hz, 1H), 6.88 (d, J = 8.1 Hz, 1H), 6.79 (dd, J = 8.2, 2.0 Hz, 1H), 6.68 (s, 1H), 6.47 (s, 1H), 6.44 (s, 1H), 6.26 (dd, J = 8.2, 2.6 Hz, 1H), 5.55 (d, J = 2.1 Hz, 1H), 4.08 (d, J = 5.0 Hz, 1H), 3.76 (s, 3H), 3.67 (s, 3H), 3.57 (m, 1H), 3.30 - 3.10 (m, 5H), 3.16 (s, 3H), 2.96 (m, 1H), 2.89 - 2.60 (m, 5H), 2.59 (s, 3H), 2.55 (s, 3H), 2.38 - 2.28 (m, 3H), 1.51 (p, J = 7.1 Hz, 2H), 1.36 (h, J = 7.4 Hz, 2H), 0.87 (t, J = 7.3 Hz, 3H). ESI-MS (*m*/*z*): 708.49 [M + H]⁺.

### Example 3 Preparation of compound 3

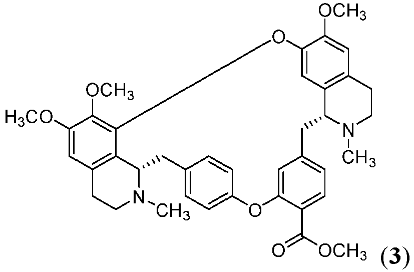

The steps are shown below:

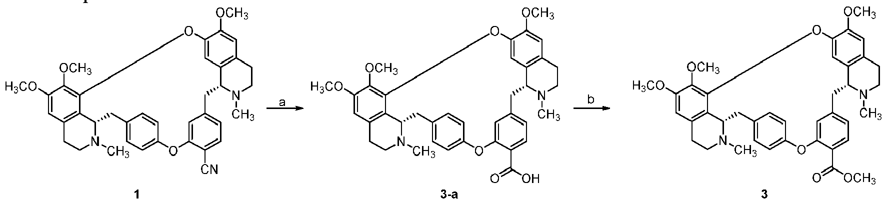

### Step 1:

To a mixture of compound **1** (1.1 g, 1.78 mmol) and ethylene glycol (11 mL) was added potassium hydroxide (600 mg, 10.70 mmol, 6 eq) and water (0.5 mL), and the reaction was replaced with nitrogen for several times, and heated to 150 °C for 7 h. The reaction solution was then cooled down to room temperature, diluted with water (25 mL), and adjusted to pH7-8 with dilute hydrochloric acid, and extracted with dichloromethane; the organic phase was separated, washed once with water, washed once with saline, dried over anhydrous sodium sulfate, filtered, concentrated, and subjected to a silica gel column chromatography to obtain compound **3-a** (300 mg), as a pale yellow solid with a yield of 26%. ¹H NMR (600 MHz, Chloroform-*d*) δ 7.67 (d, 1H), 7.48 (d, 1H), 6.92 (m, 1H), 6.80 (m, 1H), 6.58 (s, 1H), 6.55 (m, 1H), 6.36 (s, 1H), 6.20 (s, 1H), 6.06 (d, 1H), 5.51 (s, 1H), 4.45 (d, 1H), 3.98 (m, 1H), 3.78 (s, 3H), 3.59-3.39 (m, 2H), 3.50 (s, 3H), 3.36-3.20 (m, 2H), 3.17 (s, 3H), 3.14-3.00 (m, 2H), 2.90-2.62 (m, 4H), 2.75 (s, 3H), 2.57 (s, 3H), 2.43-2.25 (m, 2H). ESI-MS (*m*/*z*): 637.36 [M + H]⁺, 635.37 [M - H]⁻.

### Step 2:

To a mixture of compound **3-a** (600 mg, 0.94 mmol) and methanol (6 mL) was added concentrated sulfuric acid (352 mg, 3.59 mmol, 3.8 eq) at room temperature, after addition, the temperature was raised to reflux and the reaction was continued for 7 h. TLC showed complete conversion of raw material. The resulting solution was added with sodium bicarbonate (938 mg, 11.8 eq) and stirred for 10 min, diluted with dichloromethane, added with water and stirred to partition. The organic phase was separated, washed with saline, dried over anhydrous sodium sulfate, filtered, concentrated, and subjected to silica gel column chromatography to obtain the title compound **3** (486 mg), as an off-white solid with a yield of 80%. ¹H NMR (600 MHz, Chloroform-*d*) δ 7.70 (d, 1H), 7.45 (brs, 1H), 6.95 (m, 2H), 6.89 (d, 1H), 6.60 (s, 1H), 6.35 (s, 1H), 6.30 (dd, 1H), 6.30 (s, 1H), 5.57 (m, 1H), 4.22 (brs, 1H), 3.85 (s, 3H), 3.78 (s, 3H), 3.70 (m, 1H), 3.61 (s, 3H), 3.47-3.20 (m, 3H), 3.17 (s, 3H), 3.10-2.95 (m, 2H), 2.88-2.65 (m, 4H), 2.68 (s, 3H), 2.57 (s, 3H), 2.46-2.22 (m, 3H). ESI-MS *(m*/*z):* 651.47 [M + H]⁺.

### Example 4 Preparation of compound 4

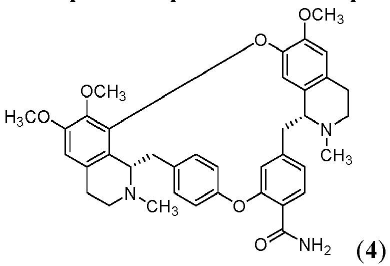

To a suspension of compound **1** (1.1 g, 1.78 mmol) in tert-butanol (8 mL) was added potassium tert-butoxide (1.4 g, 12.48 mmol, 7 eq), then heated to 80 °C and reacted until the raw material conversion was complete. The reaction solution was then cooled down to room temperature, and concentrated to remove most of the tert-butanol; to the residual solution was added water and ethyl acetate, and stirred to partition. The ethyl acetate layer was separated, washed once with saline, dried over anhydrous sodium sulfate, filtered, concentrated and subjected to silica gel column chromatography to obtain the title compound **4** (820 mg), as a pale yellow solid with a yield of 73%. ¹H NMR (600 MHz, Acetone-*d₆*) δ 7.90 (d, J = 8.0 Hz, 1H), 7.57 (brs, 1H), 7.49 (dd, J = 8.4, 2.3 Hz, 1H), 7.03 (td, J = 8.0, 2.4 Hz, 2H), 6.96 (dd, J = 8.0, 1.6 Hz, 1H), 6.69 (m, 1H), 6.68 (s, 1H), 6.47 (s, 1H), 6.45 (s, 1H), 6.32 (dd, J = 8.2, 2.7 Hz, 1H), 5.63 (d, J = 1.5 Hz, 1H), 4.09 (d, J = 5.3 Hz, 1H), 3.76 (s, 3H), 3.68 (s, 3H), 3.63 (m, 1H), 3.33 - 3.22 (m, 2H), 3.16 (s, 3H), 3.12 (m, 1H), 2.97 (m, 1H), 2.89 (dd, J = 13.9, 3.0 Hz, 1H), 2.82 (m, 2H), 2.73 (m, 1H), 2.62 (m, 1H), 2.60 (s, 3H), 2.56 (s, 3H), 2.37 - 2.24 (m, 3H). ESI-MS *(m*/*z):* 636.38 [M + H]⁺.

### Example 5 Preparation of compound 5

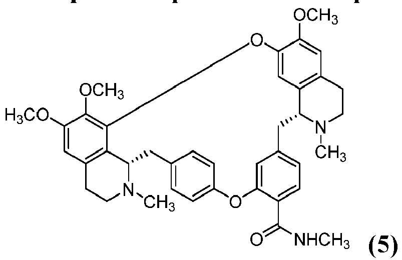

To a mixture of compound **3-a** (300 mg, 0.47 mmol) and tetrahydrofuran (2 mL) was added CDI (230 mg, 1.42 mmol) at room temperature, and stirred for 30 min at room temperature. The reaction solution was added with 28% aqueous methylamine solution (468 mg, 4.23 mmol), and continued stirring for 30 min after addition. The reaction solution was poured into water and extracted with ethyl acetate, the organic layer was separated, concentrated, and subjected to silica gel column chromatography to obtain the title compound **5** (230 mg), as a pale yellow solid with a yield of 75%. ¹H NMR (600 MHz, Chloroform-*d*) δ 8.03 (d, 1H), 7.79 (q, 1H), 7.50 (m, 1H), 7.00 (m, 3H), 6.59 (s, 1H), 6.34 (s, 1H), 6.31 (m, 2H), 5.56 (d, 1H), 4.15 (d, 1H), 3.80 (s, 3H), 3.68 (t, 1H), 3.61 (s, 3H), 3.40-3.38 (m, 1H), 3.35-3.22 (m, 2H), 3.17 (s, 3H), 3.03 (m, 1H), 2.99 (m, 3H), 2.98-2.82 (m, 3H), 2.70 (m, 2H), 2.65 (s, 3H), 2.55 (s, 3H), 2.45-2.22 (m, 3H). ESI-MS *(m*/*z):* 650.3 [M + H]⁺.

### Example 6 Preparation of compound 6

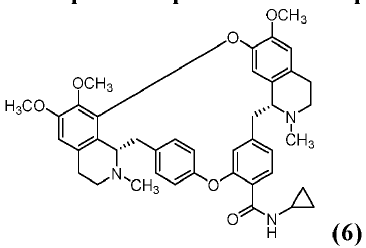

To a solution of compound **3-a** (600 mg, 0.94 mmol) in dichloromethane (5 mL) was added N,N-dimethylformamide (8 µL), and oxalyl chloride (0.4 mL, 4.72 mmol) was added dropwise at ambient temperature, after addition, the reaction continued stirring for 40 min under nitrogen protection. Then the reaction solution was concentrated to dryness and re-dissolved in dichloromethane (5 mL), the resulting solution was added dropwise to a solution of cyclopropylamine (330 µL, 4.76 mmol) in dichloromethane (4 mL) at ambient temperature. After addition, the reaction continued stirring for 30 min, and was then added with saturated aqueous ammonium chloride (10 mL) and stirred to partition. The organic phase was separated, dried over anhydrous sodium sulfate, filtered, concentrated, and subjected to column chromatography on silica gel to obtain the title compound **6** (504 mg, 0.75 mmol), as a pale yellow solid with a yield of 79%. ¹H NMR (600 MHz, Chloroform-*d*) δ 8.07 (d, 1H), 7.83 (m, 1H), 7.64 (m, 1H), 6.95 (m, 3H), 6.60 (s, 1H), 6.36 (s, 1H), 6.30 (s, 1H), 6.27 (dd, 1H), 5.52 (d, 1H), 4.30 (m, 1H), 3.78 (s, 3H), 3.71 (m, 1H), 3.61 (s, 3H), 3.55 (m, 1H), 3.42-3.20 (m, 2H), 3.17 (s, 3H), 3.15-3.00 (m, 2H), 2.90-2.82 (m, 4H), 2.71 (s, 3H), 2.64 (m, 1H), 2.57 (s, 3H), 2.48-2.25 (m, 3H), 0.77 (m, 2H), 0.51 (m, 2H). ESI-MS (*m*/*z*): 676.58 [M + H]⁺.

### Example 7 Preparation of compound 7

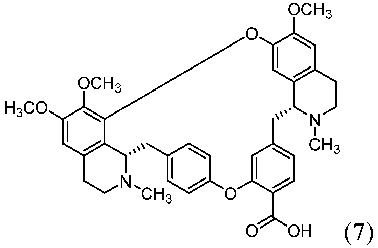

The preparation method of compound **7** (i.e., compound **3-a**) has been described in Example 3.

### Example 8 Preparation of compound 8

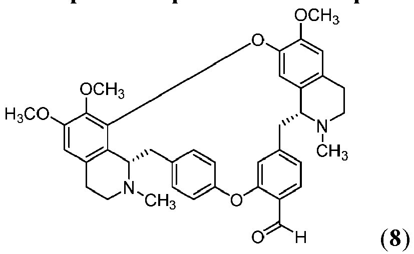

To a 50 mL round-bottomed single-necked flask was added compound **1** (500 mg, 0.81 mmol), formic acid (15 mL), and Raney nickel (1.03 g), and replaced with nitrogen. The reaction was heated to 80-90 °C for 1-2 h. TLC showed complete conversion of raw material. The reaction solution was then cooled to ambient temperature, filtered to remove insoluble material, and the filtrate was concentrated to dryness, diluted with dichloromethane, and washed with aqueous sodium bicarbonate; the organic layer was separated, dried over anhydrous sodium sulfate, filtered and concentrated, and subjected to silica gel column chromatography to obtain the title compound **8** (248 mg, 0.4 mmol), as a yellow solid with a yield of 49%. ESI-MS (*m*/*z*): 621.37 [M + H]⁺.

### Example 9 Preparation of compound 9

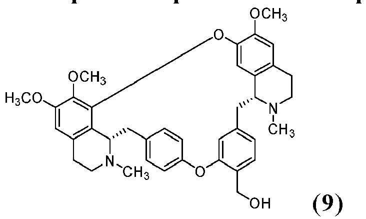

Compound **8** (80 mg, 0.13 mmol) was dissolved in 1 mL of methanol, and cooled down to 5-10 °C. Sodium borohydride (9.3 mg, 0.245 mmol, 1.9 eq) was added, after addition, the reaction was slowly warmed up to ambient temperature. TLC showed complete conversion of raw material after 1 h. The reaction was then quenched by the careful addition of saturated aqueous ammonium chloride drop by drop, added with dichloromethane and water, and stirred to partition; the organic layer was separated, dried over anhydrous sodium sulfate, filtered, concentrated, and subjected to silica gel column chromatography to obtain the title compound 9 (52 mg, 0.084 mmol), as a pale yellow solid with a yield of 64%. ESI-MS (*m*/*z*): 623.38 [M + H]⁺.

### Example 10 Preparation of compound 10 and 33

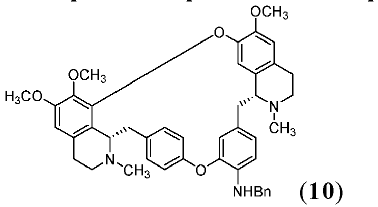

**1-a** (250 mg, 0.34 mmol), palladium acetate (12 mg, 0.05 mmol, 0.15 eq), 2-dicyclohexylphosphino-2',4',6'-triisopropylbiphenyl (49 mg, 0.10 mmol, 0.3 eq), cesium carbonate (328 mg, 1.00 mmol, 3 eq), and benzylamine(75 µL, 0.69 mmol, 2 eq) were mixed in dioxane (4 mL), and replaced with nitrogen three times. The reaction was warmed to 100-105 °C and continued to react for 14 h. TLC showed complete conversion of raw material. The reaction solution was then cooled to room temperature, filtered through diatomaceous earth, and the residue was washed with ethyl acetate; the filtrate was added with water and ethyl acetate, and stirred to partition. The ethyl acetate layer was separated, washed with saline once, dried over anhydrous sodium sulfate, concentrated, and subjected to silica gel column chromatography to obtain the title compound **10** (192 mg), as a pale yellow solid with a yield of 82%. ESI-MS (*m*/*z*): 698.47 [M + H]⁺.

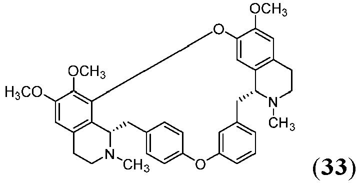

Similarly, the major by-product compound **33** (10 mg)was also isolated during silica gel column chromatography, as a pale yellow solid with a yield of 5%. ESI-MS (*m*/*z*): 593.48 [M + H]⁺.

### Example 11 Preparation of compound 11

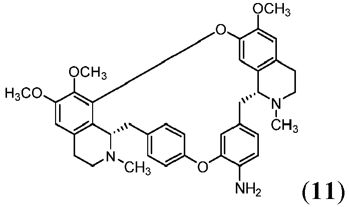

To a 25 mL round-bottomed single-necked flask was added compound **10** (190 mg, 0.27 mmol), palladium carbon (17 mg), ammonium formate (170 mg), and methanol (3 mL), and replaced twice with nitrogen. The reaction was heated to reflux for 4-5 hours. TLC showed complete conversion of raw material. The reaction solution was filtered through diatomaceous earth, and the residue was washed with ethyl acetate, the filtrate was added with water and ethyl acetate, and stirred to partition. The ethyl acetate layer was separated, washed with saline once, dried over anhydrous sodium sulfate, concentrated, and subjected to silica gel column chromatography to obtain the title compound **11** (114 mg), as an off-white solid with a yield of 69%. ESI-MS (*m*/*z*): 608.38 [M + H]⁺.

### Example 12 Preparation of compound 12 and 14

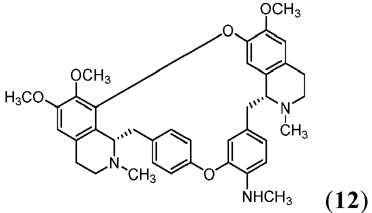

The steps are shown below:

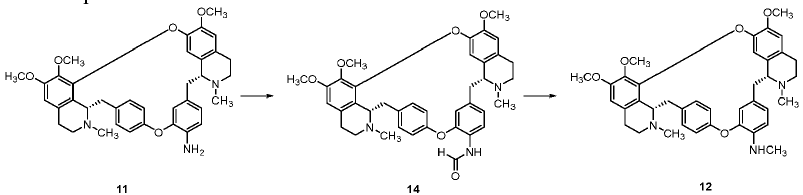

### Step 1:

To a mixture of compound **11** dihydrochloride (300 mg, 0.44 mmol) and ethyl formate (10 mL) was added triethylamine (400 mg, 3.95 mmol, 9eq) at room temperature, and the reaction was warmed to reflux to react overnight. TLC showed complete conversion of raw material. After cooling to room temperature, the reaction solution was added with 0.5 N dilute hydrochloric acid (7 mL) and stirred to partition, the organic layer was separated, then washed with saline, concentrated, and subjected to silica gel column chromatography to obtain Compound **14** (216 mg), as an off-white solid with a yield of 77%. ESI-MS (*m*/*z*): 636.37 [M + H]⁺.

### Step 2:

To a suspension of compound **14** (100 mg, 0.157 mmol) in dry tetrahydrofuran (1.2 mL) was added borane tetrahydrofuran (1 M in THF, 0.79 mL, 5 eq) dropwise under ice bath. After addition, the temperature was raised to reflux under nitrogen protection. TLC showed complete conversion of raw material. The reaction solution was cooled down to room temperature, then placed under an ice bath for 10 min, and then methanol was added carefully and dropwise with stirring. When no gas was generated, excess methanol and concentrated hydrochloric acid (95 µL) were added, and the reaction solution was refluxed for 3 h and then concentrated. The residual solution was diluted with dichloromethane, washed with saturated aqueous sodium bicarbonate, and the organic layer was separated, dried over anhydrous sodium sulfate, filtered, concentrated, and subjected to silica gel column chromatography to obtain the title compound **12** (55 mg), as an off-white solid with a yield of 56%. ESI-MS *(m*/*z):* 622.48 [M + H]⁺.

### Example 13 Preparation of compound 13

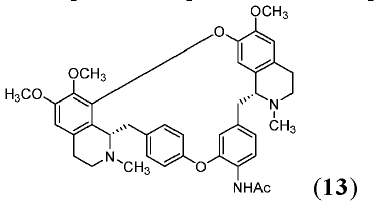

To a solution of compound **11**dihydrochloride (150 mg, 0.220 mmol) in pyridine (2.0 mL) was added acetyl chloride (41 µL, 0.57 mmol, 2.6 eq) dropwise at ambient temperature. After addition, the reaction was heated to 60 °C and reacted for 2-3 h. The reaction solution was then concentrated, and subjected to silica gel chromatography to obtain the title compound **13** (135 mg) as an off-white solid with a yield of 95%. ESI-MS *(m*/*z):* 650.48 [M + H]⁺.

### Example 14 Preparation of compound 15

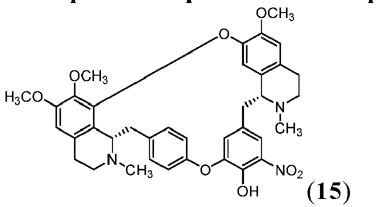

To a solution of oxyacanthine hydrochloride (1.0 g, 1.47 mmol) in acetic acid (8 mL) was added concentrated nitric acid (65-68% aqueous nitric acid, 128 µL, 1.2 eq) dropwise at ambient temperature. After addition, the reaction was stirred at room temperature for 5 min, and the reaction solution was then slowly poured into a saturated aqueous sodium bicarbonate solution, stirred until no gas was generated, and then extracted with dichloromethane. The organic layer was separated, dried over anhydrous sodium sulfate, filtered and concentrated, and subjected to silica gel column chromatography to obtain the title compound **15** (486 mg) as a yellow solid with a yield of 51%. ESI-MS (*m*/*z*): 654.36 [M + H]⁺, 652.37 [M - H]⁻.

### Example 15 Preparation of compound 16

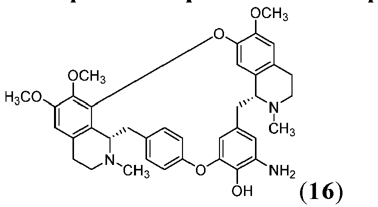

To a 25 mL round-bottomed single-necked flask was added compound **15** (525 mg, 0.80 mmol), 10% palladium carbon (55 mg), ammonium formate (610 mg, 9.68 mmol, 12 eq), methanol (12 mL), and replaced with nitrogen. The reaction was heated to reflux for 1 h. TLC showed complete conversion of raw material. The insoluble material was filtered off, and the filtrate was washed with methanol and concentrated to a small volume, and the residue was diluted with dichloromethane, washed with saturated aqueous sodium bicarbonate, washed with saline, dried over anhydrous sodium sulfate, filtered, concentrated, and subjected to silica gel column chromatography to obtain the title compound **16** (360 mg) as a pale yellow solid with a yield of 72%. ESI-MS (*m*/*z*): 624.38 [M + H]⁺.

### Example 16 Preparation of compound 17

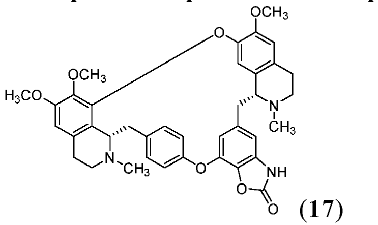

To a solution of compound **16** dihydrochloride (225 mg, 0.32 mmol) in N,N-dimethylformamide (3 mL) was added N, N'-carbonyldiimidazole (260 mg, 1.60 mmol, 5 eq) at room temperature and stirred at ambient temperature for 2 h. TLC showed complete conversion of raw material. The reaction solution was poured into 12 mL of iced water and stirred for 10 min, filtered, and the filter cake was washed twice, and blast-dried at 50 °C for 6 h to obtain the title compound **17** (127 mg) as an off-white solid with a yield of 61%. ¹H NMR (500 MHz, Chloroform-d) δ 7.45 (d, 1H), 6.90 (dd, 1H), 6.84 (dd, 1H), 6.69 (s, 1H), 6.60 (m, 1H), 6.44 (s, 1H), 6.40 (m, 1H), 6.35 (s, 1H), 5.31 (d, 1H), 4.23 (d, 1H), 3.77 (s, 3H), 3.67 (m, 1H), 3.62 (s, 3H), 3.34 (m, 1H), 3.24-3.13 (m, 3H), 3.22 (s, 3H), 3.05 - 2.91 (m, 3H), 2.90 - 2.81 (m, 2H), 2.71 (m, 1H), 2.66 (s, 3H), 2.64 (s, 3H), 2.56 - 2.45 (m, 2H). ESI-MS (*m*/*z*): 650.30 [M + H]⁺.

### Example 17 Preparation of compound 18

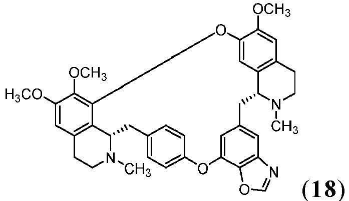

To a solution of compound **16**dihydrochloride (215 mg, 0.31 mmol) in ethanol (3 mL) was added trimethylorthoformate (0.9 mL, 8.2 mmol, 27 eq) and p-toluenesulfonic acid monohydrate (60 mg, 0.32 mmol, 1 eq) at ambient temperature, replaced three times with nitrogen, and reacted at 65-70 °C for 14 h. TLC showed complete conversion of raw material. The reaction solution was diluted with dichloromethane, washed with saturated aqueous sodium bicarbonate, and the organic layer was separated, dried over anhydrous sodium sulfate, filtered, concentrated, and subjected to silica gel column chromatography to obtain the title compound **18** (168 mg), as an off-white solid with a yield of 86%. ESI-MS (*m*/*z*): 634.48 [M + H]⁺.

### Example 18 Preparation of compound 19

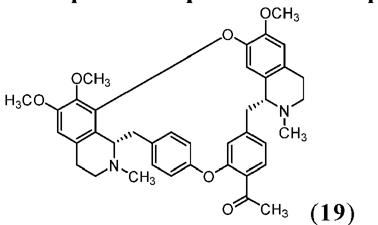

Compound 1 (605 mg, 0.98 mmol) was dissolved in dry tetrahydrofuran (5 mL) under nitrogen protection, placed in ice bath for 5 min, and then slowly added with methyl lithium (1.53 mL, 1.6 M ethyl ether solution, 2.5 eq) dropwise. After addition, the reaction continued under ice bath for 80 min, and then carefully poured into ice water, and the pH was adjusted to 2-3 by addition of 2 N aqueous sulfuric acid. The reaction solution was stirred at ambient temperature for 1 h, adjusted to weakly basic of pH with sodium bicarbonate, extracted with dichloromethane, and the organic phase was separated, dried over anhydrous sodium sulfate, filtered, concentrated, and subjected to silica gel column chromatography to obtain the title compound **19** (143 mg), as a pale yellow solid with a yield of 23%. ESI-MS (*m*/*z*): 635.38 [M + H]⁺.

### Example 19 Preparation of compound 21

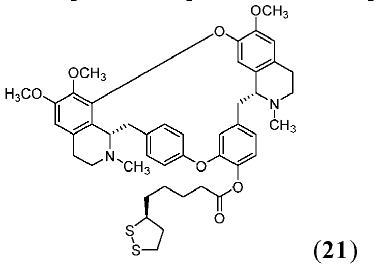

To a suspension of 500 mg of oxyacanthine hydrochloride in dichloromethane (5 mL) was added lipoic acid (160 mg), 1-(3-dimethylaminopropyl)-3-ethylcarbodiimide hydrochloride (220 mg), and 4-dimethylaminopyridine (15 mg) sequentially, and stirred at 23-25 °C for 4-6 h. TLC showed complete conversion of raw material. The reaction solution was added with 4 mL of water and stirred to partition; the organic layer was separated, then washed once with 2 mL of saturated saline; the organic layer was separated, dried over anhydrous sodium sulfate, filtered, concentrated, and subjected to silica gel column chromatography to obtain 380 mg of the title compound **21**, as a pale yellow solid with a yield of 65%. ESI-MS (m/z): 797.48 [M + H]⁺.

### Example 20 Preparation of compound 22

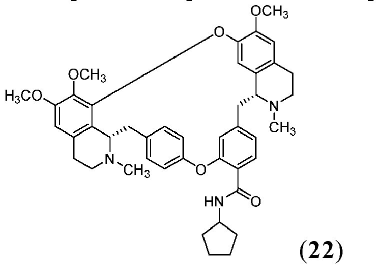

By referring to the method of Example 6, the title compound **22** was prepared from the reaction of compound 7 and cyclopentylamine, as a pale yellow solid with a yield of 57%. ESI-MS *(m*/*z):* 704.48 [M + H]⁺.

### Example 21 Preparation of compound 27

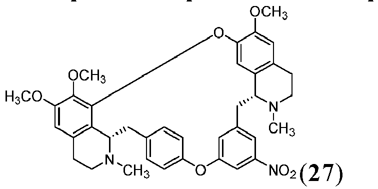

The steps are shown below:

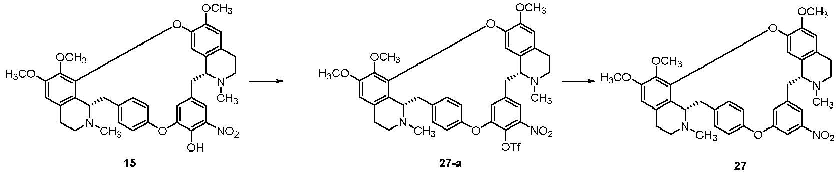

### Step 1:

To a solution of compound **15** (950 mg, 1.45 mmol) in dichloromethane (7 mL) was added pyridine (565 mg, 7.14 mmol, 4.9 eq) and 4-dimethylaminopyridine (14 mg, 0.115 mmol, 0.08 eq), and stirred for 5-10 min under ice bath. Then trifluoromethanesulfonic anhydride (0.49 mL, 2 eq) was added dropwise, after addition, the temperature was maintained for 15 min. TLC showed complete conversion of raw material. The reaction solution was then washed with 0.5 N dilute hydrochloric acid, and the organic phase was separated, dried over anhydrous sodium sulfate, filtered, concentrated, and subjected to silica gel column chromatography to obtain the product **27-a** (800 mg), as a yellow solid with a yield of 70%, ESI-MS (*m*/*z*): 786.28 [M + H]⁺.

### Step 2:

To a 10 mL round-bottomed single-necked flask was added **27-a** (205 mg, 0.26 mmol), palladium acetate (7 mg, 0.12 eq), 2-dicyclohexylphosphino-2,4,6-triisopropylbiphenyl (27 mg, 0.21 eq), formic acid (33 µL, 3.3 eq) and N, N-diisopropylethylamine (152 µL, 3.3 eq), and dioxane (2.0 mL) in turn, replaced with nitrogen for several times, and stirred at 85-90 °C for 50 min. TLC showed complete conversion of raw material. The reaction solution was then cooled to room temperature, diluted with ethyl acetate, filtered to remove insoluble matter, the filtrate was washed with water, and the ethyl acetate layer was separated, dried over anhydrous sodium sulfate, filtered, concentrated, and subjected to silica gel column chromatography to obtain the title compound **27**(97 mg), as a pale yellow solid with a yield of 59%. ESI-MS *(m*/*z):* 638.24 [M + H]⁺.

### Example 22 Preparation of compound 34

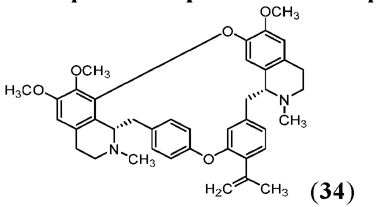

To a 5 mL round-bottomed single-necked flask was added Compound **1-a** (120 mg, 0.162 mmol), palladium acetate (4 mg, 0.11 eq), 2-dicyclohexylphosphino-2,4,6-triisopropylbiphenyl (15 mg, 0.19 eq), isopropenylboronic acid pinacol ester (60 µL, 2 eq), dioxane (1.5 mL), and water (0.4 mL) successively, and finally, sodium carbonate (53 mg, 3 eq) was added, and replaced with nitrogen three times. The reaction was raised to 70-75 °C and reacted overnight. After cooling to room temperature, the reaction solution was diluted with dichloromethane, washed with water, and the organic phase was separated, dried over anhydrous sodium sulfate, filtered, concentrated, and subjected to silica gel column chromatography to obtain the title compound **34** (55 mg) as a pale yellow solid with a yield of 54%. ESI-MS *(m*/*z):* 633.57 [M + H]⁺.

### Example 23 Preparation of compound 35

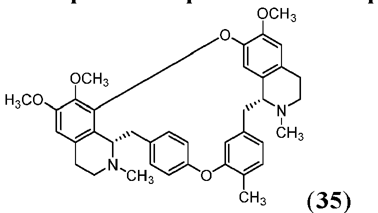

By referring to the method of Example 22, the title compound **35** was prepared from the reaction of compound **1-a** and methylboronic acid, as a pale yellow solid with a yield of 54%. ESI-MS *(m*/*z):* 607.38 [M + H]⁺.

### Example 24 Preparation of compound 36

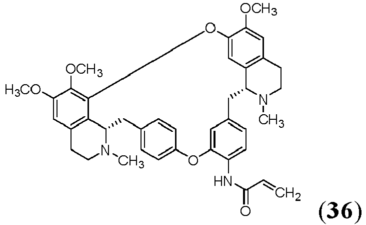

To a solution of compound **11** (100 mg, 0.164 mmol) in dichloromethane (2.0 mL) was added pyridine (66 µL, 5 eq) and acryloyl chloride (27 µL, 2 eq) sequentially at ambient temperature. After addition, the reaction was carried out at ambient temperature for 30 min. TLC showed complete conversion of raw material. The reaction solution was concentrated to dryness and subjected to silica gel column chromatography to obtain the title compound **36** (70 mg) as an off-white solid with a yield of 65%. ESI-MS (*m*/*z*): 662.48 [M + H]⁺.

### Example 25 Preparation of compound 37

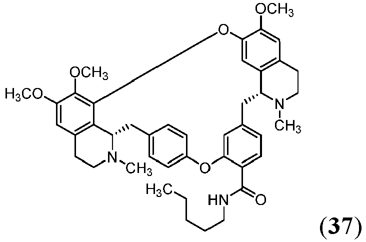

By referring to the method of Example 6, the title compound **37** was prepared from the reaction of compound **7** and n-amylamine, as a pale yellow solid with a yield of 67%. ESI-MS *(m*/*z):* 706.48 [M + H]⁺.

### Example 26 Preparation of compound 38

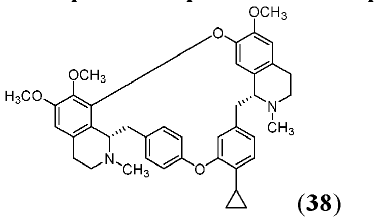

By referring to the method of Example 22, the title compound **38** was prepared from the reaction of compound **1-a** and cyclopropylboronic acid, as a pale yellow solid with a yield of 59%. ESI-MS *(m*/*z):* 633.38 [M + H]⁺.

### Example 27 Preparation of compound 39

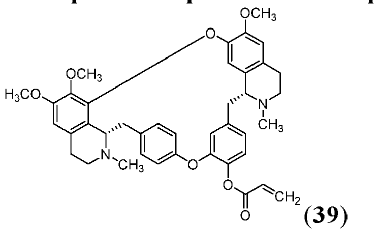

To a suspension of oxyacanthine hydrochloride (600 mg, 0.88 mmol) in dichloromethane (8 mL) was added triethylamine (0.62 mL, 5 eq), and stirred for 5-10 min under ice bath. Acryloyl chloride (110 µL, 1.5 eq) was added dropwise, and after addition, the reaction solution was stirred at room temperature for 50 min, TLC showed complete conversion of raw material. Saturated aqueous ammonium chloride was added and stirred to partition; the organic layer was separated, dried over anhydrous sodium sulfate, filtered, concentrated, and subjected to silica gel column chromatography to obtain the title compound **39** (240 mg) as an off-white solid with a yield of 41%. ESI-MS (*m*/*z*): 663.2 [M + H]⁺.

### Example 28 Preparation of compound 40

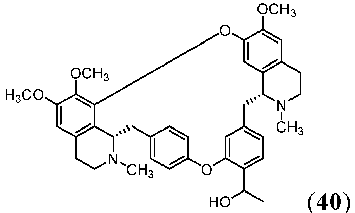

Under nitrogen protection, compound **8** (300 mg, 0.483 mmol) was mixed with methylmagnesium bromide (7.4 mL, 3.0 M tetrahydrofuran solution) under an ice bath, then the reaction solution was slowly warmed up to room temperature and stirred for 2-3 h. The reaction solution was then poured into ice water, added with dichloromethane and an appropriate amount of ammonium chloride, and stirred to partition; the organic layer was separated, washed with saline, dried over anhydrous sodium sulfate, filtered, concentrated, and subjected to silica gel column chromatography to obtain the title compound **40** (165 mg) as a pale yellow solid with a yield of 54%. ESI-MS *(m*/*z):* 637.2 [M + H]⁺.

### Example 29 Preparation of compound 41

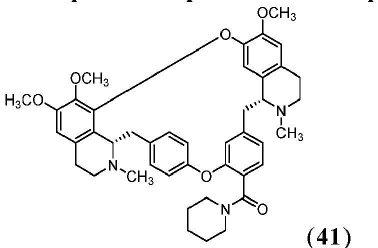

By referring to the method of Example 6, the title compound **41** was prepared from the reaction of compound **7** and piperidine, as a pale yellow solid with a yield of 76%. ESI-MS *(m*/*z):* 704.68 [M + H]⁺.

### Example 30 Preparation of compound 43

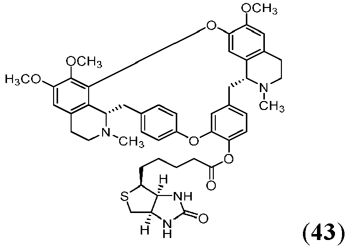

To a suspension of oxyacanthine (480 mg, 0.79 mmol) and D-biotin (240 mg, 0.98 mmol, 1.2 eq) in dichloromethane (5 mL) was added 1-(3-dimethylaminopropyl)-3-ethylcarbodiimide hydrochloride (230 mg, 1.20 mmol, 1.5 eq), and 4-dimethylaminopyridine (20 mg, 0.16 mmol, 0.2 eq) successively, after addition, the reaction was carried out overnight at ambient temperature. TLC showed complete conversion of raw material. The reaction solution was added with saturated aqueous ammonium chloride solution and stirred to partition; the organic layer was separated, washed with salt water, dried over anhydrous sodium sulfate, concentrated, and subjected to silica gel column chromatography to obtain the title compound **43** as an off-white solid (435 mg) with a yield of 66%. ESI-MS *(m*/*z):* 835.48 [M + H]⁺.

### Example 31 Preparation of compound 44

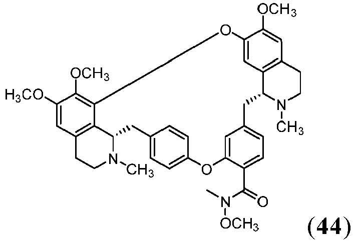

By referring to the method of Example 6, the title compound **44** was prepared from the reaction of compound 7 and dimethylhydroxylamine hydrochloride, as a pale yellow solid with a yield of 78%. ESI-MS *(m*/*z):* 680.3 [M + H]⁺.

### Example 32 Preparation of compound 45

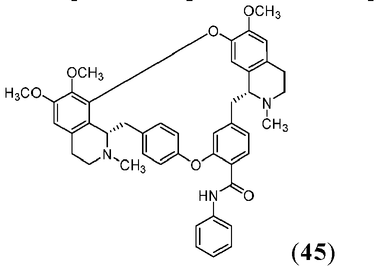

To a solution of compound **7** (452 mg, 0.709 mmol) in dichloromethane (2 mL) was added N,N-dimethylformamide (5.5 µL), and oxalyl chloride (0.305 mL, 3.61 mmol) was added dropwise at ambient temperature. After addition, the reaction continued for 20 min under nitrogen protection. Then the reaction solution was concentrated to dryness and re-dissolved in dichloromethane (5 mL). The resulting solution was added dropwise to a solution of aniline (0.34 mL, 3.73 mmol), pyridine (0.28 mL, 3.46 mmol) in dichloromethane (5 mL) at room temperature. After addition, the reaction solution continued stirring at ambient temperature for 10 min, TLC showed complete conversion of raw material. The reaction solution was added with saturated aqueous ammonium chloride and stirred to partition; the organic layer was separated, dried over anhydrous sodium sulfate, filtered, concentrated, and subjected to silica gel column chromatography to obtain the title compound **45** (259 mg), as a pale yellow solid with a yield of 51%. ESI-MS (*m*/*z*): 712.2 [M + H]⁺.

### Examople 33 Preparation of compound 46

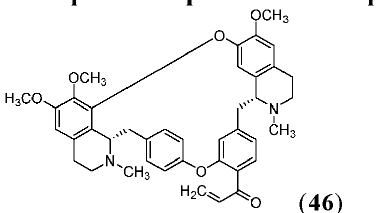

Compound **44** (393 mg, 0.573 mmol) was dissolved in dried tetrahydrofuran (5 mL) under nitrogen protection, cooled down to 0-5 °C under an ice bath, and vinylmagnesium bromide (1.75 mL, 1.75 mmol, 3 eq, 1 M tetrahydrofuran solution) was slowly added dropwise. After addition, the reaction was carried out for 1-2 h at ambient temperature. TLC showed complete conversion of raw material. The reaction solution was carefully added to 1 N hydrochloric acid (5 mL), and stirred for 20 min before adjusting the pH to about 8 with aqueous sodium hydroxide, and then extracted with dichloromethane. The organic phase was dried over anhydrous sodium sulfate, filtered, concentrated, and subjected to silica gel column chromatography to obtain the title compound **46** (290 mg, 0.449 mmol), as a pale yellow solid with a yield of 78%. ESI-MS (*m*/*z*): 647.48 [M + H]⁺.

### Example 34 Preparation of compound 47

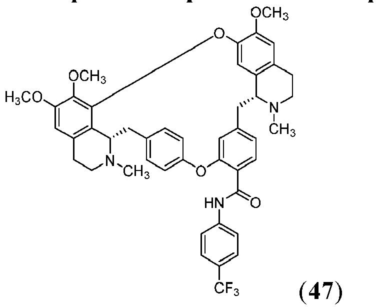

By referring to the method of Example 32, the title compound **47** was prepared from the reaction of compound 7 and 4-trifluoromethylaniline, as a pale yellow solid with a yield of 58%. ESI-MS (m/z): 780.48 [M + H]⁺.

### Example 35 Preparation of compound 48

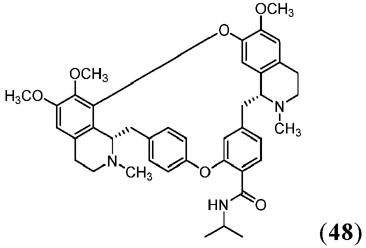

By referring to the method of Example 6, the title compound **48** was prepared from the reaction of compound 7 and isopropylamine, as a pale yellow solid with a yield of 59%. ESI-MS (m/z): 678.48 [M + H]⁺.

### Example 36 Preparation of compound 49

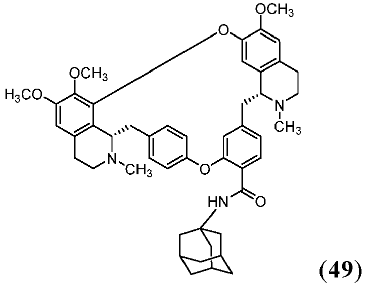

By referring to the method of Example 6, the title compound **49** was prepared from the reaction of compound 7 and 1-amantadine, as a pale yellow solid with a yield of 64%. ESI-MS (m/z): 770.58 [M + H]⁺.

### Example 37 Preparation of compound 50

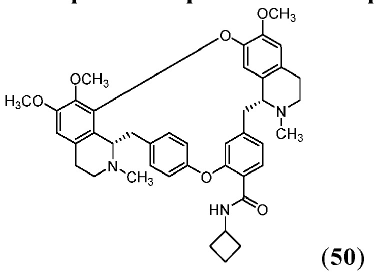

By referring to the method of Example 6, the title compound **50** was prepared from the reaction of compound 7 and cyclobutylamine, as a pale yellow solid with a yield of 58%. ESI-MS (m/z): 690.48 [M + H]⁺.

### Example 38 Preparation of compound 51

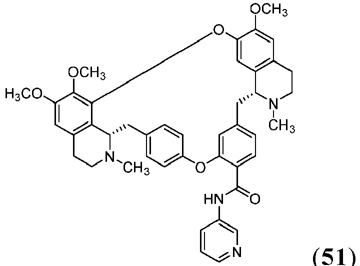

By referring to the method of Example 32, the title compound **51** was prepared from the reaction of compound 7 and 3-aminopyridine, as a pale yellow solid with a yield of 71%. ESI-MS (m/z): 713.38 [M + H]⁺.

### Example 39 Preparation of compound 52

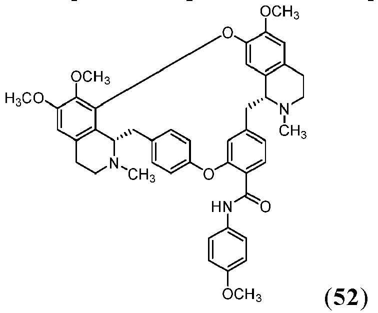

By referring to the method of Example 32, the title compound **52** was prepared from the reaction of compound 7 and methoxyaniline, as a pale yellow solid with a yield of 72%. ESI-MS (m/z): 742.52 [M + H]⁺.

### Example 40 Preparation of compound 53

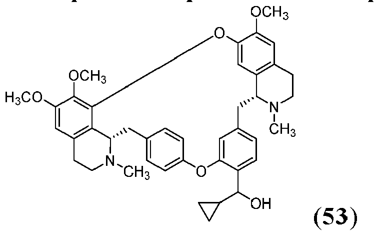

By referring to the method of Example 28, the title compound **53** was prepared from the reaction of compound **8** and cyclopropyl magnesium bromide as a pale yellow solid with a yield of 64 %, ESI-MS (m/z): 663.35 [M + H]⁺.

### Example 41 Preparation of compound 54

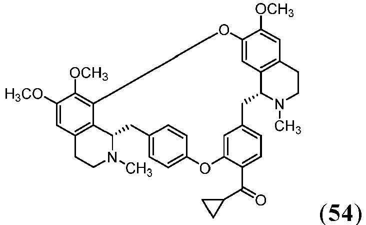

To a solution of compound **53** (183 mg, 0.28 mmol) in dichloromethane (2 mL) was added Dess-Martin Periodinane (174 mg, 0.41 mmol, 1.5 eq) at ambient temperature. After addition, the reaction continued for 1 h under nitrogen protection. TLC showed complete conversion of raw material. The reaction solution was then diluted with dichloromethane, washed once with saturated sodium bicarbonate solution, and the organic layer was separated, dried over anhydrous sodium sulfate, filtered, concentrated, and subjected to silica gel column chromatography to obtain the title compound **54** (38 mg, 0.057 mmol), as a pale yellow solid with a yield of 20%. ESI-MS (m/z): 661.28 [M + H]⁺.

### Example 42 Preparation of compound 55

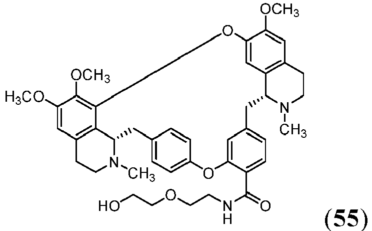

By referring to the method of Example 6, the title compound **55** was prepared from the reaction of compound 7 and diglycolamine as a pale yellow solid with a yield of 61%. ESI-MS (m/z): 724.28 [M + H]⁺.

### Example 43 Preparation of compound 56

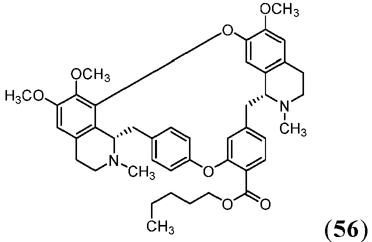

By referring to the method of step 2 in Example 3, the title compound **56** was prepared from the reaction of compound 7 and n-pentanol, as an off-white solid with a yield of 82%. ESI-MS (m/z): 707.46 [M + H]⁺.

### Example 44 Preparation of compound 57

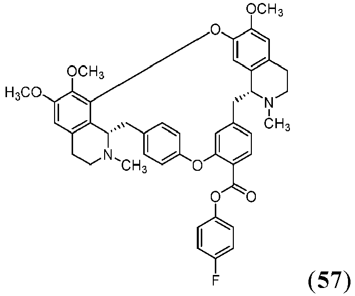

By referring to the method of Example 32, the title compound **57** was prepared from the reaction of compound 7 and p-fluorophenol, as a pale yellow solid with a yield of 55%. ESI-MS (m/z): 731.26 [M + H]⁺.

### Example 45 Preparation of compound 58

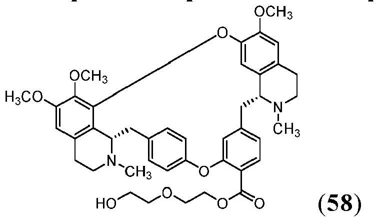

By referring to the method of step 2 in Example 3, the title compound **58** was prepared from the reaction of compound 7 and diethylene glycol, as an off-white solid with a yield of72%. ¹H NMR (500 MHz, Chloroform-d) δ 7.70 (d, 1H), 7.34 (m, 1H), 7.02 (m, 1H), 6.94 (dd, 1H), 6.89 (dd, 1H), 6.60 (s, 1H), 6.38 (s, 1H), 6.30 - 6.29 (m, 2H), 5.49 (d, 1H), 4.50 (m, 1H), 4.42 (m, 2H), 3.78 (s, 3H), 3.77 (m, 2H), 3.70 (m, 1H), 3.63 (m, 2H), 3.61 (s, 3H), 3.58 (m, 2H), 3.47 (m, 1H), 3.29-3.05 (m, 3H), 3.17 (s, 3H), 3.02-2.67 (m, 5H), 2.77 (s, 3H), 2.60 (s, 3H), 2.55-2.26 (m, 3H).ESI-MS (m/z): 725.26 [M + H]⁺.

### Example 46 Preparation of compound 59

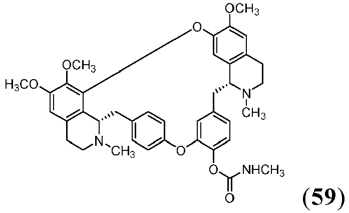

By referring to the method of Example 2, the title compound **59** was prepared from the reaction of oxyacanthine hydrochloride and methyl isocyanate, as a pale yellow solid with a yield of 73%. ESI-MS (m/z): 666.26 [M + H]⁺.

### Example 47 Preparation of compound 60

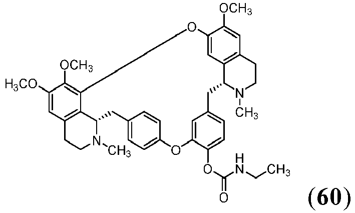

By referring to the method of Example 2, the title compound **60** was prepared from the reaction of oxyacanthine hydrochloride and ethyl isocyanate, as a pale yellow solid with a yield of 69%. ESI-MS (m/z): 680.28 [M + H]⁺.

### Example 48 Preparation of compound 61

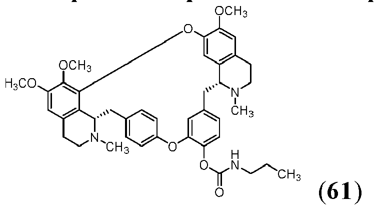

By referring to the method of Example 2, the title compound **61** was prepared from the reaction of oxyacanthine hydrochloride and n-propyl isocyanate, as a pale yellow solid with a yield of 76%. ESI-MS (m/z): 694.24 [M + H]⁺.

### Example 49 Preparation of compound 62

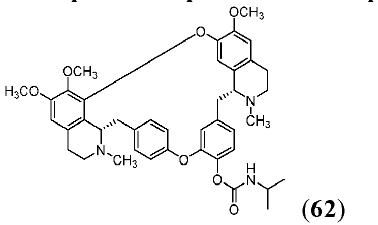

By referring to the method of Example 2, the title compound **62** was prepared from the reaction of oxyacanthine hydrochloride and isopropyl isocyanate, as a pale yellow solid with a yield of 70%. ESI-MS (m/z): 694.28 [M + H]⁺.

### Example 50 Preparation of compound 63

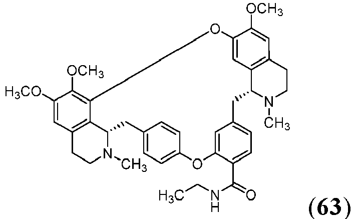

By referring to the method of Example 5, the title compound **63** was prepared from the reaction of compound 7 and ethylamine aqueous solution, as a pale yellow solid with a yield of 80%. ESI-MS (m/z): 664.24 [M + H]⁺.

### Example 51 Preparation of compound 64

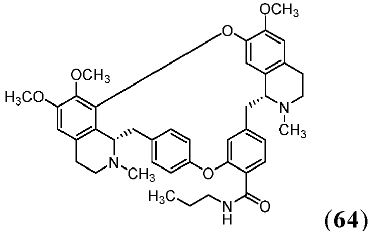

By referring to the method of Example 6, the title compound **64** was prepared from the reaction of compound **7** and n-propylamine, as a pale yellow solid with a yield of 73%. ESI-MS (m/z): 678.26 [M + H]⁺.

### Example 52 Preparation of compound 65

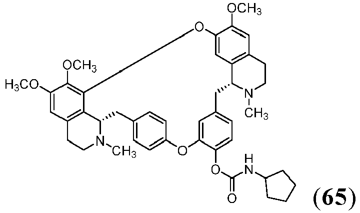

By referring to the method of Example 2, the title compound **65** was prepared from the reaction of oxyacanthine hydrochloride and cyclopentylisocyanate, as an off-white solid with a yield of 76%. ESI-MS (m/z): 720.45 [M + H]⁺.

### Example 53 Preparation of compound 66

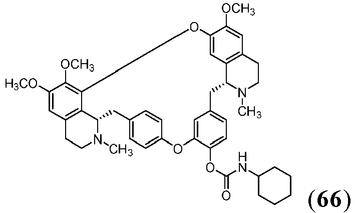

By referring to the method of Example 2, the title compound **66** was prepared from the reaction of oxyacanthine hydrochloride and cyclohexyl isocyanate, as an off-white solid with a yield of 71%. ESI-MS (m/z): 734.48 [M + H]⁺.

### Pharmacological experiments

### 1) Anti novel coronavirus activity test

Determination of replication inhibitory activity of compounds against 2019 novel coronavirus (SARS-CoV-2): Vero E6 cells were purchased from ATCC and SARS-CoV-2 virus was obtained from the National Virus Resource Bank Microbial Virus Species Conservation Center. Vero E6 cells were cultured overnight in 48-well cell culture dishes at a density of 5 × 10⁴ cells/well, and pre-treated with different concentrations of the samples to be tested for 1 h. Viruses were then added (infection complex MOI was 0.01) to infect the cells for 1 h. The virus-compound mixtures were then removed, and the cells were further cultured with fresh medium containing the samples to be tested. At 24 h p.i., cell supernatants were collected and cells were lysed in lysis buffer, and the viral copy number in the cell supernatants was quantitatively assessed by quantitative real-time RT-PCR (qRT-PCR).

The results show (Table 1) that multiple compounds significantly inhibited SARS-CoV-2 virus replication, and the EC₅₀ values of which inhibiting SARS-CoV-2 virus replication were all significantly better than that of oxyacanthine hydrochloride, with only about 1/10 or less of that of oxyacanthine hydrochloride. This suggests that the compounds of the present invention have significantly improved inhibitory activity against the coronavirus SARS-CoV-2. In addition, activity test against novel coronavirus variant strains showed that compounds 2 and 4 showed strong inhibition against the delta strain, and compounds 5 and 6 also showed strong inhibition against the South African strain.

### 2) Novel coronavirus envelope protein (2-E) ion channel activity test in vitro

Determination of ion channel inhibitory activity of compounds against novel coronavirus envelope protein (2-E): the channel activity of purified 2-E protein was assayed by planar lipid bilayer workstation (BLM) at room temperature. Phosphatidylcholine (PC) and phosphatidylserine (PS) stored in chloroform were mixed in a ratio of 3:2, blown dry with nitrogen, and then dissolved with decane to a final concentration of 50 mg/ml. The cup and trough in the BLM system were mounted with the cup side defined as the cis side, and the contralateral side as the trans side, and 1 ml of internal and external fluid was added. The mounted device was placed on the operating table of the planar lipid bilayer workstation, and the small holes in the middle of the cup were observed using a stereomicroscope, and the prepared phospholipids were applied to the hole of the cup, and then the purified 2-E protein was added on the cis side. Driven by an electrochemical gradient as well as a voltage, the protein was inserted into the artificial lipid bilayer membrane. The currents were recorded by voltage clamp mode of the membrane clamp amplifier, the signals were acquired with Clampex 10.3 software, and the conductance and open frequency of a single channel were fitted by a Gaussian function, where the current open time less than 0.5-1.5 ms will be considered as noise. The current amplitude was recorded under different conditions by varying different voltages and the reversal potential was observed.

In evaluating the channel inhibitory activity of the compounds using the BLM system, the current was observed to appear and then recorded for three minutes then the compound to be tested was added on the trans side and stirred for three seconds, followed by the recording of the current signal for ten minutes and the change in current was observed.

For recording the inhibition of multiple concentrations of drugs and calculating the half inhibitory concentration (IC₅₀), after the current was opened and recorded for three minutes, compounds with final concentrations of 1, 10, 50, and 100 µM were added one by one and recorded continuously for three minutes each. The inhibition of this channel by specific compounds at different concentrations was observed and the IC₅₀ was calculated.

### 3) In vitro activity test of cardiotoxicity-related targets (hERG and Nav1.5)

hERG:CHO (Chinese Hamster Ovary) cells stably expressing hERG potassium channels were used to record hERG potassium channel currents by whole-cell membrane clamp technique at room temperature. The glass microelectrodes were made from glass electrode blanks (BF150-86-10, Sutter) by pulling through a puller, and the tip resistance was about 2-5 MS2 after filling the electrode with internal fluid, and the glass microelectrodes were connected to the membrane clamp amplifier by inserting the glass microelectrodes into the amplifier probe. Clamp voltage and data recording were controlled and recorded by pClamp 10 software via a computer with a sampling frequency of 10 kHz and a filtering frequency of 2 kHz. After obtaining whole-cell recordings, the cells were clamped at -80 mV, and the step voltage inducing hERG potassium current (I hERG) was given a depolarizing voltage from -80 mV for 2 s to +20 mV, then repolarized to -50 mV for 1 s and returned to -80 mV. This voltage stimulus was given every 10 s; after the hERG potassium current was determined to be stabilized (for at least 1 min), begins the dosing process. Compounds were given for at least 1 min for each tested concentration, and at least 2 cells (n≥2) were tested per concentration.

Extracellular fluid formulation (mM): 140 NaCl, 5 KCl, 1 CaCl₂, 1.25 MgCl₂, 10 HEPES and 10 Glucose, adjusting the pH to 7.4 with NaOH.

Intracellular fluid formulation (mM): 140 KCl, 1 MgCl₂, 1 CaCl₂ , 10 EGTA and 10 HEPES, adjusting the pH to 7.2 with KOH.

**Nav1.5:** CHL cells stably expressing hNav1.5 sodium channel was used to record hNav1.5 sodium channel currents by whole-cell membrane clamp technique at room temperature. The glass microelectrodes were made from glass electrode blanks (BF150-86-10, Sutter) by pulling through a puller, and the tip resistance was about 2-5 MS2 after filling the electrode with internal fluid, and the glass microelectrodes were connected to Axopatch 200B (Molecular Devices) membrane clamp amplifier by inserting the glass microelectrodes into the amplifier probe. Clamp voltage and data recording were controlled and recorded by pClamp 10 software via a computer with a sampling frequency of 10 kHz and a filtering frequency of 2 kHz. After obtaining whole-cell recordings, the cells were clamped at -80 mV, given a hyperpolarizing voltage of 200 ms to -120 mV, and then depolarized to -20 mV for 20 ms to evoke a Nav 1. 5 current. This voltage stimulus was given every 10 s; after the hNav1.5 sodium current was determined to be stabilized (for at least 1 min), begins the dosing process. Concentrations of the sample to be tested were administered continuously starting from low test concentration, and each test concentration was given for 1 min to reach steady state of action or for a maximum of 3 min. A minimum of 3 cells (n≥3) were tested at each concentration of the sample to be tested, and a minimum of 2 cells (n≥2) were tested at each concentration of the positive control.

Extracellular fluid formulation (mM): 140 NaCl, 5 KCl, 1 CaCl₂, 1.25 MgCl₂, 10 HEPES and 10 Glucose, adjusting the pH to 7.4 with NaOH.

Intracellular fluid formulation (mM): 130 CsF, 10 NaCl, 10 EGTA and 10 HEPES, adjusting the pH to 7.2 with CsOH.

### 4) Cytotoxicity test

In this embodiment, the half toxicity concentration of each embodiment on Vero E6 cells was determined by CCK8 kit analysis (CC50).

The results showed that compounds 2, 3, 4, 5, 6 and 58 showed weaker cytotoxicity than oxyacanthine hydrochloride on Vero E6 cells, and the therapeutic index SIs were all much better than that of oxyacanthine hydrochloride (Table 1). Especially, compounds 3, 4, 6 and 58 had SI values greater than compound50, and much higher than the other compounds.

**Table 1. Inhibitory activity against 2019 novel coronavirus (SARS-CoV-2) original strain, South African variant and delta variant strains, and HCoV OC43 replication, and novel coronavirus envelope protein (2-E) ion channels, cardiotoxicity-related targets and cytotoxicity results**

| Compou nd No. | Inhibiti on against viral replicat ion of the origina 1 strain EC50( µM) | Inhibiti on against viral replicat ion of South African mutant strain EC50(µ M) | Inhibiti on against viral replicati on of delta mutant strain EC50(µ M) | Inhibiti on against HCoV OC43 virus replicat ion EC50 (µM) | Inhibiti on against 2-E ion channel s IC₅₀ (µM) | hERG IC₅₀ (µM) | Nav1.5 IC₅₀ (µM) | Cytotoxicity IC₅₀(µM) | Therapeuti c indexSI*^{a}* |
|---|---|---|---|---|---|---|---|---|---|
| Compou nd **1** | 0.1-1 | | | 4.2 | <10 | 6.4 | | 10-30 | 30-50 |
| Compou nd **2** | 0.1-1 | | 0.3 | 1.2 | <10 | 19 | >10 | 30-50 | 30-50 |
| Compou nd **3** | 0.1-1 | | | 2.3 | <10 | 7.0 | | 30-50 | >50 |
| Compou nd **4** | 0.1-1 | | 1.5 | 5.0 | <1 | 20 | >50 | >50 | >100 |
| Compou nd **5** | 1-10 | 2.7 | | | | 9.4 | | >50 | 10-30 |
| Compou nd **6** | 0.1-1 | 2.2 | | | | 8.9 | | >50 | >50 |
| Compou nd **7** | | | | | 10-30 | >40 | | >50 | |
| Compou nd **9** | 0.5-1.5 | | | 3.0 | 10-30 | | | >50 | >33 |
| Compou **nd 17** | 0.1-1 | | | | | | | 10-30 | >10 |
| Compou nd **19** | 0.5-1.5 | | | | | | | 30-50 | >20 |
| Compou nd **22** | 0.5-1.5 | | | | | | | 10-30 | >6 |
| Compou nd **33** | 0.1-1 | | | 4.1 | | >40 | | 10-30 | >10 |
| Compou nd **34** | 0.5-1.5 | | | | <10 | | | 10-30 | >6 |
| Compou nd **35** | 1-5 | | | | | | | 10-30 | >2 |
| Compou nd **37** | 1-5 | | | | <10 | >20 | | 10-30 | >2 |
| Compou nd **38** | 1-5 | | | | | | | 10-30 | >2 |
| Compou nd **40** | 0.1-1 | | | | | | | 30-50 | >30 |
| Compou nd **45** | 1-5 | | | | <10 | >20 | | 10-30 | >2 |
| Compou nd **48** | 0.1-1 | | | | | | | 10-30 | >10 |
| Compou nd **49** | 0.1-1 | | | | | | | 5-10 | >5 |
| Compou nd **50** | 0.1-1 | | | | | 4.5 | | 30-50 | >30 |
| Compou nd **51** | 0.1-1 | | | | | | | 10-30 | >10 |
| Compou nd **52** | 0.1-1 | | | | | | | 10-30 | >10 |
| Compou nd **54** | 1-5 | | | | | | | 10-30 | >2 |
| Compou nd **55** | 1-5 | | | | | >40 | | >100 | >20 |
| Compou nd **57** | 0.5-1.5 | | | | | | | 5-10 | >3 |
| Compou nd **58** | 0.1-1 | | | | | 16.5 | | >50 | >50 |
| **Oxyaca nthine hydroc hloride** | >10 | | | | >100 | | | <30 | <3 |

| | | | | | | | | | |
|---|---|---|---|---|---|---|---|---|---|
| *^{a}*SI = Cytotoxicity CC₅₀ value / Inhibition against viral replication of original strain EC₅₀ value. | | | | | | | | | |

As shown in Table 1, the compounds of the present invention inhibited the viral replication of the original strain of SARS-CoV-2 virus with significantly better IC₅₀ values than oxyacanthine hydrochloride, and had a large therapeutic index and good safety, especially compounds 3, 4, 6, and 58. The compounds of the present invention also has a better inhibitory effect on the viral replication of the mutant strain of SARS-CoV-2 virus, wherein compounds 5 and 6 significantly inhibited the viral replication of the South African mutant strain, and compounds 2 and 4 significantly inhibited the viral replication of the delta variant strain. The compounds of the present invention showed a better inhibitory effect on the E protein ion channel of SARS-CoV-2, with IC₅₀ values significantly better than those of oxyacanthine hydrochloride, especially compound 4 (IC₅₀<1 µM). In addition, compounds 2 and 4 were weakly active against hERG ion channels and Nav1.5 channels, which are highly associated with cardiotoxicity, and are expected to have less cardiac-related toxicities. In addition, compounds 1, 2, 3 and 4 also showed strong inhibition against human coronavirus HCoV OC43.

### 5) CD4⁺T cell differentiation assay

The inhibitory effect of compounds on Th1/Th17 activity was investigated by a CD4⁺T cell in vitro differentiation system, which assayed the levels of IFN-γ in Th1 CD4⁺T differentiation and IL-17A in Th17 CD4⁺T differentiation.

### Experimental Methods:

CD4⁺T cell isolation: CD4⁺T cells were isolated from the spleens of 7-8 week old C57BL/6 mice using the Mouse CD4⁺T Cell Sorting Kit (Dynabeads^{®} UntouchedTM Mouse CD4 Cells Isolation Kit, Invitrogen, Catalog nos:11415D).

In vitro differentiation of CD4⁺T cells: the isolated CD4⁺T cells were inoculated into 96-well plates while different concentrations of compounds were added, and then corresponding cytokines and antibodies were added: anti-CD28 (2 µg/ml), anti-CD3 (2 µg/ml), Anti-IL-4 (10 µg/ml), IL-12 (10 ng/ml), Anti-IFN-γ (10 µg/ml), anti-IL-4 (10 µg/ml), IL-6 (30 ng/ml), TGF-β1 (3 ng/ml), IL-1β (10 ng/ml), IL-23 (10 ng/ml), and TNF-α (10 ng/ml). Cells were cultured in 5% CO₂ at 37 °C in a cell culture chamber for 72 h. Cells were collected, stained intracellularly, analyzed by flow cytometry, and tested for IFN-γ/ IL-17A content to assess the effect of compounds on CD4⁺T differentiation into Th1/Th17 in vitro.

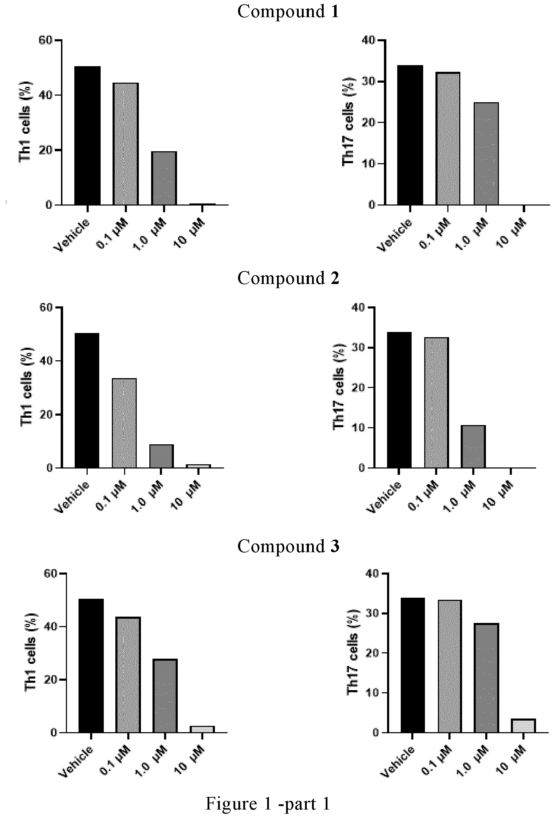

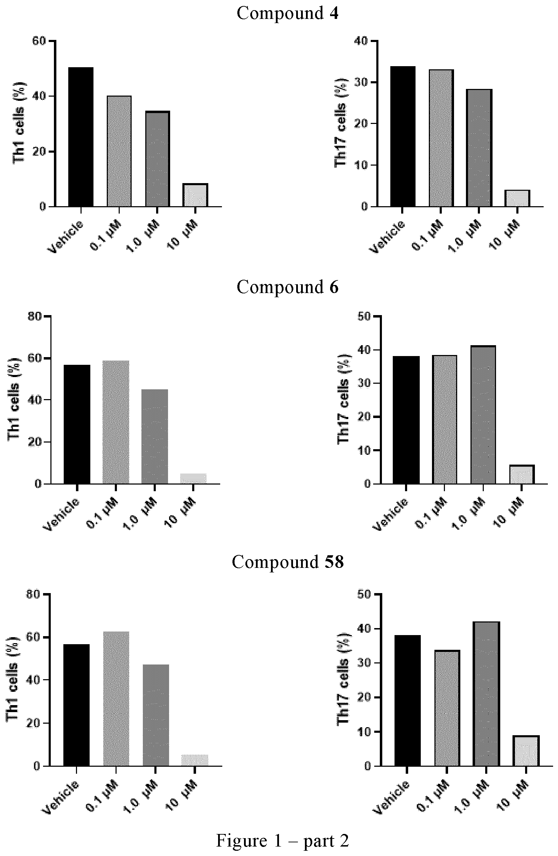

The results showed that compounds **1, 2, 3, 4,** 6, and **58** had a strong inhibitory effect on Th1/Th17 differentiation at the same time at a concentration of 10 µM. Compound **2** still had a strong inhibitory effect on Th1/Th17 differentiation at a lower concentration of 1 µM (Figure 1).

### 6) Anti-inflammatory tests in vivo and in vitro

### 6.1) Anti LPS-induced inflammation assay in Raw 264.7 cells

In this example, the effect of the compounds of the present invention on the inflammation level of the LPS-induced inflammation model of Raw 264.7 cells was analyzed by cell viability in combination with inflammatory factors; cell viability was analyzed by CCK-8 assay; and mRNA expression levels of inflammation-related indicators were detected by a real-time quantitative PCR (Q-PCR) method. Raw 264.7 cells were purchased from ATCC; CCK-8 kit was purchased from Meilunbio, item no. MA0218-3; LPS was purchased from Sigma, item no. L2630.

### 6.1.1) Effect of compounds on cell viability in LPS-induced inflammation model of Raw 264.7 cells

Experimental methods: first, 8000 cells/well Raw 264.7 cells were placed in 96-well plates and incubated overnight. On the second day, 0.1% DMSO was added to the control cells, 1 µg/ml LPS as well as 0.1% DMSO was added to the model group, 1 µg/ml LPS as well as 1 µM of the compounds (oxyacanthine hydrochloride (oxy), **1, 2,** tetrandrine (tet), **3, 4, 6, 58)** was added to the treatment group, and the 96-well plates were sterilized and incubated in a constant temperature incubator for 16 hours. Finally, the viability of the cells was detected using the CCK-8 kit.

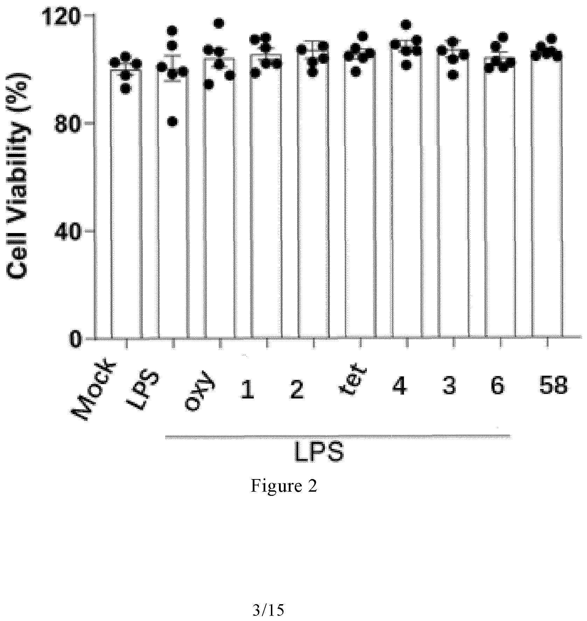

As shown in Figure 2, no damage was observed in all cells under the LPS-induced inflammation model, and there was no difference in cell viability between the treatment group and model group, indicating that the tested compounds did not affect cell viability of the cells in LPS-induced inflammation model.

### 6.1.2) Detection of compounds on mRNA levels of related inflammatory indicators in the LPS-induced inflammation model of Raw264.7 cells

Experimental methods: first, 5*10⁴ cells/well Raw 264.7 cells were placed in 12-well plates and incubated overnight. On the second day, 0.1% DMSO was added to the control cells, 1 µg/ml LPS as well as 0.1% DMSO was added to the model group, 1 µg/ml LPS as well as 1 µM of the compounds (oxyacanthine hydrochloride (oxy), **1, 2,** tetrandrine (tet), **3, 4, 6, 58)** was added to the treatment group, and the 12-well plates were sterilized and incubated in a constant temperature incubator for 16 hours. Cellular mRNA was extracted by lysis using Trizol (Yisheng, 10606ES60) and detected by real-time quantitative PCR after reverse transcription.

**Table 2. Primer sequences for target genes**

| **Primer Name** | **FWD sequence** | **REV sequence** |
|---|---|---|
| IL1β | GAAGTTGACGGACCCCAAAA | CCACAGCCACAATGAGTGATAC |
| IL-6 | AGTTGCCTTCTTGGGACTGA | TCCACGATTTCCCAGAGAAC |
| CXCL9 | GAACGGAGATCAAACCTGCC | CGACGACTTTGGGGTGTTTT |
| GAPDH | AACTTTGGCATTGTGGAAGG | ACACATTGGGGGTAGGAACA |

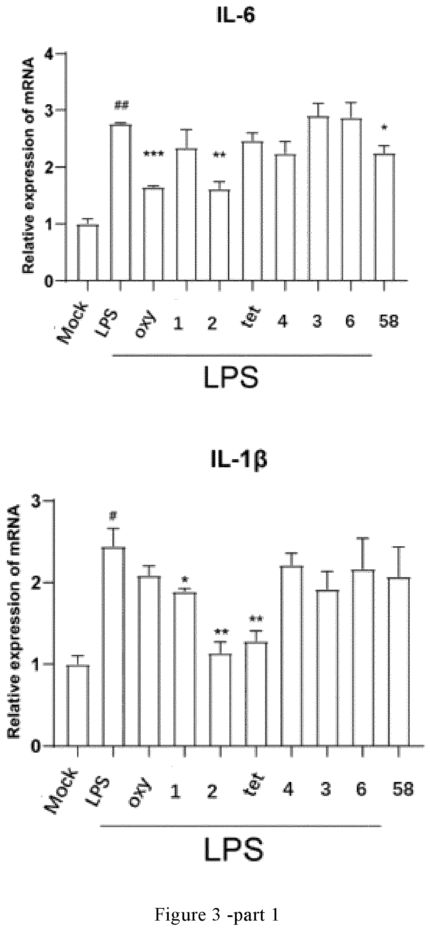

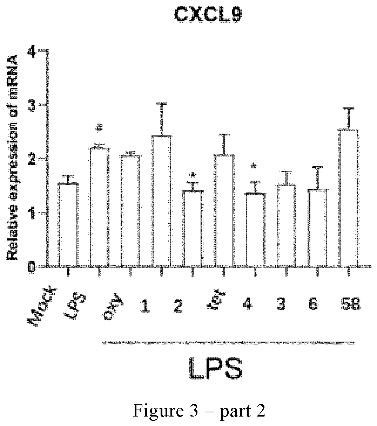

As shown in Figure 3, the cytokines IL-6 and IL-1β and the chemokine CXCL9 increased significantly under 1 µg/ml LPS induction, indicating that the inflammation model was effective; administration of different compounds (1 µM) resulted in a decrease of inflammatory and chemokine factors compared to the model group, in which the compound oxy, **1, 2,** tet, **4,** and **58** showed significant inhibition against different inflammatory factors, suggesting that the above compounds have strong anti-inflammatory activity and the result is not achieved by affecting cell viability.

### 6.2) Anti TNF-α induced inflammation assay in Raw 264.7 cells

In this embodiment, the effect of compounds on TNF-α-induced inflammation levels in Raw 264.7 cells was analyzed by cell viability combined with inflammatory factor expression levels; cell viability was analyzed by CCK-8 assay; and mRNA expression levels of inflammation-associated indicators were detected by real-time quantitative PCR (Q-PCR) method. Raw 264.7 cells were purchased from ATCC, CCK-8 kit was purchased from Meilunbio, item number MA0218-3, TNF-α was purchased from Merck, item no. GF314.

### 6.2.1) Effect of compounds on cell viability in TNF-α induced inflammation model of Raw 264.7 cells

Experimental methods: first, 8000 cells/well Raw 264.7 cells were placed in 96-well plates and incubated overnight. On the second day, 0.1% DMSO was added to the control cells, 10 ng/ml TNF-α as well as 0.1% DMSO was added to the model group, 10 ng/ml TNF-α as well as 1 µM of the compounds **(oxyacanthine hydrochloride (oxy), 1, 2, tetrandrine (tet), 3, 4, 6, 58)** was added to the treatment group, and the 96-well plates were sterilized and incubated in a constant temperature incubator for 16 hours. Finally, the viability of the cells was detected using the CCK-8 kit.

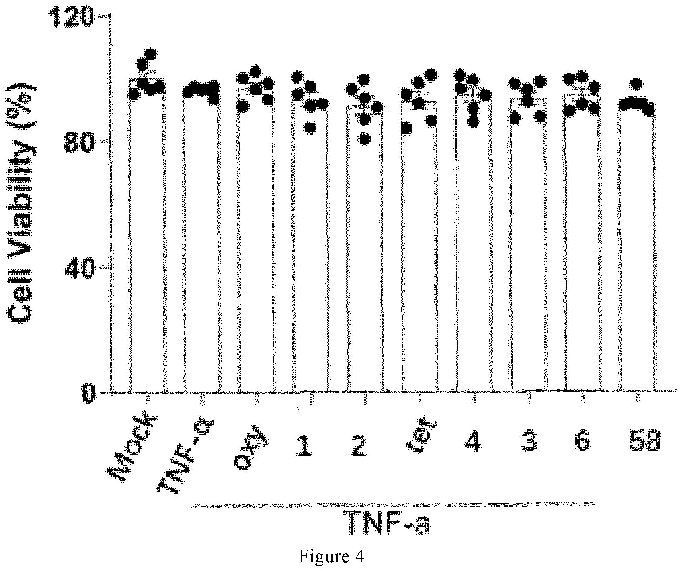

As shown in Figure 4, no damage was observed in all cells under the TNF-α-induced inflammation model, and there was no difference in cell viability between the treatment group and model group, indicating that the tested compounds did not affect cell viability of the cells in TNF-α-induced inflammation model.

### 6.2.2) Detection of compounds on mRNA levels of related inflammatory indicators in the TNF-α-induced inflammation model of Raw264.7 cells

Experimental methods: first, 5*10⁴ cells/well Raw 264.7 cells were placed in 12-well plates and incubated overnight. On the second day, 0.1% DMSO was added to the control cells, 10 ng/ml TNF-α as well as 0.1% DMSO was added to the model group, 10 ng/ml TNF-α as well as 1 µM of the compounds **(oxyacanthine hydrochloride (oxy), 1, 2, tetrandrine (tet), 3, 4, 6, 58)** was added to the treatment group, and the 12-well plates were sterilized and incubated in a constant temperature incubator for 16 hours. Cellular mRNA was extracted by lysis using Trizol (Yisheng, 10606ES60) and detected by real-time quantitative PCR after reverse transcription, the primers are the same as in Table 2.

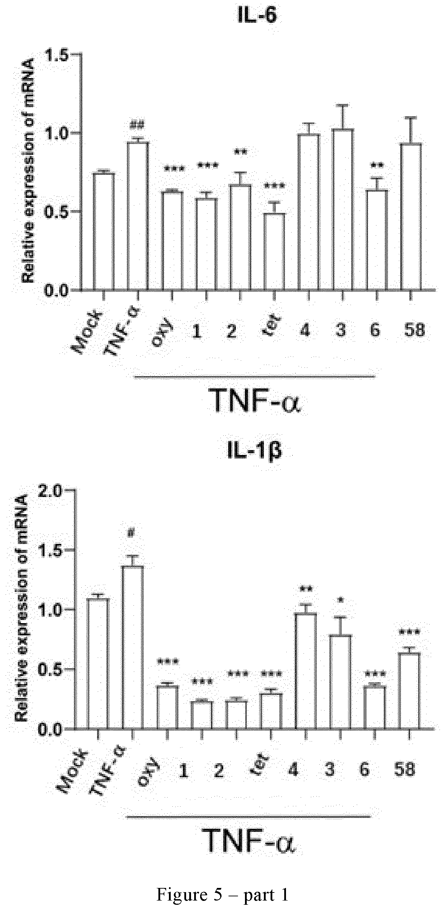

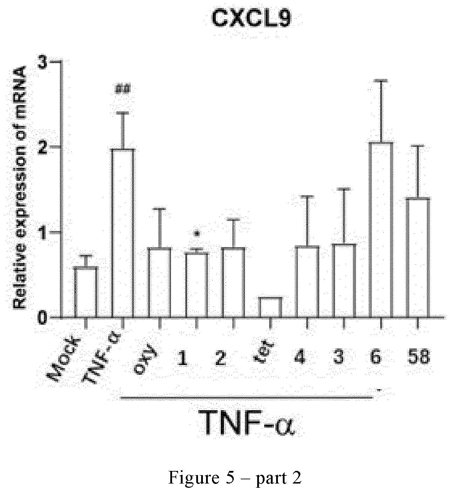

As shown in Figure 5, the cytokines IL-6 and IL-1β and the chemokine CXCL9 increased significantly under 10 ng/ml TNF-α induction, indicating that the inflammation model was effective; administration of different compounds (1 µM) resulted in a decrease of inflammatory and chemokine factors compared to the model group, in which the compound **oxy, 1, 2, tet, 3, 4, 6 and 58** showed significant inhibition against different inflammatory factors, suggesting that the above compounds have strong anti-inflammatory activity and the result is not achieved by affecting cell viability.

### 6.3) In vivo experiments against LPS-induced acute inflammation

In this example, the effects of compounds on LPS-induced acute inflammation model in vivo were analyzed by the expression levels of inflammatory factors; the mRNA expression levels of tissue inflammation-related indicators were detected by the method of real-time quantitative PCR (Q-PCR), and the levels of inflammatory factors in serum were detected by the method of Elisa. C57BL/6 mice were purchased from SLAC, and the Elisa kits were purchased from Novusbio, with item no.890860for IL-6 and item no. 266117 for TNF-α.

### Experimental Methods:

Solution preparation: LPS solution was prepared into1 mg/ml stock solution with PBS and filtered by 0.22 µm aqueous filter head; the compound was diluted to 3 mg/ml with 85% saline+5% DMSO+5% HS-15+5% PEG400 as solvent.

Construction of inflammation model in mice: C57BL/6 mice were randomly divided into three groups: one control group, five experimental animals; one LPS modeling group, with five experimental animals; and two treatment groups, with five experimental animals. The control group was injected intraperitoneally with 0.1 ml/10 g of PBS, and both the model group and the treatment groups were injected intraperitoneally with 3 mg/kg of LPS, and the drug was administered two hours later; 0.1 ml/10 g of solvent was given orally to the control and model groups, and 30 mg/kg of compound **4** was given orally to the treatment group. The blood was collected from the orbit of mice in each group at 4 h after the administration of the drug, and the spleen and lung tissues were collected after the euthanasia. mRNA was extracted from lung and spleen tissues respectively, and real-time quantitative PCR was performed after reverse transcription to assess the mRNA expression level of inflammation-related indicators, and Elisa assay was performed after serum extraction to assess the secretion level of inflammatory factors.

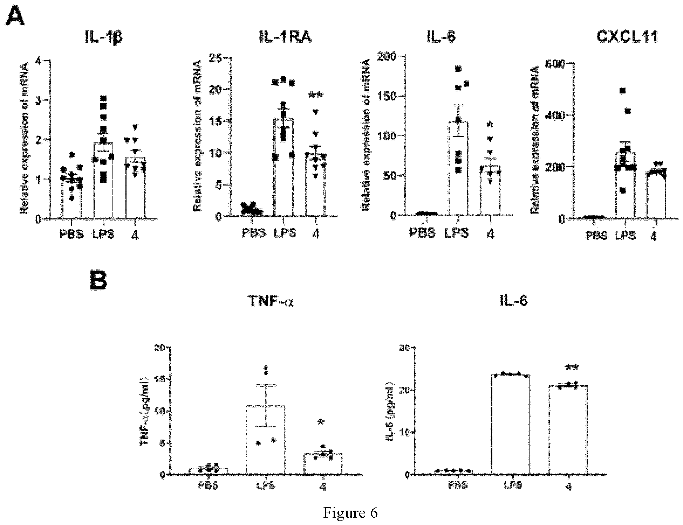

The experimental results are shown in Figure 6, 3 mg/kg LPS stimulation resulted in a significant increase in cytokines and chemokines, indicating that the in vivo inflammation model was effective; when compound **4** was given orally at 30 mg/kg, inflammatory factors and chemokines showed a significant decrease in mRNA levels and cytokine secretion levels compared to the model group. This indicates that compound **4** has anti-inflammatory activity in vivo.

### 7) Lung injury protective activity test

In this example, cell viability combined with cell death-associated molecules were used to analyze the effect of compounds on the level of TNF-α-induced injury in A549 lung epithelial cells. Cell injury was observed by microscopy; cell viability was analyzed by CCK-8 assay; and mRNA expression levels of injury-related indicators were detected by real-time quantitative PCR (Q-PCR) method. A549 cells were purchased from ATCC, CCK-8 kit was purchased from Meilunbio, item no. MA0218-3, and LPS was purchased from Sigma, item no. L2630.

### 7.1) Effect of compounds on TNF-α induced activity in A549 cells

Experimental methods: first, 8000 cells/well A549 cells were placed in 96-well plates and incubated overnight. On the second day, 0.1% DMSO was added to the control cells, 10 ng/ml TNF-α as well as 0.1% DMSO was added to the model group, 10 ng/ml TNF-α as well as 1 µM of the compounds **(oxy, 1, 2, tet, 3, 4, 6, 58)** was added to the treatment group, and the 96-well plates were sterilized and incubated in a constant temperature incubator for 24 hours. Finally, the viability of the cells was detected using the CCK-8 kit.

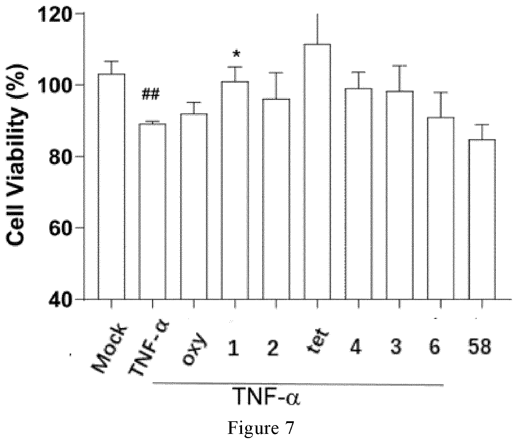

As shown in Figure 7, under the TNF-α-induced lung injury model, the cell viability of the model group was affected and the lung epithelial cells tended to be in a dead state. By comparing the treatment group and the model group, some of the compounds could alleviate the lung epithelial cell injury mediated by TNF-α, with Compound 1 showing a significant difference.

### 7.2) Detection of compounds on mRNA levels of indicators related to TNF-α-induced injury in A549 cells

Experimental methods: first, 5*10⁴ cells/well A549 cells were placed in 12-well plates and incubated overnight. On the second day, 0.1% DMSO was added to the control cells, 10 ng/ml TNF-α as well as 0.1% DMSO was added to the model group, 10 ng/ml TNF-α as well as 1 µM of the compounds **(oxy, 1, 2, tet, 3, 4, 6, 58)** was added to the treatment group, and the 12-well plates were sterilized and incubated in a constant temperature incubator for 24 hours. Cellular mRNA was extracted by lysis using Trizol (Yisheng, 10606ES60) and detected by real-time quantitative PCR after reverse transcription.

**Table 3. Primer sequences for target genes**

| **Primer Name** | **FWD sequence** | **REV sequence** |
|---|---|---|
| TNF-α | GCTGCACTTTGGAGTGATCG | TCACTCGGGGTTCGAGAAGA |
| IL1β | GGAGAATGACCTGAGCACCT | GGAGGTGGAGAGCTTTCAGT |
| GAPDH | AGGTCGGAGTCAACGGATTT | ATCGCCCCACTTGATTTTGG |

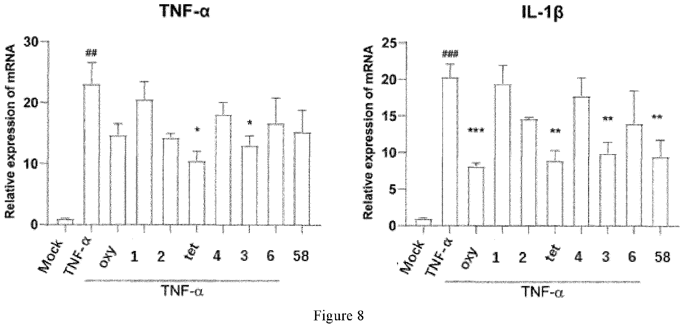

The experimental results are shown in Figure 8, under 10 ng/ml TNF-α induction, a significant increase in lung epithelial cell injury, TNF-α and IL-1β was observed, indicating that the inflammation model was effective; after administration of the different compounds (1 µM), the injury factors decreased compared to the model group, wherein the compounds **oxy, tet, 3, and 58** significantly inhibited different injury factors. It indicates that the compounds of the present invention have protective activity after lung epithelial cell injury, in which **oxy, tet, 3 and 58** have stronger protective activity.

### 8) Anti H₂O₂-induced necrotic injury assay in ALM12 hepatocytes

In this example, the effect of the compounds of the present invention on the level of necrotic injury induced by H₂O₂ in ALM12 hepatocytes was analyzed by microscopic morphological observations, cell viability in combination with cell death-associated molecules. Cell viability was analyzed by CCK-8 assay.ALM12 cells were cultured in 1640 medium (gibco, c11995500BT) + 10% FBS (gibco, 10099141C) in a constant temperature incubator at 37° C, 5% CO₂; the CCK-8 kit was purchased from Meilunbio, item no. MA0218-3; H₂O₂ was purchased from Sinopharm Chemical Reagent Co. item no. 10011208.

Experimental methods: first, 8000 cells/well ALM12 cells were placed in 96-well plates and incubated overnight. On the second day, 0.1% DMSO was added to the control cells, 2 mM H₂O₂ as well as 0.1% DMSO was added to the model group, 2 mM H₂O₂ as well as 1 µM of the compounds **(oxy, 1, 2, tet, 3, 4, 6, 58)** was added to the treatment group, and the 96-well plates were sterilized and incubated in a constant temperature incubator for 3 hours. Finally, cell injury and growth were observed using a microscope; the viability of the cells was detected by CCK-8 kit.

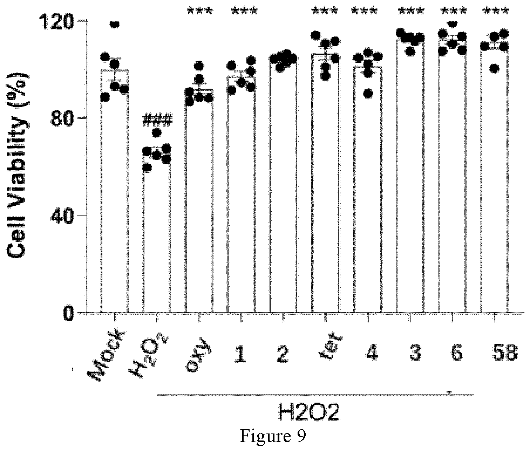

The experimental results are shown in Figure 9, under the H₂O₂-induced liver injury model, the cell viability of the model group was affected and the liver parenchymal cells tended to be in a state of death with a significant difference; the compounds of the present invention were observed to significantly alleviate the hepatocyte injury mediated by H₂O₂ under the microscope when comparing the treatment group and the model group, and all compounds significantly protected the hepatocytes from death as can be concluded from the cell viability assay.

### 9) AntiTGF-β1-induced fibrosis assay in A549 lung epithelial cells

In this example, the mitigative effect of the compounds of the present invention on the fibrosis of A549 cells was analyzed by microscopic observation, CCK-8 kit combined with WesternBlot assay. Cell injury was observed by microscopic observation; cell viability was analyzed by CCK-8 assay; and protein expression levels of fibrosis-related indicators were detected by Western Blot method. A549 cells were purchased from ATCC; CCK-8 kit was purchased from Meilunbio, item number MA0218-3; TGF-β1 was purchased from MCE, item number HY- P70543; primary antibodies: Collagen was purchased from ARG21965, item no. arigobio; α-smooth muscle actin was purchased from CST, item no. mAb#19245; VIMENTN was purchased from CST, item no. mAb#5741; GAPDH was purchased from Yeasen, item no. 30201ES20.

Experimental methods: first, 8000 cells/well A549 cells were placed in 96-well plates and incubated overnight. On the second day, 0.1% DMSO was added to the control cells, 10 ng/ml TGF-β1 as well as 0.1% DMSO was added to the model group, 10 ng/ml TGF-β1 as well as 1 µM of the compounds **(oxy, 1, 2, tet, 3, 4, 6, 58)** was added to the treatment group, and the 96-well plates were sterilized and incubated in a constant temperature incubator for 72 hours. Finally, cell injury and growth were observed using a microscope; the viability of the cells was detected by CCK-8 kit.

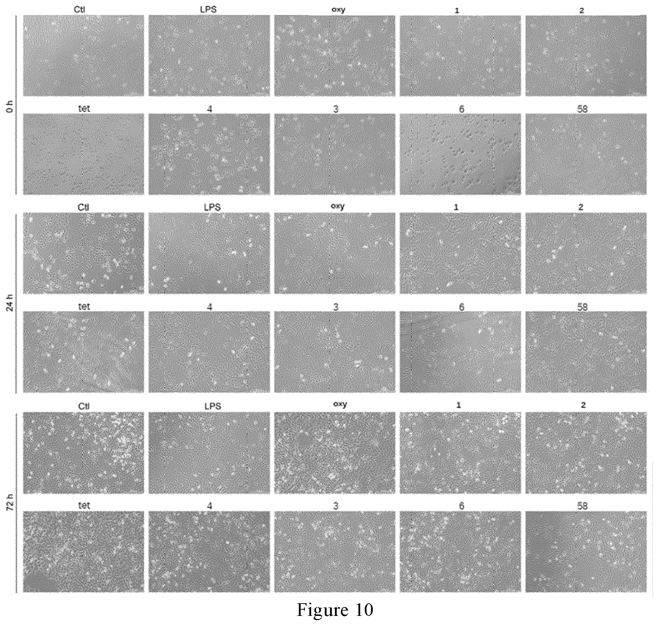

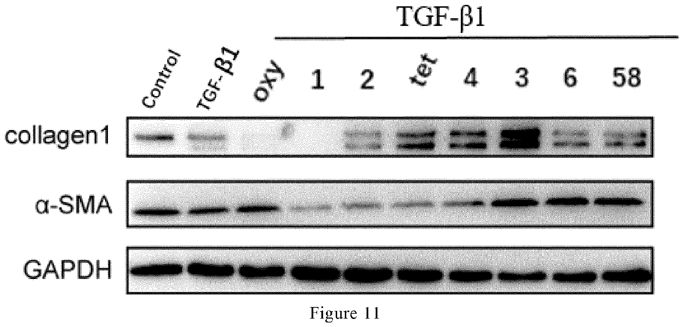

The experimental results are shown in Figure 10 and Figure 11, after adding 10 ng/ml TGF-β1 to stimulate A549 cells, microscopic observation was carried out at 24 h and 72 h. At 24 h, it can be seen that the A549 cells have undergone deformation, changing from elliptical shape into elongated shape, indicating that the lung epithelial cells have undergone fibroblast-like changes, and the changes in the treatment group were not obvious under the microscopic observation; the fibroblast indicators were detected by WB assay, and it can be seen that there was upregulations of Collagen1 and α-SMA in the model group, indicating that the model was effective. By comparing the treatment group with the model group, it was seen that compounds **oxy, 1, 2, tet and 4** could partially alleviate fibrosis, indicating that compounds **oxy, 1, 2, tet and 4** could partially alleviate fibrosis indicators, and had a certain protective effect.

### 10) Animal experiments of anti-carbon tetrachloride-induced acute liver fibrosis

In this example, the effect of the compounds of the present invention on the carbon tetrachloride-induced acute liver fibrosis model was analyzed by tissue observation, fibrosis indicators, HE and Sirius red staining; the injury of mice was determined by body weight testing and tissue bright-field photographing; the protein expression levels of fibrosis-related indicators were detected by WesternBlot assay; and the fibrosis progression was analyzed by HE and Sirius red staining. C57BL/6 mice were purchased from SLAC; CC14 was purchased from Sinopharm Chemical Reagent Co, Ltd, item no. 10006464; olive oil was purchased from Sinopharm, item no. 69018028; primary antibody:Collagen was purchased from ARG21965, item no. arigobio; α-smooth muscle actin was purchased from CST, item no. mAb#19245; VIMENTN was purchased from CST, item no. mAb#5741; GAPDH was purchased from Yeasen, item no. mAb#30201ES20.

### Experimental Methods:

Solution preparation: CC14 was diluted with olive oil (1:9, vol:vol) and the compounds were configured with 85% saline + 5% DMSO + 5% HS-15 + 5% PEG400 as solvents toa concentration of 3 mg/ml.

Construction of fibrosis model mice: 32 C57 mice were randomly divided into 3 groups after 1 week of adaptive feeding: blank group, model group, and 2 treatment groups; with 6 experimental animals in the blank group and 8 experimental animals in each of the remaining 3 groups. Except for the blank group, the remaining three groups were injected intraperitoneally with CCl₄ (0.6 ml/kg) in D1, D3, and D5, respectively, and the blank group was injected intraperitoneally with olive oil at the same time. At the same time of modeling, mice in the treatment group were continuously administered by gavage for 6 days, once a day, 2 h after the injection of CCl₄, and the treatment group was administered orally30 mg/kg of Compound **2** or Compound **4;** mice in the blank group and the model group were given an equal amount of saline by intraperitoneal injection, and the mice were euthanized 24 h after the last administration of CCl₄ for test. A portion of the liver tissue was cut to appropriate size and placed in 4% PFA, -80° C refrigerator for subsequent detection of pathological changes in tissue sample slices, and WB assay, respectively.

### Detection methods: 1. After autopsy, liver in situ photos were taken; 2. Liver tissue was retained and lysed using RIPA to extract proteins for WB detection; 3. HE staining as well as Sirius red detection were performed when liver tissue sections were fixed.

### Results:

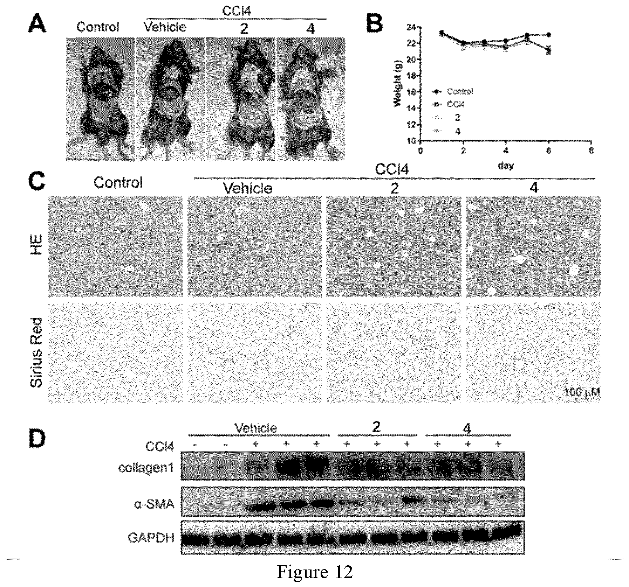

a. No animals died until the end of the experiment, and animals lost weight on day 6 after modeling (Figure 12B);
b. After euthanasia of the experimental animals, the injury state of the liver organs of the mice was observed in the brightfield, and the fibrotic lesions on the surface of the livers of the mice in the model group could be clearly seen, and the degree of coarse roughness on the surface of the liver organs of the mice in the treatment group was alleviated (Figure 12A);
c. HE staining showed obvious formation of hepatic pseudo-lobules and densification of hepatic parenchyma cells in mice in the modeling group, indicating successful modeling, which was alleviated in the treatment group; Sirius red staining revealed an increase in fibrous tissue in the modeling group, which was reduced in the treatment group (Figure 12C);
d. The liver fibrosis indicators were detected by WB assay, and the fibrosis indicators Collagen1 and α-SMA were significantly up-regulated in the model group, which indicated that the model construction was successful; the liver fibrosis indicators were found to be down-regulated after the administration of 30 mg/kg of Compound **2** or Compound **4** (Figure 12D);

The above experimental results indicate that oral administration of Compound **2** or Compound **4** has a mitigative effect on acute hepatic fibrosis injury.

In summary, in the present invention, the present inventors have disclosed, for the first time, a novel class of cyclic bisbenzyl isoquinoline alkaloids or pharmaceutically acceptable salts thereof, preparation method therefor, and have found that they have a significant inhibitory effect on SARS-CoV-2 replication, with an inhibitory EC₅₀ reaching single digit micromolar levels or less. Thus, the cyclic bisbenzyl isoquinoline alkaloids or pharmaceutically acceptable salts thereof described in the present invention have excellent anti-novel coronavirus (SARS-CoV-2) activity, and have good prospects for clinical application. In addition, the cyclic bisbenzyl isoquinoline alkaloids described herein or pharmaceutically acceptable salts thereof have anti-inflammatory, anti-fibrotic, and anti-Th1 and Th17 cell differentiation effects, and are expected to be used for the prevention and alleviation of diseases associated with viral infection, inflammation-related diseases (pneumonia, non-alcoholic steatohepatitis, colitis, nephritis, pancreatitis, myocarditis, arthritis, inflammatory pain), fibrosis-related diseases (pulmonary fibrosis, silicosis, hepatic fibrosis, renal fibrosis, myocardial fibrosis, dermatofibrosis, retinal fibrosis, myelofibrosis)), Th1 and/or Th17 abnormal differentiation-related autoimmune diseases (psoriasis, rheumatoid arthritis, systemic lupus erythematosus, multiple sclerosis, neuromyelitisoptica, myasthenia gravis, ankylosing spondylitis, ulcerative colitis, Crohn's disease, delayed-type hypersensitivity, graft-versus-host disease, etc.), osteoporosis, and neurodegenerative diseases.

All documents referred to in the present invention are incorporated by reference herein as if each document is individually incorporated by reference. Further, it should be understood that upon reading the above teachings of the present invention, various modifications or alternations may be made to the present invention by those skilled in the art, and those equivalents also fall within the scope defined by the appended claims of the present application.

## Claims

1. A cyclic bisbenzyl tetrahydroisoquinoline compound of Formula I, or a pharmaceutically acceptable salt, an enantiomer, a diastereoisomer, a racemate, a crystalline hydrate, a solvate thereof, or a mixture thereof, 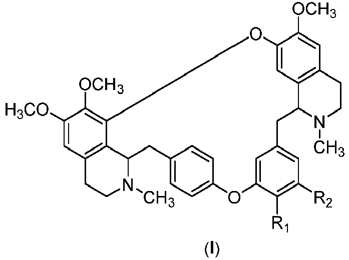 wherein,
R₁ and R₂ are each independently selected from: hydrogen, halogen, nitro, hydroxyl, thiol, C₁-C₆alkoxy, C₁-C₆haloalkoxy, hydroxyl C₁-C₆alkoxy, C₁-C₆alkylthiol, C₁-C₆ alkyl, C₁-C₆haloalkyl, amino C₁-C₆ alkyl, hydroxyl C₁-C₆ alkyl, cyano C₁-C₆ alkyl, C₃-C₈cycloalkyl, C₃-C₈cycloalkoxy, C₂-C₆ alkenyl, C₂-C₆ alkenyl carbonyl, C₂-C₆alkenoxy, C₂-C₆alkynyl, C₂-C₆alkynoxy, amino, C₁-C₆ alkyl-substituted amino, benzyl-substituted amino, C₁-C₆alkanoyl-substituted amino, C₂-C₆alkenoyl-substituted amino, cyano, C₁ -C₆ carboxyl, C₁ -C₆ aldehyde, C₁-C₆alkanoyl, C₃-C₆cycloalkyl acyl, C₁-C₆haloalkanoyl, sulfonamido, C₁-C₆ alkyl-substituted sulfonamido, carbamoyl, phenylcarbamoyl, N-methyl-N-methoxyamino, C₁-C₆ alkyl-substituted carbamoyl, C₃-C₆cycloalkyl-substituted carbamoyl, adamantylcarbamoyl, pyridinyl- or pyrimidinyl-substituted carbamoyl, hydroxy C₁-C₆alkoxy C₁-C₆ alkyl-substituted carbamoyl, C₁-C₆alkoxy C₁-C₆ alkyl-substituted carbamoyl, hydroxy C₁-C₆alkoxy-substituted C₁-C₆alkoxycarbonyl, phenoxycarbonyl, C₃-C₆cycloalkyl-substitutedhydroxymethyl, carboxyl C₁-C₆ alkyl, C₁-C₆alkylsulfonyl, C₁-C₆haloalkylsulfonyl, C₁-C₆ alkyl-substituted amino C₁-C₆ alkyl, C₁-C₂₀ alkyl-substituted carbamoyloxy, C₃-C₁₀cycloalkyl-substituted carbamoyloxy, C₁-C₆alkanoyl-substituted amino C₁-C₆ alkyl, C₁-C₆alkoxycarbonyl, carbamoyl C₁-C₆ alkyl, C₁-C₆ alkyl-substituted carbamoyl C₁-C₆ alkyl, C₂-C₆ alkenyl acyloxy (C₂-C₆ alkenyl-CO-O-), C₂-C₁₀ ester group, 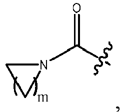 and -O-Z, wherein Z is 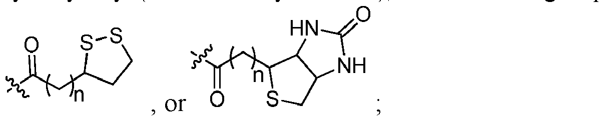
wherein n is 0, 1, 2, 3, or 4; and m is 1, 2, 3, or 4;
wherein the phenylcarbamoyl, and phenoxycarbonyl are optionally substituted by one or more substituents selected from F, Cl, Br, I, C₁-C₆ alkyl, C₁-C₆haloalkyl, C₁-C₆alkoxy and C₁-C₆haloalkoxy;
or R₁ and R₂ together with the adjacent benzene ring form a benzo[5 to 6-membered monocyclic heterocycle]which is unsubstituted or substituted by 1 to 4 substituents selected from halogen, hydroxyl, thiol, oxo-, thio-, C₁-C₆ alkyl, C₁-C₆alkoxy and cyano; and the heterocycle contains from 1 to 3 heteroatoms selected from N, O, and S;
with the proviso that the compound does not include the following compound:

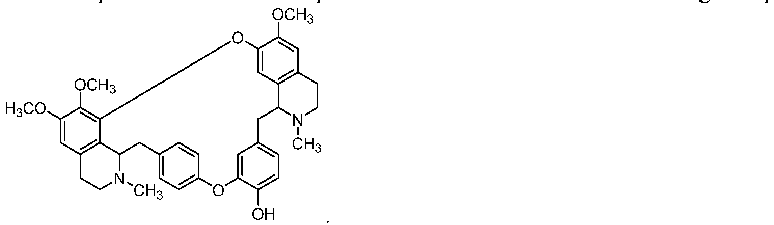

2. The compound according to claim 1, wherein,
R₁ and R₂ are each independently selected from: hydrogen, halogen, nitro, hydroxyl, thiol, C₁-C₄alkoxy, C₁-C₄haloalkoxy, hydroxyl C₁-C₄alkoxy, C₁-C₄alkylthiol, C₁-C₄ alkyl, C₁-C₄haloalkyl, amino C₁-C₄ alkyl, hydroxyl C₁-C₄ alkyl, cyanoC₁-C₄ alkyl, C₃-C₆cycloalkyl, C₃-C₆cycloalkoxy, C₂-C₅ alkenyl, C₂-C₅ alkenyl carbonyl, C₂-C₅alkenoxy, C₂-C₅alkynyl, C₂-C₅alkynoxy, amino, C₁-C₄alkyl-substituted amino, benzyl-substituted amino, C₁-C₄alkanoyl-substituted amino, C₂-C₅alkenoyl-substituted amino, cyano, C₁-C₄ carboxyl, C₁-C₄ aldehyde, C₁-C₄alkanoyl, C₃-C₅cycloalkyl acyl, C₁-C₄haloalkanoyl, sulfonamido (-SO₂NH₂), C₁-C₄ alkyl-substituted sulfonamido (-SO₂NH₂), carbamoyl(-CONH₂), phenylcarbamoyl, N-methyl-N-methoxyamino, C₁-C₄ alkyl-substituted carbamoyl, Cs-Cscycloalkyl-substituted carbamoyl, adamantylcarbamoyl, pyridinyl- or pyrimidinyl-substituted carbamoyl, hydroxyC₁-C₄alkoxyC₁-C₄ alkyl-substituted carbamoyl, C₁-C₄alkoxyC₁-C₄ alkyl-substituted carbamoyl, hydroxyC₁-C₄alkoxy-substituted C₁-C₄alkoxycarbonyl, phenoxycarbonyl, C₃-C₅cycloalkyl-substituted hydroxymethyl, carboxyl C₁-C₄ alkyl, C₁-C₄alkylsulfonyl, C₁-C₄haloalkylsulfonyl, C₁-C₄ alkyl-substituted amino C₁-C₄ alkyl, C₁-C₁₀ alkyl-substituted carbamoyloxy, C₃-C₈cycloalkyl-substituted carbamoyloxy, C₁-C₄alkanoyl-substituted amino C₁-C₄ alkyl, C₁-C₄alkoxycarbonyl, carbamoyl C₁-C₄ alkyl, C₁-C₄ alkyl-substituted carbamoyl C₁-C₄ alkyl, C₂-C₅ alkenyl acyloxy (C₂-C₅ alkenyl-CO-O-), C₂-C₆ ester group, 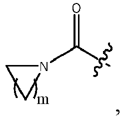 and -O-Z, wherein Z is 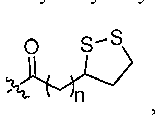 or 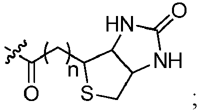
or R₁ and R₂ together with the adjacent benzene ring form a benzo[5 to 6-membered monocyclic heterocycle] which is unsubstituted or substituted by 1 to 2 substituents selected from halogen, hydroxyl, thiol, oxo- (=O), thio- (=S), C₁-C₆ alkyl, C₁-C₆alkoxy and cyano; and the heterocycle contains from 1 to 3 heteroatoms selected from N, O, and S.

3. The compound according to claim 1, wherein,
R₁ and R₂ are each independently selected from: hydrogen, fluorine, chlorine, bromine, nitro, hydroxyl, thiol, methoxy, ethoxy, trifluoromethoxy, -SCH₃, -SCH₂CH₃, propyl, cyclopropyl, isopropyl, tert-butyl, trifluoromethyl, difluoromethoxy, bromomethyl, chloromethyl, vinyl, vinylmethyl, amino, N-methylamino, N-ethyl amino, N,N-dimethylamino, N,N-diethylamino, cyano, carboxyl, aldehyde, -CH₂NH₂, -CH₂CH₂NH₂, -CH₂OH, -CH₂CH₂OH, -CH₂CN, -CH₂CH₂CN, formyl, acetyl, propionyl, trifluoroacetyl, sulfonamido, carbamoyl, N-methylcarbamoyl, N,N-dimethylcarbamoyl, N-ethylcarbamoyl, N,N-diethylcarbamoyl, -O(C=O)NH(C4 alkyl), cyclopentylcarbamoyloxy, cyclohexylcarbamoyloxy, -CH₂CO₂H, -CH₂CH₂CO₂H, -SO₂CH₃, -SO₂CF₃, -CH₂NHMe, -CH₂NMe₂, -CH₂CONH₂, -CONH₂, -NHCOCH₃, -CH₂NHCOCH₃, -(C=O)OCH₂CH₂OCH₂CH₂OH, -(C=O)OCH₃, -CH₂CONHMe, -CONHMe, -CONH(cyclopropyl), -CH₂CONMe₂, and -O-Z, wherein Z is 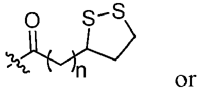 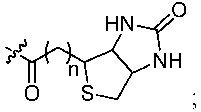 n is an integer from 0 to 4;
or R₁ and R₂ together with the adjacent benzene ring form a benzo[5 to 6-membered monocyclic heterocycle] which is unsubstituted or substituted by 1 to 2 substituents selected from halogen, hydroxyl, thiol, oxo-, thio-, and C₁-C₆ alkyl; and the heterocycle contains from 1 to 3 heteroatoms selected from N, O, and S.

4. The compound according to claim 1, wherein the compound has a structure of formula I-a: 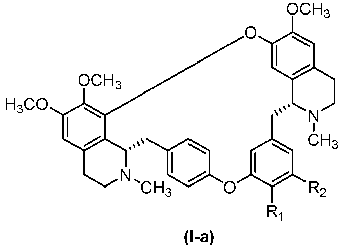 wherein R₁ and R₂ are as defined in claim 1.

5. The compound of claim 1, wherein the compound of formula I is selected from the following compound:

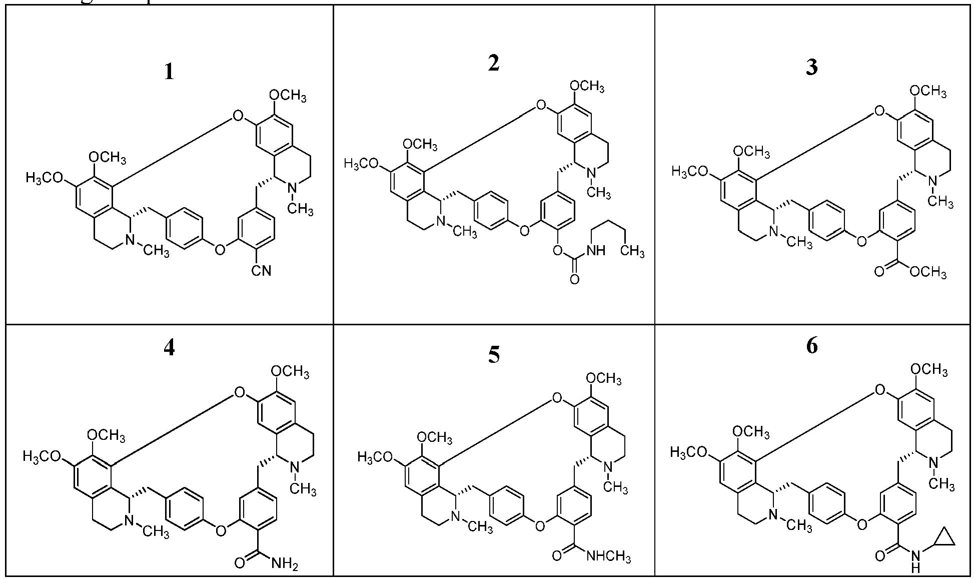

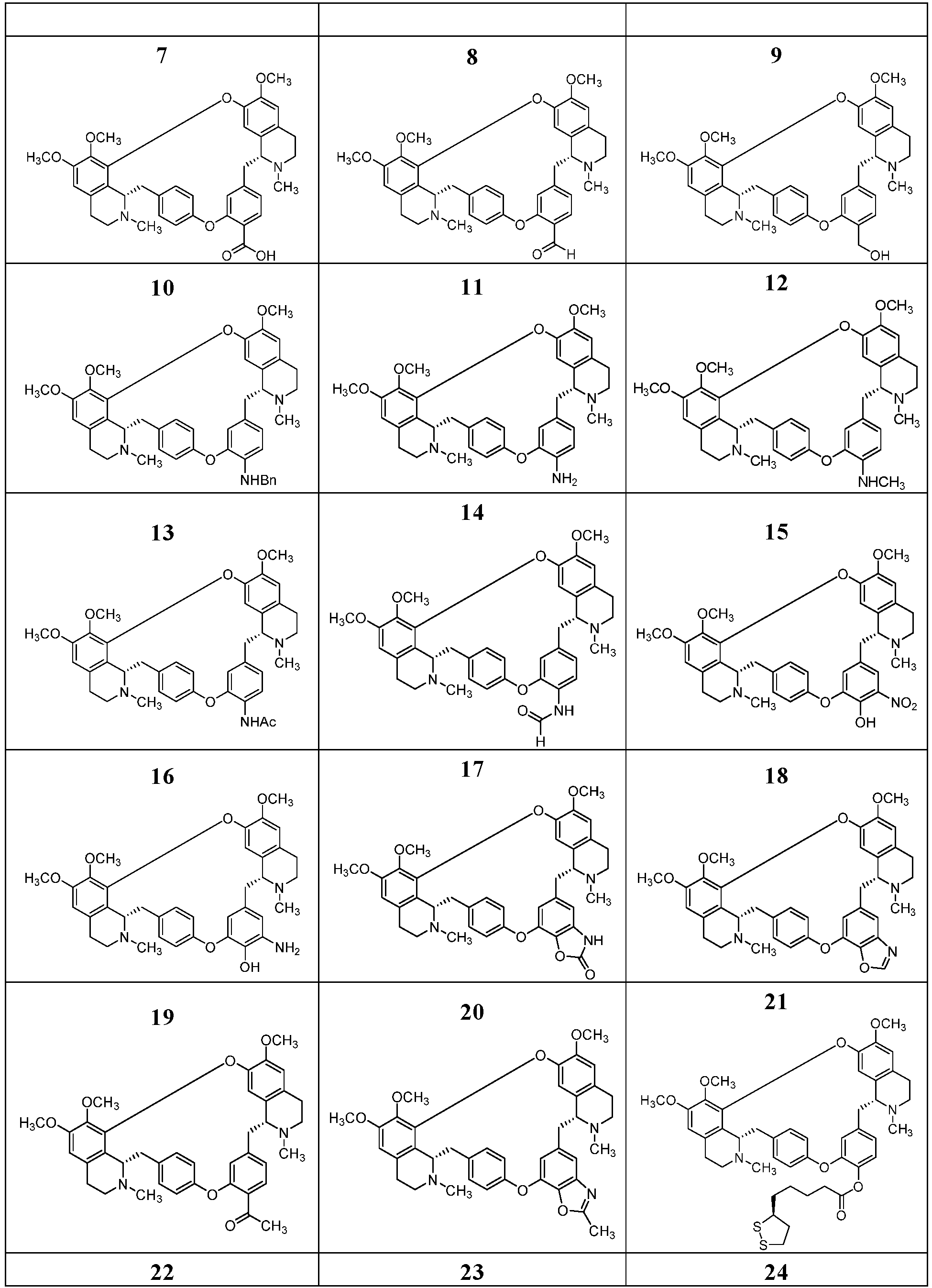

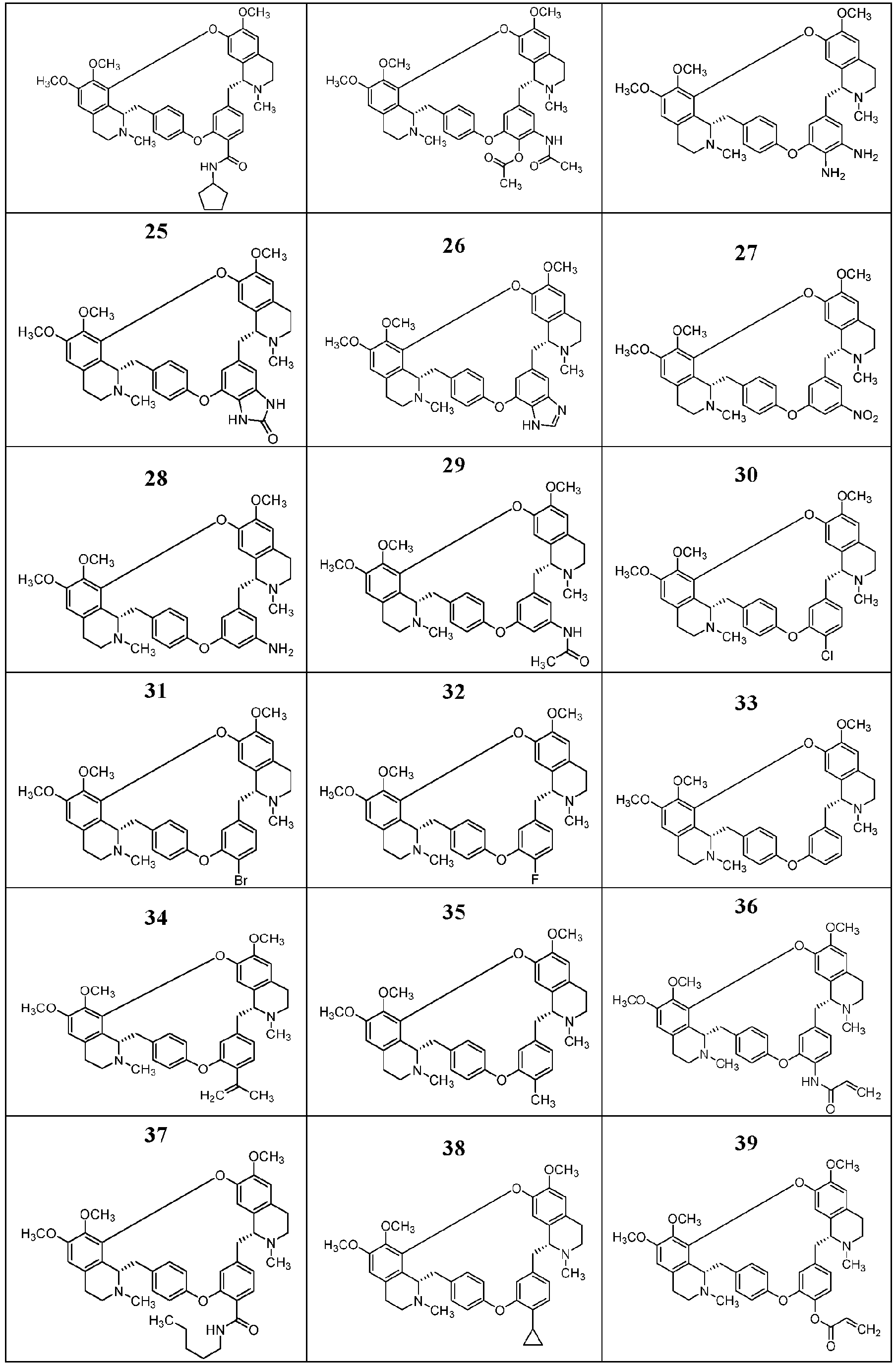

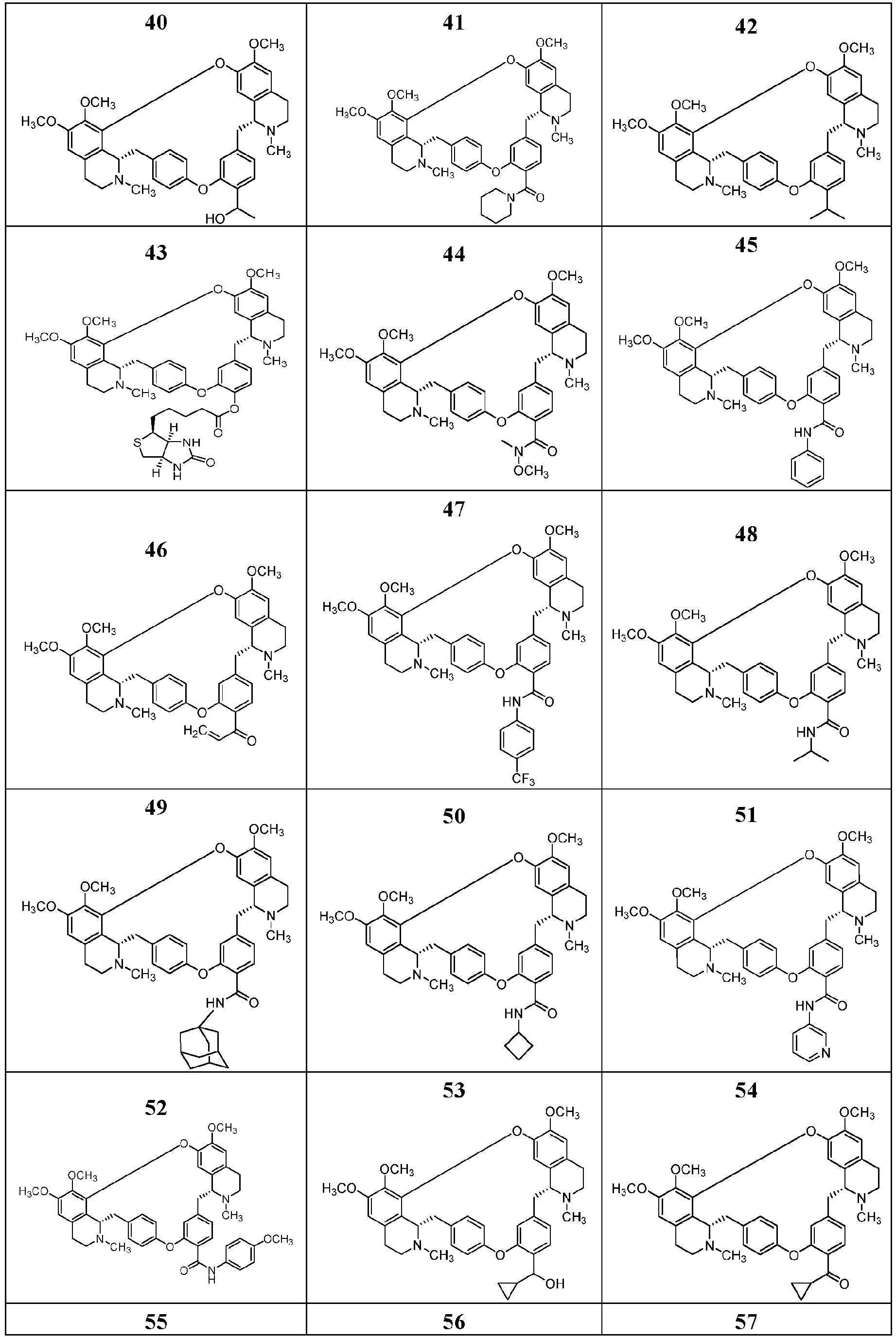

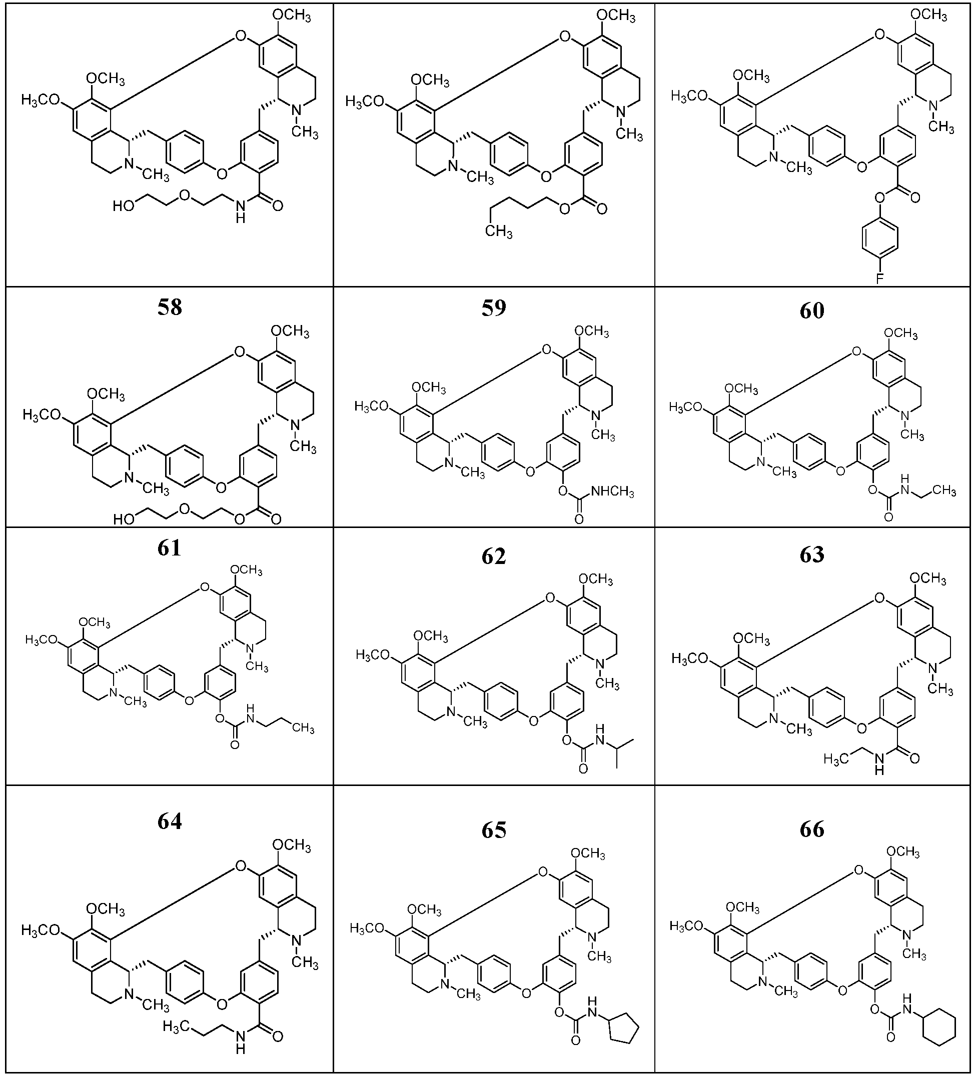

6. The compound of claim 1, wherein the compound of formula I is selected from the following compound:

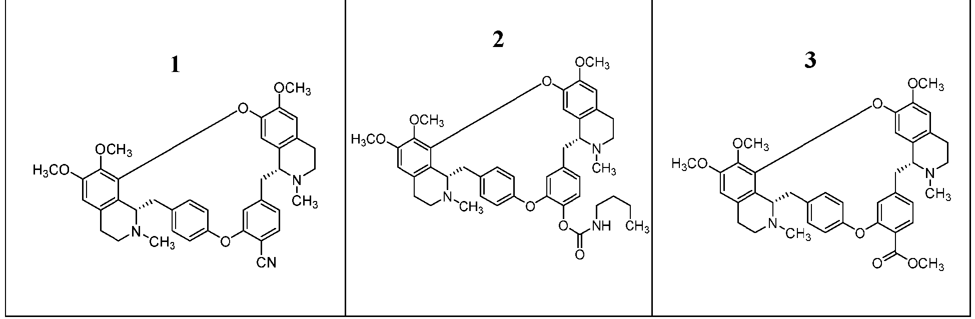

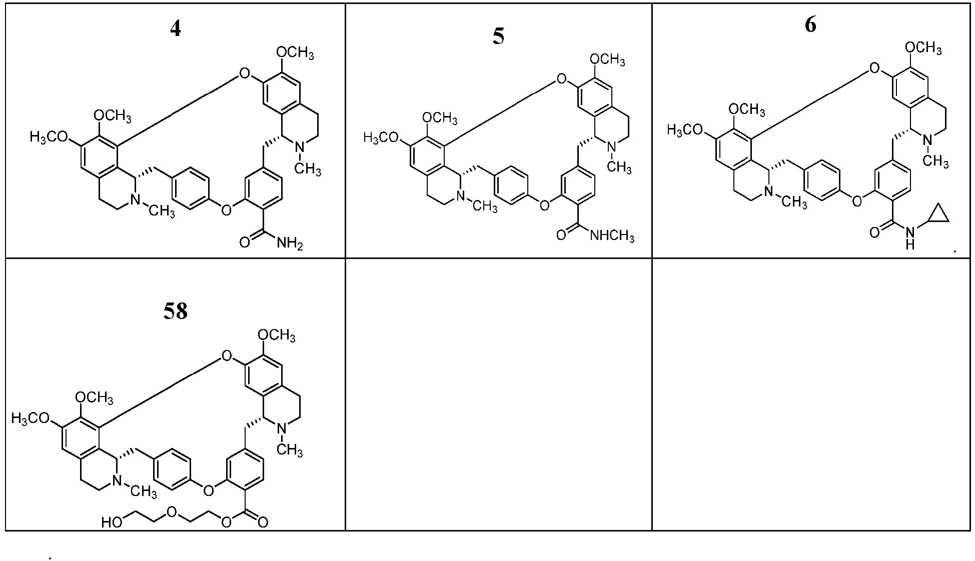

7. A method for preparing cyclic bisbenzyl tetrahydroisoquinoline compound according to claim 1, wherein the method is selected from the group consisting of:
a) Cyclic bisbenzyl tetrahydroisoquinoline containing phenolic hydroxyl group is taken as a raw material, and subjected to an alkylation reaction with alkylation reagent to obtain the cyclic bisbenzyl tetrahydroisoquinoline compound;
b) Cyclic bisbenzyl tetrahydroisoquinoline containing phenolic hydroxyl group is taken as a raw material, and subjected to an acylation reaction with acylation reagent to obtain the cyclic bisbenzyl tetrahydroisoquinoline compound;
c) Oxyacanthine is taken as a raw material, and reacts with sulfonylation reagent in the presence of base to obtain compound (I-1b);
Compound (I-1b) and a coupling reagent undergo a coupling reaction to obtain the cyclic bisbenzyl tetrahydroisoquinoline compound 1-1 of formula 1-1, and the reaction is as shown in reaction formula 1: 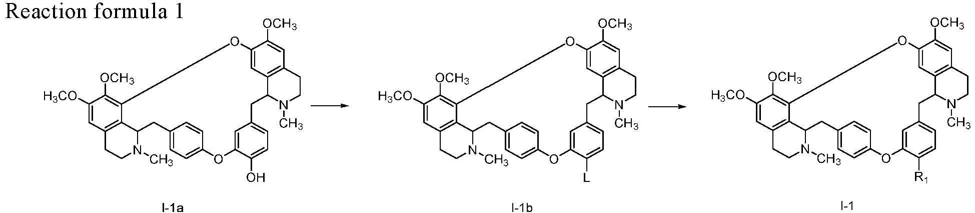
in Formula 1-1, R₁ is selected from hydrogen, halogen, C₁-C₆alkylthiol, C₁-C₆ alkyl, C₃-C₆cycloalkyl, C₂-C₆ alkenyl, C₂-C₆alkynyl, C₁-C₆ alkyl-substituted amino, benzyl-substituted amino, cyano, carboxyl, aldehyde and C₁-C₆alkanoyl;
L in formula 1-1b is selected from the leaving group;
d) Oxyacanthine is taken as a raw material, and subjected to nitration, reduction, and ring-closing reaction to obtain the compound of formula (1-2), as shown in the following reaction formula 2: 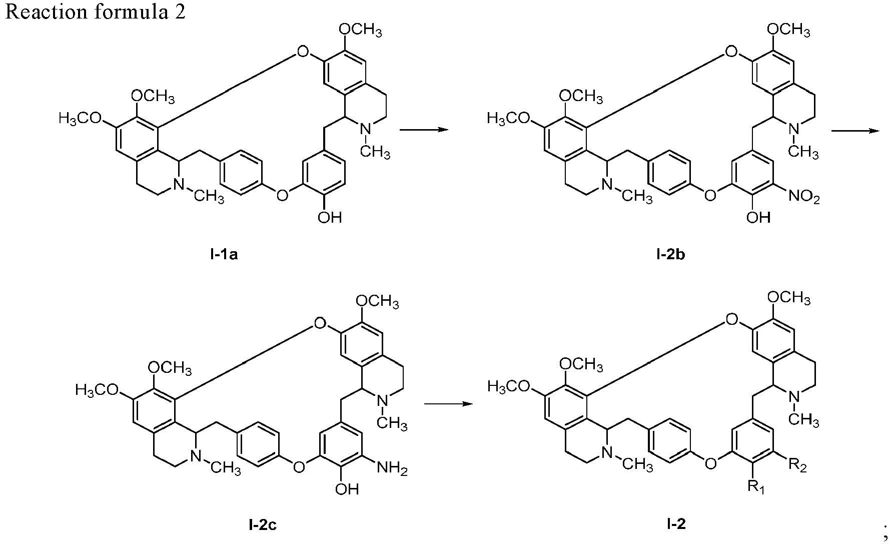 in Formula 1-2, R₁ and R₂ together with the adjacent benzene ring form a benzo[5- to 6-membered monocyclic heterocycle]which is unsubstituted or substituted by 1 to 4 substituents;
e) Oxyacanthine is taken as a raw material, and undergoes multi-steps of reaction to obtain the comnound of formula (1-3), as shown in reaction formula 3: 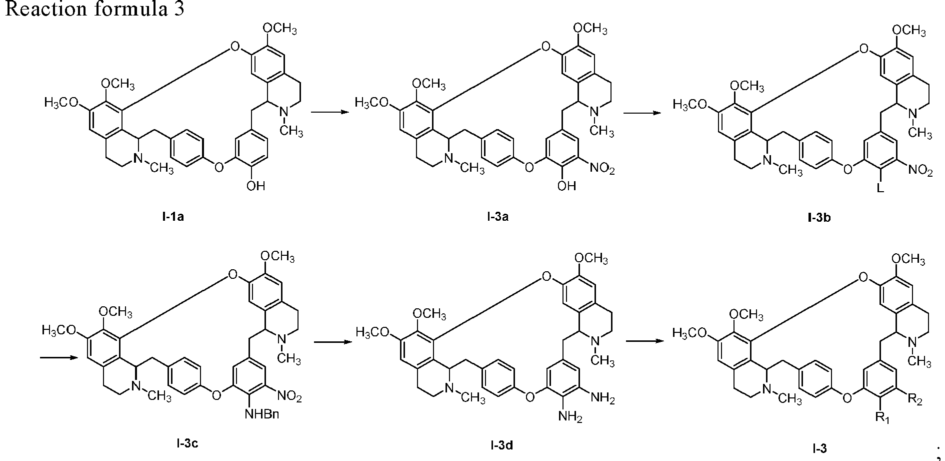 in Formula 1-3, R₁ and R₂ together with the adjacent benzene ring form a benzo[5- to 6-membered monocyclic heterocycle] which is unsubstituted or substituted by 1 to 4 substituents;
f) The compounds obtained from methods a) to e) are subjected to a functional group conversion to obtain the cyclic bisbenzyl tetrahydroisoquinoline compound.

8. A pharmaceutical composition comprising:
(A1) A first active ingredient, the first active ingredient comprising a therapeutically effective amount of one or a mixture of more of the cyclic bisbenzyl tetrahydroisoquinoline compound of Formula I according to any one of claims 1-6, an enantiomer, a diastereoisomer, a racemate, a pharmaceutically acceptable salt, a crystalline hydrate, and a solvate thereof, and
(B) Pharmaceutically acceptable carriers.

9. A use of the cyclic bisbenzyl tetrahydroisoquinoline compound according to any one of claims 1-6 or the pharmaceutical composition according to claim 8 in the preparation of an inhibitor against viral replication, inflammation, fibrosis, and abnormal differentiation of T-cells; and/or in the preparation of a medication for preventing and/or treating associated diseases caused by viral infection, inflammation/ fibrosis-related diseases (pulmonary fibrosis, silicosis, hepatic fibrosis, nonalcoholic steatohepatitis, renal fibrosis, myocardial fibrosis, dermatofibrosis, retinal fibrosis, myelofibrosis), autoimmune diseases associated with abnormal differentiation of Th1 and/or Th17 (rheumatoid arthritis, systemic lupus erythematosus, multiple sclerosis, neuromyelitisoptica), osteoporosis, inflammatory pain and neuroprotection.

10. The use according to claim 9, wherein the virus is a coronavirus selected from the group consisting of: coronaviruses infecting humans, severe acute respiratory syndrome coronavirus SARS-CoV, the 2019 novel coronavirus and its variants (SARS-CoV-2), Middle East respiratory syndrome coronavirus MERS-CoV, coronaviruses causing common cold, and combinations thereof.

11. The use according to claim 9, wherein the disease is pulmonary fibrosis, silicosis, hepatic fibrosis, non-alcoholic steatohepatitis, renal fibrosis, myocardial fibrosis, dermal fibrosis, retinal fibrosis, myelofibrosis, rheumatoid arthritis, systemic lupus erythematosus, multiple sclerosis, neuromyelitisoptica, etc., osteoporosis, inflammatory pain, neurodegenerative diseases, or a combination thereof.

12. The use according to claim 9, wherein the associated disease caused by viral infection is selected from the group consisting of: human coronavirus-induced common cold, high-risk symptomatic infections, respiratory infections, pneumonia and complications thereof, SARS-CoV-2-induced novel coronavirus pneumonia (Corona Virus Disease 2019, COVID-19), SARS-CoV-2-induced novel coronavirus infectious disease, and combinations thereof.

13. The use according to claim 9, wherein the use is for the preparation of (a) an inhibitor for inhibiting the replication of the 2019 novel coronavirus and its variants (SARS-CoV-2); and/or (b) a medicament for treating and/or preventing, or alleviating a disease associated with the infection caused by the 2019 novel coronavirus (SARS-CoV-2).

14. The use according to claim 9, wherein the use is for the preparation of a medicament for treating and/or preventing, alleviating a disease associated with inflammation, fibrosis, or abnormal differentiation of T-cells.
